# EUROPEAN PATENT APPLICATION

(11) **EP 3 992 181 A1**
(43) Date of publication of application: **04.05.2022**
(21) Application number: 20831665.3
(22) Date of filing: 01.06.2020
(51) Int. Cl.: C07C 381/12, G03F 7/004, G03F 7/038, G03F 7/039, G03F 7/20

(54) **ACTINIC RAY-SENSITIVE OR RADIATION-SENSITIVE RESIN COMPOSITION, PATTERN FORMING METHOD, RESIST FILM, AND MANUFACTURING METHOD FOR ELECTRONIC DEVICE**

(30) Priority: 28.06.2019 JP 2019121749
(71) Applicant: FUJIFILM Corporation, Tokyo 106-8620 (JP)
(72) Inventor: USHIYAMA Aina, Haibara-gun, Shizuoka 421-0396 (JP); KOJIMA Masafumi, Haibara-gun, Shizuoka 421-0396 (JP); GOTO Akiyoshi, Haibara-gun, Shizuoka 421-0396 (JP); SHIRAKAWA Michihiro, Haibara-gun, Shizuoka 421-0396 (JP); KATO Keita, Haibara-gun, Shizuoka 421-0396 (JP); OKA Hironori, Haibara-gun, Shizuoka 421-0396 (JP)
(74) Representative: HGF
(86) International application number: PCT/JP2020/021648
(87) International publication number: WO 2020/261885

(57) **Abstract**

Provided is an actinic ray-sensitive or radiation-sensitive resin composition that is capable of obtaining a pattern having excellent LWR performance even in a case where the composition has been stored for a long period of time. In addition, also provided are a resist film, a pattern forming method, and a method for manufacturing an electronic device, each relating to the actinic ray-sensitive or radiation-sensitive resin composition. The actinic ray-sensitive or radiation-sensitive resin composition including one or more specific compounds selected from the group consisting of a compound represented by General Formula (1), a compound represented by General Formula (2), and a compound represented by General Formula (3), and an acid-decomposable resin.

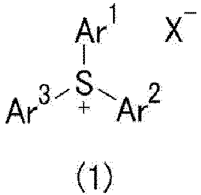

## Description

### BACKGROUND OF THE INVENTION

### 1. Field of the Invention

The present invention relates to an actinic ray-sensitive or radiation-sensitive resin composition, a pattern forming method, a resist film, and a method for manufacturing an electronic device.

### 2. Description of the Related Art

In processes for manufacturing semiconductor devices such as an integrated circuit (IC) and a large scale integrated circuit (LSI), microfabrication by lithography using a photosensitive composition has been performed.

Examples of the lithographic method include a method in which a resist film is formed with a photosensitive composition, and then the obtained film is exposed and then developed.

For example, JP2014-235248A discloses a resist composition including the following compound.

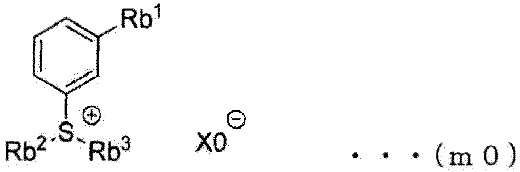

### SUMMARY OF THE INVENTION

The present inventors have specifically investigated the techniques disclosed in JP2014-235248A, and have thus found that in a case where the composition of JP2014-235248A is applied to pattern formation after the composition has been produced and then stored for a long period of time (for example, 3 months), it has room for improvement in the line width roughness (LWR) performance of a pattern thus obtained.

Therefore, an object of the present invention is to provide an actinic ray-sensitive or radiation-sensitive resin composition that is capable of obtaining a pattern having excellent LWR performance even in a case where the composition has been stored for a long period of time.

In addition, another object of the present invention is to provide a resist film, a pattern forming method, and a method for manufacturing an electronic device, each relating to the actinic ray-sensitive or radiation-sensitive resin composition.

The present inventors have found that the objects can be accomplished by the following configurations.
[1] An actinic ray-sensitive or radiation-sensitive resin composition comprising:
   one or more specific compounds selected from the group consisting of a compound represented by General Formula (1), a compound represented by General Formula (2), and a compound represented by General Formula (3); and
   an acid-decomposable resin, 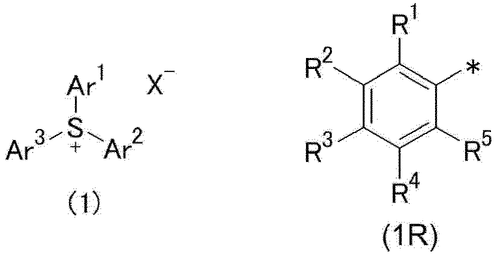
   in General Formula (1), X⁻ represents an organic anion,
   Ar¹ and Ar² each independently represent an aromatic hydrocarbon ring group having at least one substituent, and
   Ar³ represents an aromatic hydrocarbon ring group represented by General Formula (1R),
   in General Formula (1R), ^{∗} represents a bonding position,
   R¹ to R⁵ each independently represent a hydrogen atom, a fluorine atom, or an alkyl group which may have a substituent,
   provided that at least one of R¹ or R⁵ represents a fluorine-containing group selected from the group consisting of a fluorine atom and a fluoroalkyl group, and
   in General Formula (1), at least two of Ar¹, Ar², or Ar³ represent groups having different structures, 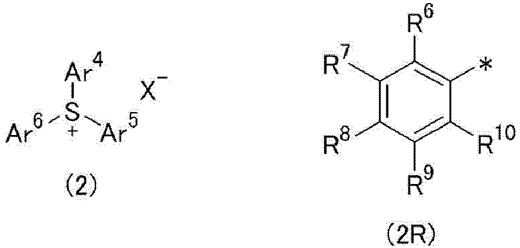
   in General Formula (2), X⁻ represents an organic anion,
   Ar⁴ and Ar⁵ each independently represent an aromatic hydrocarbon ring group having at least one substituent,
   Ar⁶ represents an aromatic hydrocarbon ring group represented by General Formula (2R),
   in General Formula (2R), ^{∗} represents a bonding position, R⁶, R⁷, R⁹, and R¹⁰ each independently represent a hydrogen atom, a fluorine atom, or an alkyl group which may have a substituent,
   R⁸ represents a fluorine-containing group selected from the group consisting of a fluorine atom and a fluoroalkyl group,
   provided that in General Formula (2), at least two of Ar⁴, Ar⁵, or Ar⁶ represent groups having different structures, and
   in General Formula (2), the aromatic hydrocarbon ring group represented by each of Ar⁴, Ar⁵, and Ar⁶ has a total of two or more fluorine-containing groups, 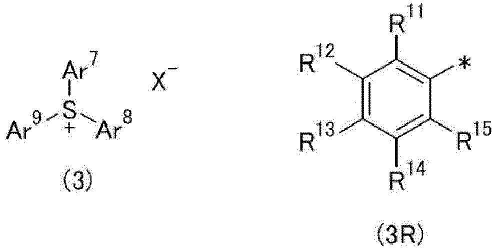 in General Formula (3), X⁻ represents an organic anion,
   Ar⁷ and Ar⁸ each independently represent an aromatic hydrocarbon ring group which may have an organic group other than an electron-withdrawing group,
   provided that the aromatic hydrocarbon ring group represented by each of Ar⁷ and Ar⁸ has a total of one or more organic groups other than an electron-withdrawing group,
   Ar⁹ represents an aromatic hydrocarbon ring group represented by General Formula (3R),
   in General Formula (3R), ^{∗} represents a bonding position,
   R¹¹, R¹³, and R¹⁵ each independently represent a hydrogen atom, a fluorine atom, or an alkyl group which may have a substituent, and
   R¹² and R¹⁴ each independently represent a fluorine-containing group selected from the group consisting of a fluorine atom and a fluoroalkyl group.
[2] The actinic ray-sensitive or radiation-sensitive resin composition as described in [1],
   in which the fluorine-containing group in each of General Formulae (1) to (3) is a fluoroalkyl group.
[3] The actinic ray-sensitive or radiation-sensitive resin composition as described in [1] or [2],
   in which in General Formula (1), the aromatic hydrocarbon ring group represented by each of Ar¹, Ar², and Ar³ has a total of three or more fluoroalkyl groups or has a total of one or more organic groups other than an electron-withdrawing group, and a total number of carbon atoms included in the total of one or more organic groups other than an electron-withdrawing group is 3 or more,
   in General Formula (2), the aromatic hydrocarbon ring group represented by each of Ar⁴, Ar⁵, and Ar⁶ has a total of three or more fluorine-containing groups, or
   the aromatic hydrocarbon ring group represented by each of Ar⁴, Ar⁵, and Ar⁶ has a total of one or more linear or branched organic groups other than an electron-withdrawing group, and a total number of carbon atoms included in the total of one or more linear or branched organic groups other than an electron-withdrawing group is 3 or more, and
   in General Formula (3), a total number of carbon atoms included in the organic groups other than an electron-withdrawing group, contained in the aromatic hydrocarbon ring group represented by each of Ar⁷, Ar⁸, and Ar⁹, is 3 or more.
[4] The actinic ray-sensitive or radiation-sensitive resin composition as described in any one of [1] to [3],
   in which Ar⁶ represents a group represented by General Formula (2S), 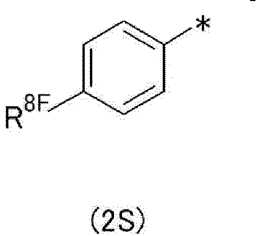
   in General Formula (2S), ^{∗} represents a bonding position, and
   R^{8F} represents a fluoroalkyl group.
[5] The actinic ray-sensitive or radiation-sensitive resin composition as described in [4],
   in which Ar⁵ and Ar⁶ each represent a group represented by General Formula (2S).
[6] A resist film formed of the actinic ray-sensitive or radiation-sensitive resin composition as described in any one of [1] to [5].
[7] A pattern forming method comprising:
   a step of forming a resist film on a substrate, using the actinic ray-sensitive or radiation-sensitive resin composition as described in any one of [1] to [5];
   a step of exposing the resist film; and
   a step of developing the exposed resist film, using a developer, to form a pattern.
[8] A method for manufacturing an electronic device comprising the pattern forming method as described in [7].

According to the present invention, it is possible to provide an actinic ray-sensitive or radiation-sensitive resin composition that is capable of obtaining a pattern having excellent LWR performance even in a case where the composition has been stored for a long period of time.

In addition, the present invention can also provide a resist film, a pattern forming method, and a method for manufacturing an electronic device, each relating to the actinic ray-sensitive or radiation-sensitive resin composition.

### DESCRIPTION OF THE PREFERRED EMBODIMENTS

Hereinafter, an example of a form for carrying out the present invention will be described.

Furthermore, in the present specification, a numerical value range expressed using "to" means a range that includes the preceding and succeeding numerical values of "to" as a lower limit value and an upper limit value, respectively.

In notations for a group (atomic group) in the present specification, in a case where the group is cited without specifying whether it is substituted or unsubstituted, the group includes both a group having no substituent and a group having a substituent. For example, an "alkyl group" includes not only an alkyl group having no substituent (unsubstituted alkyl group), but also an alkyl group having a substituent (substituted alkyl group).

The substituent is preferably a monovalent substituent unless otherwise specified.

An "organic group" in the present specification refers to a group including at least one carbon atom.

In the present specification, examples of the halogen atom include a fluorine atom, a chlorine atom, a bromine atom, and an iodine atom.

The bonding direction of divalent groups cited in the present specification is not limited unless otherwise specified. For example, in a case where Y in a compound represented by General Formula "X-Y-Z" is -COO-, Y may be -CO-O- or -O-CO-. In addition, the compound may be "X-CO-O-Z" or "X-O-CO-Z".

"(Meth)acryl" in the present specification is a generic term encompassing acryl and methacryl, and means "at least one of acryl or methacryl". Similarly, "(meth)acrylic acid" means "at least one of acrylic acid or methacrylic acid".

"Actinic rays" or "radiation" in the present specification means, for example, a bright line spectrum of a mercury lamp, far ultraviolet rays typified by an excimer laser, extreme ultraviolet rays (EUV light), X-rays, electron beams (EB), or the like. "Light" in the present specification means actinic rays or radiation.

Unless otherwise specified, "exposure" in the present specification encompasses not only exposure by a bright line spectrum of a mercury lamp, far ultraviolet rays typified by an excimer laser (an ArF excimer laser and the like), extreme ultraviolet rays (EUV light), X-rays, or the like, but also lithography by particle beams such as electron beams and ion beams.

In the present specification, a weight-average molecular weight (Mw), a number-average molecular weight (Mn), and a dispersity (also referred to as a molecular weight distribution) (Mw/Mn) of a resin are defined as values expressed in terms of polystyrene by means of gel permeation chromatography (GPC) measurement (solvent: tetrahydrofuran, flow amount (amount of a sample injected): 10 µL, columns: TSK gel Multipore HXL-M manufactured by Tosoh Corporation, column temperature: 40°C, flow rate: 1.0 mL/min, and detector: differential refractive index detector) using a GPC apparatus (HLC-8120GPC manufactured by Tosoh Corporation).

1 Å is 1 × 10⁻¹⁰ m.

In the present specification, an acid dissociation constant (pKa) represents a pKa in an aqueous solution, and is specifically a value determined by computation from a value based on a Hammett's substituent constant and database of publicly known literature values, using the following software package 1. Any of the pKa values described in the present specification indicate values determined by computation using the software package.

Software Package 1: Advanced Chemistry Development (ACD/Labs) Software V 8.14 for Solaris (1994 - 2007 ACD/Labs).

On the other hand, the pKa can also be determined by a molecular orbital computation method. Examples of a specific method therefor include a method for performing calculation by computing H⁺ dissociation free energy in a solvent based on a thermodynamic cycle. (Furthermore, in the present specification, water is usually used as the solvent, and in a case where a pKa is not determined with water, dimethyl sulfoxide (DMSO) is used.)

With regard to a computation method for H⁺ dissociation free energy, the H⁺ dissociation free energy can be computed by, for example, density functional theory (DFT), but various other methods have been reported in literature and the like, and are not limited thereto. Furthermore, there are a plurality of software applications capable of performing DFT, and examples thereof include Gaussian 16.

As described above, the pKa in the present specification refers to a value determined by computation from a value based on a Hammett's substituent constant and database of publicly known literature values, using the software package 1, but in a case where the pKa cannot be calculated by the method, a value obtained by Gaussian 16 based on density functional theory (DFT) shall be adopted.

### [Actinic Ray-Sensitive or Radiation-Sensitive Resin Composition]

The actinic ray-sensitive or radiation-sensitive resin composition of an embodiment of the present invention (hereinafter also referred to as a "resist composition") will be described.

The resist composition of the embodiment of the present invention may be either a positive tone resist composition or a negative tone resist composition. In addition, the resist composition may be either a resist composition for alkali development or a resist composition for organic solvent development.

The composition of the embodiment of the present invention is typically a chemically amplified resist composition.

### The resist composition of the embodiment of the present invention includes one or more specific compounds selected from the group consisting of a compound represented by General Formula (1), a compound represented by General Formula (2), and a compound represented by General Formula (3), and an acid-decomposable resin.

Mechanism by which the objects of the present invention can be accomplished through such configurations is not necessarily clear, but is considered to be as follows by the present inventors.

The specific compound is an onium salt including an organic anion and an organic cation, and usually acts as a photoacid generator. In each of specific compounds represented by each general formula, the organic cation has a fluorine-containing group (a fluorine atom or a fluoroalkyl group) and a substituent so that conditions such as a predetermined number, a position, and/or a type, and other conditions are satisfied. Since such a specific compound has excellent decomposition efficiency upon exposure and also has excellent compatibility with an acid-decomposable resin, the LWR performance of a pattern formed is improved. In addition, it is considered that since the electron-withdrawing property of the entire cation is appropriately adjusted and/or the nucleophilicity to a sulfonium atom is lowered, the stability of the specific compound is enhanced, and even in a case where the resist composition has been stored for a long period of time, good LWR performance can be maintained.

Hereinafter, in the present specification, a fact that a pattern having excellent LWR performance can be obtained even in a case where the resist composition has been stored for a long period of time can also be expressed as follows: the effect of the present invention is excellent.

### [Components of Resist Composition]

Hereinafter, components that can be included in the resist composition will be described in detail.

### <Specific Compound>

The resist composition of the embodiment of the present invention includes a specific compound.

The specific compound usually acts as a photoacid generator. The photoacid generator is a compound that generates an acid upon irradiation (exposure) with actinic rays or radiation (preferably EUV light or ArF).

The photoacid generator is preferably in the form of a low-molecular-weight compound.

In a case where the photoacid generator is in the form of a low-molecular-weight compound, the molecular weight is preferably 3,000 or less, more preferably 2,000 or less, and still more preferably 1,200 or less.

The specific compound is preferably a compound that generates an organic acid upon exposure.

Examples of the organic acid include sulfonic acids (an aliphatic sulfonic acid, an aromatic sulfonic acid, and a camphor sulfonic acid), carboxylic acids (an aliphatic carboxylic acid, an aromatic carboxylic acid, and an aralkyl carboxylic acid), a carbonylsulfonylimide acid, a bis(alkylsulfonyl)imide acid, and a tris(alkylsulfonyl)methide acid.

The volume of an acid generated from the photoacid generator is not particularly limited, but from the viewpoint of suppressing the diffusion of the acid generated upon exposure into the non-exposed area and improving the resolution, the volume is preferably 240 Å³ or more, more preferably 305 Å³ or more, still more preferably 350 Å³ or more, and particularly preferably 400 Å³ or more. Incidentally, from the viewpoint of the sensitivity or the solubility in an application solvent, the volume of the acid generated from the photoacid generator is preferably 1,500 Å³ or less, more preferably 1,000 Å³ or less, and still more preferably 700 Å³ or less.

The value of the volume is obtained using "WinMOPAC" manufactured by Fujitsu Limited. For the computation of the value of the volume, first, the chemical structure of an acid according to each example is input, next, using this structure as an initial structure, the most stable conformation of each acid is determined by molecular force field computation using a Molecular Mechanics (MM) 3 method, and thereafter with respect to the most stable conformation, molecular orbital calculation using a Parameterized Model number (PM) 3 method is performed, whereby the "accessible volume" of each acid can be computed.

The structure of an acid generated from the photoacid generator is not particularly limited, but from the viewpoint that the diffusion of the acid is suppression and the resolution is improved, it is preferable that the interaction between the acid generated from the photoacid generator and a resin (A) which will be described is strong. From this viewpoint, in a case where the acid generated from the photoacid generator is an organic acid, it is preferable that a polar group is further contained, in addition to an organic acid group such as a sulfonic acid group, a carboxylic acid group, a carbonylsulfonylimide acid group, a bissulfonylimide acid group, and a trissulfonylmethide acid group.

Examples of the polar group include an ether group, an ester group, an amide group, an acyl group, a sulfo group, a sulfonyloxy group, a sulfonamide group, a thioether group, a thioester group, a urea group, a carbonate group, a carbamate group, a hydroxyl group, and a mercapto group.

The number of the polar groups contained in the acid generated is not particularly limited, and is preferably 1 or more, and more preferably 2 or more. It should be noted that from the viewpoint of suppressing excessive development, the number of the polar groups is preferably less than 6, and more preferably less than 4.

The photoacid generator is preferably a photoacid generator that generates acids as exemplified below. Furthermore, in some of the examples, the computed values of the volumes are added (unit: Å³).

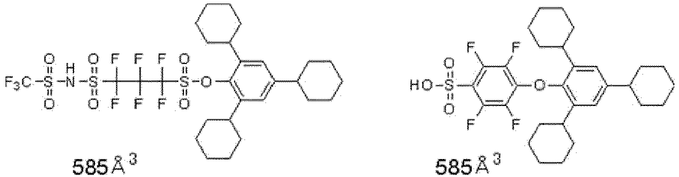

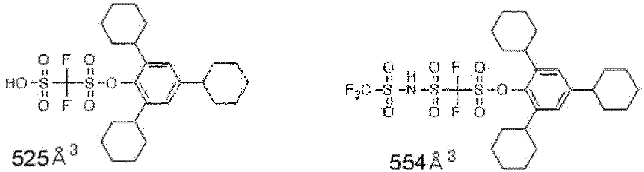

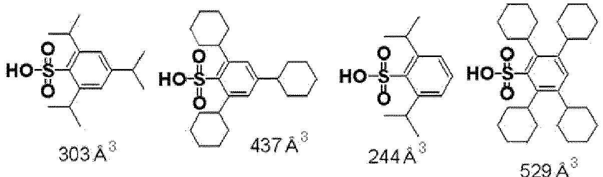

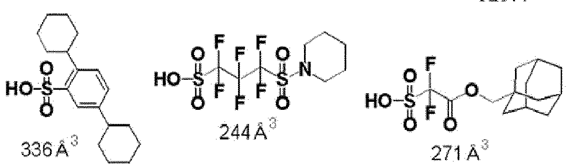

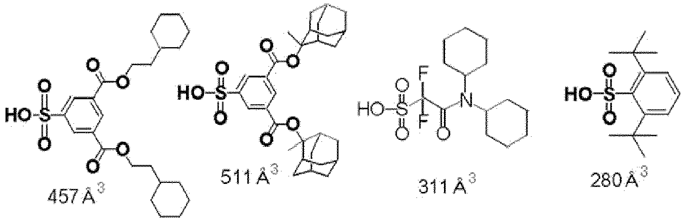

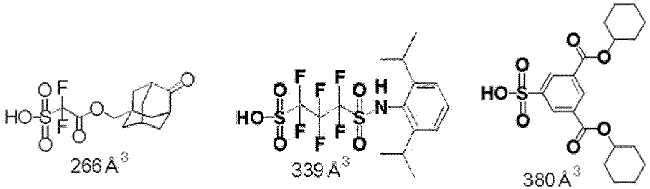

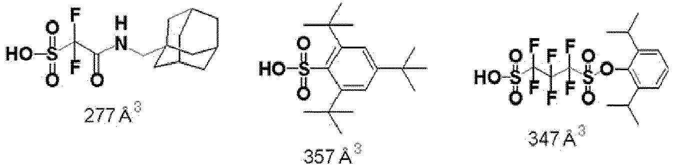

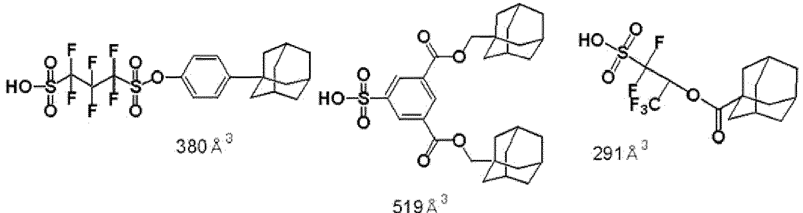

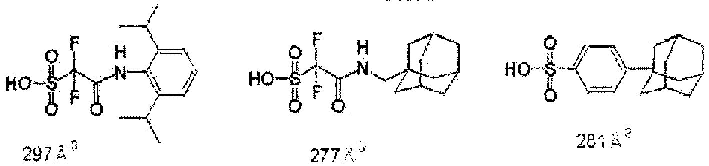

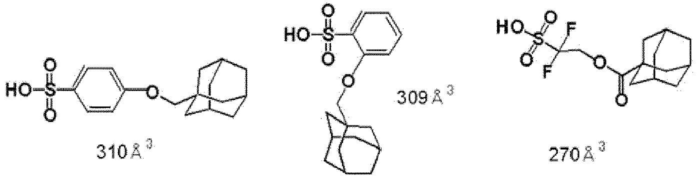

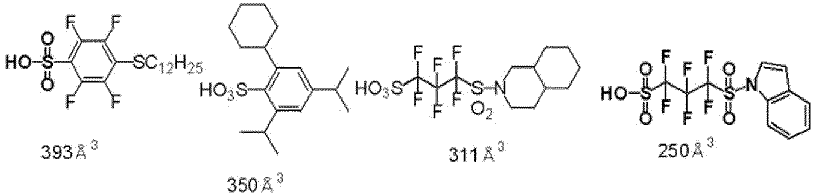

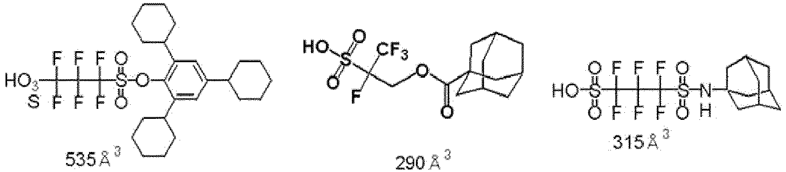

The specific compound is one or more selected from the group consisting of the compound represented by General Formula (1), the compound represented by General Formula (2), and the compound represented by General Formula (3).

Hereinafter, the compound represented by General Formula (1), the compound represented by General Formula (2), and the compound represented by General Formula (3) will each be described.

### (Compound Represented by General Formula (1))

General Formula (1) is shown below.

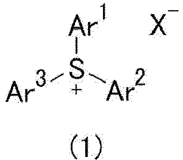

In General Formula (1), X⁻ represents an organic anion.

The organic anion is preferably a non-nucleophilic anion (anion having a significantly low ability to cause a nucleophilic reaction).

Examples of the non-nucleophilic anion include a sulfonate anion (an aliphatic sulfonate anion, an aromatic sulfonate anion, a camphor sulfonate anion, and the like), a carboxylate anion (an aliphatic carboxylate anion, an aromatic carboxylate anion, an aralkyl carboxylate anion, and the like), a sulfonylimide anion, a bis(alkylsulfonyl)imide anion, and a tris(alkylsulfonyl)methide anion.

The aliphatic moiety in the aliphatic sulfonate anion and the aliphatic carboxylate anion may be an alkyl group or a cycloalkyl group, and has a linear or branched alkyl group having 1 to 30 carbon atoms, or is preferably a cycloalkyl group having 3 to 30 carbon atoms.

The alkyl group may be, for example, a fluoroalkyl group (which may or may not have a substituent other than a fluorine atom, and may be a perfluoroalkyl group).

The aryl group in the aromatic sulfonate anion and the aromatic carboxylate anion is preferably an aryl group having 6 to 14 carbon atoms, and examples thereof include a phenyl group, a tolyl group, and a naphthyl group.

The alkyl group, the cycloalkyl group, and the aryl group exemplified above may have a substituent. The substituent is not particularly limited, but specific examples of the substituent include a nitro group, a halogen atom such as fluorine atom or a chlorine atom, a carboxyl group, a hydroxyl group, an amino group, a cyano group, an alkoxy group (preferably having 1 to 15 carbon atoms), an alkyl group (preferably having 1 to 10 carbon atoms), a cycloalkyl group (preferably having 3 to 15 carbon atoms), an aryl group (preferably having 6 to 14 carbon atoms), an alkoxycarbonyl group (preferably having 2 to 7 carbon atoms), an acyl group (preferably having 2 to 12 carbon atoms), an alkoxycarbonyloxy group (preferably having 2 to 7 carbon atoms), an alkylthio group (preferably having 1 to 15 carbon atoms), an alkylsulfonyl group (preferably having 1 to 15 carbon atoms), an alkyliminosulfonyl group (preferably having 1 to 15 carbon atoms), and an aryloxysulfonyl group (preferably having 6 to 20 carbon atoms).

The aralkyl group in the aralkyl carboxylate anion is preferably an aralkyl group having 7 to 14 carbon atoms, and examples thereof include a benzyl group, a phenethyl group, a naphthylmethyl group, a naphthylethyl group, and a naphthylbutyl group.

Examples of the sulfonylimide anion include a saccharin anion.

The alkyl group in the bis(alkylsulfonyl)imide anion and the tris(alkylsulfonyl)methide anion is preferably an alkyl group having 1 to 5 carbon atoms. Examples of the substituent of such an alkyl group include a halogen atom, an alkyl group substituted with the halogen atom, an alkoxy group, an alkylthio group, an alkyloxysulfonyl group, an aryloxysulfonyl group, and a cycloalkylaryloxysulfonyl group, and a fluorine atom or an alkyl group substituted with the fluorine atom is preferable.

In addition, the alkyl groups in the bis(alkylsulfonyl)imide anion may be bonded to each other to form a ring structure. Thus, the acid strength increases.

Examples of the other non-nucleophilic anions include fluorinated phosphorus (for example, PF₆⁻), fluorinated boron (for example, BF₄⁻), and fluorinated antimony (for example, SbF₆⁻).

As the non-nucleophilic anion, an aliphatic sulfonate anion in which at least α-position of sulfonic acid is substituted with a fluorine atom, an aromatic sulfonate anion substituted with a fluorine atom or a group having a fluorine atom, a bis(alkylsulfonyl)imide anion in which an alkyl group is substituted with a fluorine atom, or a tris(alkylsulfonyl)methide anion in which an alkyl group is substituted with a fluorine atom is preferable. Among these, a perfluoroaliphatic sulfonate anion (preferably having 4 to 8 carbon atoms) or a fluorine atom-containing benzenesulfonate anion is more preferable, and a nonafluorobutanesulfonate anion, a perfluorooctanesulfonate anion, a pentafluorobenzenesulfonate anion, or a 3,5-bis(trifluoromethyl)benzenesulfonate anion is still more preferable.

As the non-nucleophilic anion, an anion represented by Formula (AN1) is also preferable.

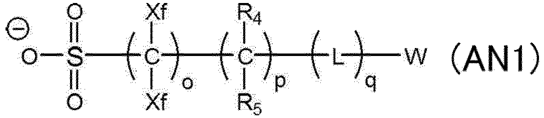

In Formula (AN1),
o represents an integer of 1 to 3. p represents an integer of 0 to 10. q represents an integer of 0 to 10.

Xf represents a fluorine atom or an alkyl group substituted with at least one fluorine atom. The alkyl group preferably has 1 to 10 carbon atoms, and more preferably has 1 to 4 carbon atoms. In addition, a perfluoroalkyl group is preferable as the alkyl group substituted with at least one fluorine atom.

Xf is preferably a fluorine atom or a perfluoroalkyl group having 1 to 4 carbon atoms, and more preferably a fluorine atom or CF₃. In particular, it is still more preferable that both Xf's are fluorine atoms.

R₄ and R₅ each independently represent a hydrogen atom, a fluorine atom, an alkyl group, or an alkyl group substituted with at least one fluorine atom. In a case where R₄'s and R₅'s are each present in a plural number, R₄'s and R₅'s may each be the same as or different from each other.

The alkyl group represented by each of R₄ and R₅ may have a substituent, and preferably has 1 to 4 carbon atoms. R₄ and R₅ are each preferably a hydrogen atom.

Specific examples and suitable aspects of the alkyl group substituted with at least one fluorine atom are the same ones as the specific examples and the suitable aspects of Xf in Formula (AN1), respectively.

L represents a divalent linking group. In a case where L's are present in a plural number, they may be the same as or different from each other.

Examples of the divalent linking group include -O-CO-O-, -COO-, -OCO-, -CONH-, -NHCO-, -CO-, -O-, -S-, -SO-, -SO₂-, an alkylene group (preferably having 1 to 6 carbon atoms), a cycloalkylene group (preferably having 3 to 15 carbon atoms), an alkenylene group (preferably having 2 to 6 carbon atoms), and a divalent linking group formed by combination of a plurality of these groups. Among those, -O-CO-O-, -COO-, -OCO-, -CONH-, -NHCO-, -CO-, -O-, -SO₂-, -O-CO-O-alkylene group-, -alkylene group-O-CO-O-, -COO-alkylene group-, -OCO-alkylene group-, -CONH-alkylene group-, or -NHCO-alkylene group- is preferable; and -O-CO-O-, -O-CO-O-alkylene group-, -alkylene group-O-CO-O-, -COO-, -OCO-, -CONH-, -SO₂-, -COO-alkylene group-, or -OCO-alkylene group- is more preferable.

W represents an organic group including a cyclic structure. Among those, W is preferably a cyclic organic group.

Examples of the cyclic organic group include an alicyclic group, an aryl group, and a heterocyclic group.

The alicyclic group may be monocyclic or polycyclic. Examples of the monocyclic alicyclic group include monocyclic cycloalkyl groups such as a cyclopentyl group, a cyclohexyl group, and a cyclooctyl group. Examples of the polycyclic alicyclic group include polycyclic cycloalkyl groups such as a norbornyl group, a tricyclodecanyl group, a tetracyclodecanyl group, a tetracyclododecanyl group, and an adamantyl group. Among those, an alicyclic group having a bulky structure having 7 or more carbon atoms, such as a norbornyl group, a tricyclodecanyl group, a tetracyclodecanyl group, a tetracyclododecanyl group, and an adamantyl group, is preferable.

The aryl group may be monocyclic or polycyclic. Examples of the aryl group include a phenyl group, a naphthyl group, a phenanthryl group, and an anthryl group.

The heterocyclic group may be monocyclic or polycyclic. The polycyclic compound can further suppress acid diffusion. Furthermore, the heterocyclic group may have aromaticity or may not have aromaticity. Examples of the heterocyclic ring having aromaticity include a furan ring, a thiophene ring, a benzofuran ring, a benzothiophene ring, a dibenzofuran ring, a dibenzothiophene ring, and a pyridine ring. Examples of the heterocyclic ring not having aromaticity include a tetrahydropyran ring, a lactone ring, a sultone ring, and a decahydroisoquinoline ring. As the heterocyclic ring in the heterocyclic group, the furan ring, the thiophene ring, the pyridine ring, or the decahydroisoquinoline ring is particularly preferable.

The cyclic organic group may have a substituent. Examples of the substituent include an alkyl group (which may be either linear or branched, preferably having 1 to 12 carbon atoms), a cycloalkyl group (which may be any of a monocycle, a polycycle, and a spirocycle, and preferably has 3 to 20 carbon atoms), an aryl group (preferably having 6 to 14 carbon atoms), a hydroxyl group, an alkoxy group, an ester group, an amide group, a urethane group, a ureide group, a thioether group, a sulfonamide group, and a sulfonic acid ester group. Incidentally, the carbon constituting the cyclic organic group (carbon contributing to ring formation) may be carbonyl carbon.

As the anion represented by Formula (AN1), SO₃⁻-CF₂-CH₂-OCO-(L)q'-W, SO₃⁻-CF₂-CHF-CH₂-OCO-(L)q'-W,SO⁻₃-CF₂-COO-(L)q'-W, SO⁻₃-CF₂-CF₂-CH₂-CH₂-(L)q-W, or SO₃⁻-CF₂-CH(CF₃)-OCO-(L)q'-W is preferable. Here, L, q, and W are each the same as in Formula (AN1). q' represents an integer of 0 to 10.

As the non-nucleophilic anion, an anion represented by Formula (AN2) is also preferable.

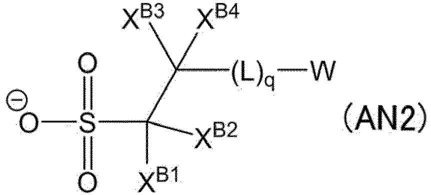

In Formula (AN2),
X^{B1} and X^{B2} each independently represent a hydrogen atom or a monovalent organic group having no fluorine atom. It is preferable that X^{B1} and X^{B2} are each the hydrogen atom.
X^{B3} and X^{B4} each independently represent a hydrogen atom or a monovalent organic group. It is preferable that at least one of X^{B3} or X^{B4} is a fluorine atom or a monovalent organic group having a fluorine atom, and it is more preferable that both of X^{B3} and X^{B4} are fluorine atoms or monovalent organic groups having a fluorine atom. It is still more preferable that both X^{B3} and X^{B4} are fluorine-substituted alkyl groups.
L, q, and W are the same as in Formula (AN1).

As the non-nucleophilic anion, an anion represented by Formula (AN3) is preferable.

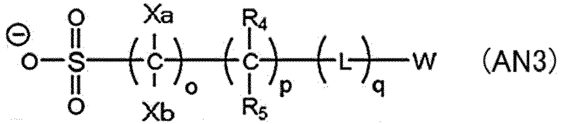

In Formula (AN3), Xa's each independently represent a fluorine atom or an alkyl group substituted with at least one fluorine atom. Xb's each independently represent a hydrogen atom or an organic group having no fluorine atom. The definitions and preferred aspects of o, p, q, R₄, R₅, L, and W are each the same as those in Formula (AN1).

As the non-nucleophilic anion, an anion represented by Formula (AN4) is also preferable.

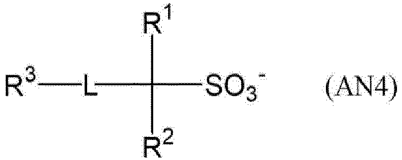

In Formula (AN4), R¹ and R² each independently represent a substituent that is not an electron-withdrawing group, or a hydrogen atom.

Examples of the substituent that is not the electron-withdrawing group include a hydrocarbon group, a hydroxyl group, an oxyhydrocarbon group, an oxycarbonyl hydrocarbon group, an amino group, a hydrocarbon-substituted amino group, and a hydrocarbon-substituted amide group.

In addition, it is preferable that the substituents which are not electron-withdrawing groups are each independently -R', -OH, -OR', -OCOR', -NH₂, -NR'₂, -NHR', or -NHCOR. R' is a monovalent hydrocarbon group.

Examples of the monovalent hydrocarbon group represented by R' include:
monovalent linear or branched hydrocarbon groups such as alkyl groups such as a methyl group, an ethyl group, a propyl group, and a butyl group; alkenyl groups such as an ethenyl group, a propenyl group, and a butenyl group; and alkynyl groups such as an ethynyl group, a propynyl group, and a butynyl group;
monovalent alicyclic hydrocarbon groups such as cycloalkyl groups such as a cyclopropyl group, a cyclobutyl group, a cyclopentyl group, a cyclohexyl group, a norbornyl group, and an adamantyl group; and cycloalkenyl groups such as a cyclopropenyl group, a cyclobutenyl group, a cyclopentenyl group, and a norbornenyl group; and
monovalent aromatic hydrocarbon groups such as aryl groups such as a phenyl group, a tolyl group, a xylyl group, a mesityl group, a naphthyl group, a methylnaphthyl group, an anthryl group, and a methylanthryl group; and aralkyl groups such as a benzyl group, a phenethyl group, a phenylpropyl group, a naphthylmethyl group, and an anthrylmethyl group.
Among those, R¹ and R² are each independently preferably the hydrocarbon group (preferably a cycloalkyl group) or the hydrogen atom.

In Formula (AN4), L represents a divalent linking group consisting of a combination of one or more linking groups S and one or more alkylene groups which may have a substituent, or a divalent linking group consisting of one or more linking groups S.

The linking group S is a group selected from the group consisting of ^{∗A}-O-CO-O-^{∗B}, ^{∗A}-CO-^{∗B}, ^{∗A}-CO-O-^{∗B}, ^{∗A}-O-CO-^{∗B}, ^{∗A}-O-^{∗B}, ^{∗A}-S-^{∗B}, and ^{∗A}-SO₂-^{∗B}.

It should be noted that in a case where L is a "divalent linking group consisting of a combination of one or more linking groups S and one or more alkylene groups which have no substituent, which is one form of a "divalent linking group consisting of a combination of one or more linking groups S and one or more alkylene groups which may have a substituent", it is preferable that the linking group S is a group selected from the group consisting of ^{∗}A-O-CO-O-^{∗B}, ^{∗A}-CO-^{∗B}, ^{∗A}-O-CO-^{∗B}, ^{∗A}-O-^{∗B}, ^{∗A}-S-^{∗B}, and ^{∗A}-SO₂-^{∗B}. In other words, in a case where the alkylene groups in the "divalent linking group consisting of a combination of one or more linking groups S and one or more alkylene groups which may have a substituent" are all unsubstituted alkylene groups, it is preferable that the linking group S is a group selected from the group consisting of ^{∗A}-O-CO-O-^{∗B}, ^{∗A}-CO-^{∗B}, ^{∗A}-O-CO-^{∗B}, ^{∗A}-O-^{∗B}, ^{∗A}-S-^{∗B}, and ^{∗A}-SO₂-^{∗B}.

^{∗A} represents a bonding position on the R³ side in Formula (AN4) and ^{∗B} represents a bonding position on the -SO₃⁻ side in Formula (AN4).

In the divalent linking group consisting of a combination of one or more linking groups S and one or more alkylene groups which may have a substituent, only one linking group S may be present, or two or more linking groups S may be present. Similarly, with regard to the alkylene group which may have a substituent, only one alkylene group which may have a substituent may be present, or two or more alkylene groups which may have a substituent may be present. In a case where the linking groups S are present in a plural number, the linking groups S that are present in a plural number may be the same as or different from each other. In a case where the alkylene groups are present in a plural number, the alkylene groups that are present in a plural number may be the same as or different from each other.

Furthermore, the linking groups S may be successively bonded to each other. It should be noted that it is preferable that groups selected from the group consisting of ^{∗A}-CO-^{∗B}, ^{∗A}-O-CO-^{∗B}, and ^{∗A}-O-^{∗B} are successively bonded not to form "^{∗A}-O-CO-O-^{∗B}". In addition, it is preferable that groups selected from the group consisting of ^{∗A}-CO-^{∗B} and ^{∗A}-O-^{∗B} are successively bonded not to form any of "^{∗A}-O-CO-^{∗B}" and "^{∗A}-CO-O-^{∗B}".

Also in the divalent linking group consisting of one or more linking groups S, only one linking group S may be present, or two or more linking groups S may be present. In a case where the linking groups S are present in a plural number, the linking groups S that are present in a plural number may be the same as or different from each other.

Also in this case, it is preferable that "^{∗A}-O-CO-O-^{∗B}" is not formed by the successive bonding of groups selected from the group consisting of ^{∗A}-CO-^{∗B}, ^{∗A}-O-CO-^{∗B}, and ^{∗A}-O-^{∗B}. In addition, it is preferable that groups selected from the group consisting of ^{∗A}-CO-^{∗B} and ^{∗A}-O-^{∗B} are successively bonded not to form any of "^{∗A}-O-CO-^{∗B}" and "^{∗A}-CO-O-^{∗B}".

It should be noted that in any case, in L, an atom at the β-position with respect to -SO₃⁻is not a carbon atom having a fluorine atom as a substituent.

Furthermore, in a case where the atom at the β-position is a carbon atom, the carbon atom only needs to be not directly substituted with a fluorine atom, and the carbon atom may have a substituent having a fluorine atom (for example, a fluoroalkyl group such as a trifluoromethyl group).

In addition, the atom at the β-position is, in other words, the atom in L directly bonded to -C(R¹)(R²)- in Formula (AN4).

Above all, it is preferable that L has only one linking group S.

That is, it is preferable that L represents a divalent linking group consisting of a combination of one linking group S and one or more alkylene groups which may have a substituent, or a divalent linking group consisting of one linking group S.

L is preferably for example, a group represented by Formula (AN4-2).

^{∗a}-(CR^{2a}₂)_{X}-Q-(CR^{2b}₂)_{Y}-^{∗b} (AN4-2)

In Formula (AN4-2), ^{∗a} represents a bonding position to R³ in Formula (AN4).

^{∗b} represents a bonding position to -C(R¹)(R²)- in Formula (AN4).

X and Y each independently represent an integer of 0 to 10, and is preferably an integer of 0 to 3.

R^{2a} and R^{2b} each independently represent a hydrogen atom or a substituent.

In a case where R^{2a}'s and R^{2b}'s are each present in a plural number, R^{2a}'s which are present in a plural number and R^{2b}'s which are present in a plural number may each be the same as or different from each other.

It should be noted that in a case where Y is 1 or more, R^{2b} in CR^{2b}₂ which is directly bonded to -C(R¹)(R²)- in Formula (AN4) is other than a fluorine atom.

Q represents ^{∗}A-O-CO-O-^{∗B}, ^{∗A}-CO-^{∗B}, ^{∗A}-CO-O-^{∗B}, ^{∗A}-O-CO-^{∗B}, ^{∗A}-O-^{∗B}, ^{∗A}-S-^{∗B}, or ^{∗A}-SO₂-^{∗B}.

It should be noted that in a case where X + Y in Formula (AN4-2) is 1 or more and both of R^{2a} and R^{2b} in Formula (AN4-2) are all hydrogen atoms, Q represents ^{∗}A-O-CO-O-^{∗B}, ^{∗A}-CO-^{∗B}, ^{∗A}-O-CO-^{∗B}, ^{∗A}-O-^{∗B}, ^{∗A}-S-^{∗B}, or ^{∗A}-SO₂-^{∗B}.

^{∗A} represents a bonding position on the R³ side in Formula (AN4) and ^{∗B} represents a bonding position on the -SO₃⁻ side in Formula (AN4).

In Formula (AN4), R³ represents an organic group.

The organic group is not limited as long as it has one or more carbon atoms, may be a linear group (for example, a linear alkyl group) or a branched group (for example, a branched alkyl group such as a t-butyl group), and may have a cyclic structure. The organic group may or may not have a substituent. The organic group may or may not have a heteroatom (an oxygen atom, a sulfur atom, a nitrogen atom, and/or the like).

Among those, R³ is preferably an organic group having a cyclic structure. The cyclic structure may be a monocycle or a polycycle, and may have a substituent. The ring in the organic group containing a cyclic structure is preferably directly bonded to L in Formula (AN4).

The organic group having a cyclic structure may or may not have, for example, a heteroatom (an oxygen atom, a sulfur atom, a nitrogen atom, and/or the like). The heteroatom may be substituted with one or more of carbon atoms forming the cyclic structure.

The organic group having a cyclic structure is preferably for example, a hydrocarbon group with a cyclic structure, a lactone ring group, or a sultone ring group. Among those, the organic group having a cyclic structure is preferably a hydrocarbon group with a cyclic structure.

The hydrocarbon group with a cyclic structure is preferably a monocyclic or polycyclic cycloalkyl group. Such a group may have a substituent.

The cycloalkyl group may be a monocycle (a cyclohexyl group or the like) or a polycycle (an adamantyl group or the like), and preferably has 5 to 12 carbon atoms.

As the lactone group and the sultone group, for example, a group formed by extracting one hydrogen atom from a ring member atom constituting the lactone structure or the sultone structure in any of the structures represented by General Formulae (LC1-1) to (LC1-21) which will be described later and the structures represented by General Formulae (SL1-1) to (SL1-3) as described above is preferable.

The non-nucleophilic anion may be a benzenesulfonate anion, and is preferably a benzenesulfonate anion substituted with a branched alkyl group or a cycloalkyl group.

As the non-nucleophilic anion, an aromatic sulfonate anion represented by Formula (AN5) is also preferable.

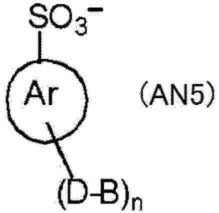

In Formula (AN5),

Ar represents an aryl group (a phenyl group and the like), and may further have a substituent other than a sulfonate anion and a -(D-B) group. Examples of the substituent which may be further contained include a fluorine atom and a hydroxyl group.

n represents an integer of 0 or more. n is preferably 1 to 4, more preferably 2 or 3, and still more preferably 3.

D represents a single bond or a divalent linking group. Examples of the divalent linking group include an ether group, a thioether group, a carbonyl group, a sulfoxide group, a sulfone group, a sulfonic acid ester group, an ester group, and a group consisting of a combination of two or more of these.

B represents a hydrocarbon group.

It is preferable that B is an aliphatic hydrocarbon structure. B is more preferably an isopropyl group, a cyclohexyl group, or an aryl group (a tricyclohexylphenyl group and the like) which may further have a substituent.

A disulfonamide anion is also preferable as the non-nucleophilic anion.

The disulfonamide anion is, for example, an anion represented by N⁻(SO₂-R^{q})₂.

Here, R^{q} represents an alkyl group which may have a substituent, and is preferably a fluoroalkyl group, and more preferably a perfluoroalkyl group. Two of R^{q}'s may be bonded to each other to form a ring. A group formed by the mutual bonding of two of R^{q}'s is preferably an alkylene group which may have a substituent, more preferably a fluoroalkylene group, and still more preferably a perfluoroalkylene group. The alkylene group preferably has 2 to 4 carbon atoms.

In addition, examples of the non-nucleophilic anion include anions represented by Formulae (d1-1) to (d1-3).

The specific compound having the anion represented by each of Formulae (d1-1) to (d1-3) as an anion can also have a function as an acid diffusion control agent which will be described later.

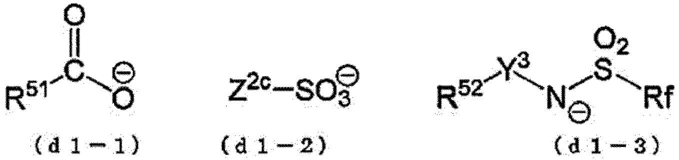

In Formula (d1-1), R⁵¹ represents a hydrocarbon group (for example, an aryl group such as a phenyl group) which may have a substituent (for example, a hydroxyl group).

In Formula (d1-2), Z^{2c} represents a hydrocarbon group having 1 to 30 carbon atoms, which may have a substituent (provided that a carbon atom adjacent to S is not substituted with a fluorine atom).

The hydrocarbon group for Z^{2c} may be linear or branched, and may have a cyclic structure. Furthermore, a carbon atom in the hydrocarbon group (preferably a carbon atom that is a ring member atom in a case where the hydrocarbon group has the cyclic structure) may be carbonyl carbon (-CO-). Examples of the hydrocarbon group include a group having a norbornyl group which may have a substituent. The carbon atom forming the norbornyl group may be carbonyl carbon.

In addition, it is preferable that "Z^{2c}-SO₃⁻" in Formula (d1-2) is different from the above-described anions represented by Formulae (AN1) to (AN5). For example, Z^{2c} is preferably a group other than an aryl group. In addition, for example, the atoms at the α-position and the β-position with respect to -SO₃⁻ in Z^{2c} are preferably atoms other than the carbon atom having a fluorine atom as a substituent. For example, in Z^{2c}, it is preferable that the atom at the α-position and/or the atom at the β-position with respect to -SO₃⁻ is a ring member atom in the cyclic group.

In Formula (d1-3), R⁵² represents an organic group (preferably a hydrocarbon group having a fluorine atom), Y³ represents a linear, branched, or cyclic alkylene group, an arylene group, or a carbonyl group, and Rf represents a hydrocarbon group.

In General Formula (1), Ar¹ and Ar² each independently represent an aromatic hydrocarbon ring group having at least one (preferably 1 to 5) substituent.

Examples of the aromatic hydrocarbon ring group include a benzene ring group, a naphthalene ring group, and an anthracene ring group. Among those, the aromatic hydrocarbon ring groups are each independently preferably the benzene ring group.

At least one (preferably 1 to 5) substituent contained in the aromatic hydrocarbon ring group is each independently preferably a fluorine atom, a fluoroalkyl group, or an organic group other than an electron-withdrawing group, and more preferably the fluoroalkyl group or the organic group other than an electron-withdrawing group.

Examples of the fluoroalkyl group include the same fluoroalkyl group as a fluoroalkyl group in a fluorine-containing group which will be described later.

The organic groups other than an electron-withdrawing group are each independently preferably a hydrocarbon group, an oxyhydrocarbon group, a hydrocarbon-substituted amino group, or a hydrocarbon-substituted amide group. It should be noted that the hydrocarbon group moiety of these organic groups does not have a halogen atom as a substituent. Furthermore, the hydrocarbon group moiety of these organic groups may be linear or branched, or may have a cyclic structure as a whole or a part. Among those, the hydrocarbon group moiety is preferably linear or branched, and preferably does not have a cyclic structure. Furthermore, it is preferable that the hydrocarbon group moiety has no substituent.

In addition, the organic groups other than an electron-withdrawing group are each independently more preferably -R', -OR', -NR'₂, -NHR', or -NHCOR'. R' is a monovalent hydrocarbon group. It should be noted that the hydrocarbon group does not have a halogen atom as a substituent. The hydrocarbon group may be linear or branched, and may have a cyclic structure as a whole or as a part. Among those, the hydrocarbon group is preferably linear or branched, and preferably does not have a cyclic structure. Furthermore, it is also preferable that the hydrocarbon group has no substituent.

The organic groups other than an electron-withdrawing group are each independently preferably a hydrocarbon group (-R'), and more preferably a linear or branched alkyl group.

Examples of the monovalent hydrocarbon group represented by R' include:
linear or branched hydrocarbon groups such as alkyl groups such as a methyl group, an ethyl group, an n-propyl group, an isopropyl group, an n-butyl group, and a tert-butyl group; alkenyl groups such as an ethenyl group, a propenyl group, and a butenyl group; and alkynyl groups such as an ethynyl group, a propynyl group, and a butynyl group;
alicyclic hydrocarbon groups such as cycloalkyl groups such as a cyclopropyl group, a cyclobutyl group, a cyclopentyl group, a cyclohexyl group, a norbornyl group, and an adamantyl group; and cycloalkenyl groups such as a cyclopropenyl group, a cyclobutenyl group, a cyclopentenyl group, and a norbornenyl group; and
aromatic hydrocarbon groups such as aryl groups such as a phenyl group, a tolyl group, a xylyl group, a mesityl group, a naphthyl group, a methylnaphthyl group, an anthryl group, and a methylanthryl group; and aralkyl groups such as a benzyl group, a phenethyl group, a phenylpropyl group, a naphthylmethyl group, and an anthrylmethyl group.

The numbers of carbon atoms of the monovalent hydrocarbon groups represented by R' are each independently preferably 1 to 10, more preferably 2 to 8, and still more preferably 3 to 6.

In a case where the organic group other than an electron-withdrawing group has a substituent (for example, a case where the R' portion in -R', -OR', -NR'₂, -NHR', or -NHCOR' has a substituent), the substituent is preferably the above-mentioned organic groups (-R', -OR', -NR'₂, -NHR', and/or -NHCOR', and the like) other than an electron-withdrawing group.

The number of carbon atoms of all the organic groups other than an electron-withdrawing group (a total number of carbon atoms, including the number of carbon atoms included in substituents in the case where the organic groups have the substituents) are each independently preferably 1 to 10, more preferably 2 to 8, and still more preferably 3 to 6.

The aromatic hydrocarbon ring group in each of Ar¹ and Ar² may or may not have a substituent other than the above-mentioned organic groups other than an electron-withdrawing group. It is preferable that the aromatic hydrocarbon ring group in each of Ar¹ and Ar² does not have a substituent other than the above-mentioned organic groups other than an electron-withdrawing group.

Furthermore, in a case where it is mentioned that the aromatic hydrocarbon group has a substituent (an organic group other than an electron-withdrawing group, and the like), it is intended to mean that the substituent (the organic group other than an electron-withdrawing group, and the like) is directly bonded to a ring member atom of the aromatic hydrocarbon group. For example, in a case where it is mentioned that the aromatic hydrocarbon group has an organic group other than an electron-withdrawing group, it is intended to mean that a part of the substituents of the aromatic hydrocarbon group does not include an organic group other than an electron-withdrawing group, but the organic group other than an electron-withdrawing group is directly bonded to a ring member atom of the aromatic hydrocarbon group. Hereinafter, in a case where the same expression is used for a specific compound, it has the same intention.

In General Formula (1), Ar³ represents an aromatic hydrocarbon ring group (benzene ring group) represented by General Formula (1R).

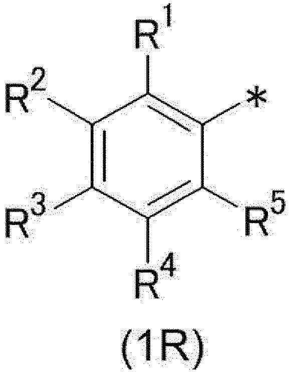

In General Formula (1R), ^{∗} represents a bonding position.

In General Formula (1R), R¹ to R⁵ each independently represent a hydrogen atom, a fluorine atom, or an alkyl group which may have a substituent.

The alkyl group which may have a substituent is preferably a fluoroalkyl group.

It should be noted that at least one of R¹ or R⁵ represents a fluorine-containing group selected from the group consisting of a fluorine atom and a fluoroalkyl group.

The fluoroalkyl group in the fluorine-containing group is an alkyl group having at least one fluorine atom as a substituent. An alkyl group in the fluoroalkyl group may be linear or branched. It is also preferable that the alkyl group in the fluoroalkyl group does not have a substituent other than a fluorine atom. The fluoroalkyl group may be a perfluoroalkyl group. The fluoroalkyl group preferably has 1 to 10 carbon atoms, and more preferably has 1 to 5 carbon atoms.

Above all, the fluorine-containing group is preferably a fluoroalkyl group.

The aromatic hydrocarbon ring group represented by each of Ar¹, Ar², and Ar³ preferably has a total of 1 to 10 fluorine-containing groups (preferably fluoroalkyl groups).

In General Formula (1), at least two of Ar¹, Ar², or Ar³ represent groups having different structures. In other words, all of Ar¹, Ar², and Ar³ are not groups having the same structure.

Furthermore, an expression that all of Ar¹, Ar², and Ar³ are groups having the same structure means, for example, that both the aromatic hydrocarbon ring groups of Ar¹ and Ar² are benzene ring groups, and each of the types and arrangements (bonding positions) of groups contained in the benzene ring groups of Ar¹, Ar², and Ar³ are the same. In other words, it is not mentioned that Ar¹, Ar², and Ar³ are groups having the same structure in a case where all of the arrangements of those groups are not the same even with combinations of the types of the groups contained in the benzene ring groups of Ar¹, Ar², and Ar³ being all the same.

Hereinafter, in a case where the same expression is used for General Formula (2) and General Formula (3), it has the same intention.

Above all, it is preferable that the compound represented by General Formula (1) satisfies the following condition A or condition B. Furthermore, in a case where the condition A or the condition B is satisfied, only one of the condition A and the condition B may be satisfied, or the both may be satisfied.

Condition A: In General Formula (1), the aromatic hydrocarbon ring group represented by each of Ar¹, Ar², and Ar³ has a total of 2 or more (preferably 3 or more, and more preferably 3 to 10) fluorine-containing groups (preferably fluoroalkyl groups).

Condition B: In General Formula (1), the aromatic hydrocarbon ring group represented by each of Ar¹, Ar², and Ar³ has a total of one or more (preferably 1 to 5, and more preferably 2) organic groups (preferably hydrocarbon groups, and more preferably alkyl groups, which are preferably linear or branched) other than an electron-withdrawing group, and the total number of carbon atoms included in a total of one or more organic groups other than an electron-withdrawing groups is 2 or more (preferably 3 or more, more preferably 3 to 20, and still more preferably 6 to 10).

Here, the expression that a total number of carbon atoms included in the total of one or more organic groups other than an electron-withdrawing groups is 3 or more means, for example, that in a case where a plurality of organic groups other than an electron-withdrawing group are present as a substituent of the aromatic hydrocarbon ring group represented by each of Ar¹, Ar², and Ar³, a total number of carbon atoms included in the plurality of organic groups other than an electron-withdrawing group is 3 or more. In addition, for example, in a case where only one organic group other than an electron-withdrawing group is present as the substituent of the aromatic hydrocarbon ring group represented by each of Ar¹, Ar², and Ar³, a total number of carbon atoms included in the one organic group other than an electron-withdrawing group is 3 or more.

More specifically, description will be made using a compound (A), a compound (B), and a compound (C), each shown below. X⁻ in the compound (A), the compound (B), and the compound (C) represents an organic anion, and any of the compound (A), the compound (B), and the compound (C) correspond to a compound represented by General Formula (1).

For example, in a case where the compound (A), the compound (B), and the compound (C) are applied to General Formula (1), any of the totals of the aromatic carbon hydrogen ring groups represented by Ar¹, Ar², and Ar³, contained in the organic groups other than electron-withdrawing groups, are 2.

In addition, in a case where the compound (A), the compound (B), and the compound (C) are applied to General Formula (1), the total numbers of carbon atoms included in the organic groups other than an electron-withdrawing group, contained in the aromatic hydrocarbon ring group represented by each of Ar¹, Ar², and Ar³, are 8, 2, and 8 from the left.

Furthermore, in a case where the compound (A), the compound (B), and the compound (C) are applied to General Formula (1), the total numbers of fluorine-containing groups contained in the aromatic hydrocarbon ring groups represented by Ar¹, Ar², and Ar³ are 1, 5, and 2 from the left.

In addition, in a case where the same expression is used for the specific compound, it has the same intention.

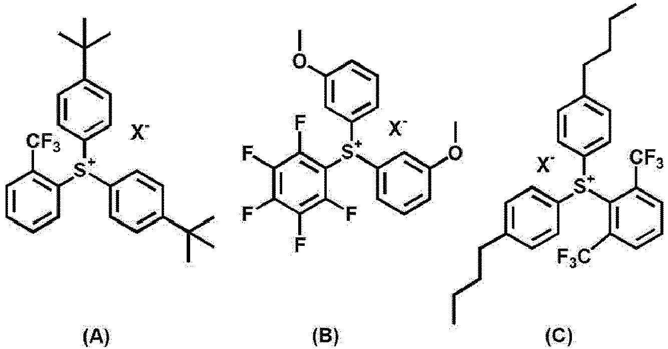

### (Compound Represented by General Formula (2))

General Formula (2) is shown below.

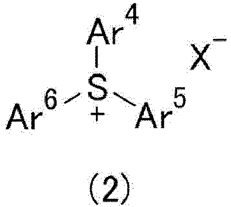

In General Formula (2), X⁻ represents an organic anion.

Examples of the organic anion represented by X⁻ in General Formula (2) include the organic anion represented by X⁻ described with respect to General Formula (1).

Ar⁴ and Ar⁵ each independently represent an aromatic hydrocarbon ring group having at least one substituent.

Examples of the aromatic hydrocarbon ring group include a benzene ring group, a naphthalene ring group, and an anthracene ring group. Among those, the aromatic hydrocarbon ring groups are each independently preferably the benzene ring group.

At least one (preferably 1 to 5) substituent contained in the aromatic hydrocarbon ring group is each independently preferably a fluorine atom, a fluoroalkyl group, or an organic group other than an electron-withdrawing group, and more preferably the fluoroalkyl group or the organic group other than an electron-withdrawing group.

Furthermore, examples of the fluoroalkyl group in General Formula (2) (including General Formula (2R)) include the same ones as the fluoroalkyl groups which can serve as the fluorine-containing group described with respect to General Formula (1) (including General Formula (1R)).

Examples of the organic group other than an electron-withdrawing group in General Formula (2) (including General Formula (2R)) include the same ones as the organic groups other than an electron-withdrawing group described with respect to General Formula (1) (including General Formula (1R)).

Among those, the organic group other than an electron-withdrawing group as a substituent of the aromatic hydrocarbon ring group of each of Ar⁴ and Ar⁵ is preferably linear or branched. For example, the organic groups other than an electron-withdrawing group are each independently -R', -OR', -OCOR', -NR'₂, -NHR', or -NHCOR', in which R' is preferably a linear or branched hydrocarbon group. It is considered that in a case where the organic group other than an electron-withdrawing group is linear or branched, the compound represented by General Formula (2) tends to have good compatibility with an acid-decomposable resin, and thus, the effect of the present invention is more excellent.

In General Formula (2), Ar⁶ represents an aromatic hydrocarbon ring group (benzene ring group) represented by General Formula (2R).

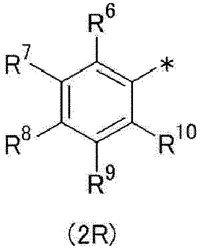

In General Formula (2R), ^{∗} represents a bonding position.

In General Formula (2R), R⁶, R⁷, R⁹, and R¹⁰ each independently represent a hydrogen atom, a fluorine atom, or an alkyl group which may have a substituent.

The alkyl group which may have a substituent is preferably a fluoroalkyl group.

That is, R⁶, R⁷, R⁹, and R¹⁰ are each independently preferably the hydrogen atom, the fluorine atom, or the fluoroalkyl group, and more preferably the hydrogen atom.

In General Formula (2R), R⁸ represents a fluorine-containing group selected from the group consisting of a fluorine atom and a fluoroalkyl group. The fluorine-containing group is preferably the fluoroalkyl group.

Ar⁶ (aromatic hydrocarbon ring group (benzene ring group) represented by General Formula (2R)) is preferably a group represented by General Formula (2S).

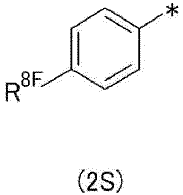

In General Formula (2S), ^{∗} represents a bonding position.

In General Formula (2S), R^{8F} represents a fluoroalkyl group. The fluoroalkyl group for R^{8F} is the same as, for example, the fluoroalkyl group for R⁸ of General Formula (2R).

It is also preferable that both Ar⁵ and Ar⁶ in General Formula (2) are the groups represented by General Formula (2S).

In this case, the group represented by General Formula (2S) for Ar⁵ and the group represented by General Formula (2S) for Ar⁶ may be the same as or different from each other.

In addition, in a case where both Ar⁵ and Ar⁶ in General Formula (2) are each independently the group represented by General Formula (2S), it is preferable that the aromatic hydrocarbon group of Ar⁴ has at least one (preferably 1 to 5) organic group other than an electron-withdrawing group, and it is also preferable that the aromatic hydrocarbon group of Ar⁴ has at least two (preferably 2 to 5) substituents.

In General Formula (2), at least two of Ar⁴, Ar⁵, or Ar⁶ represent groups having different structures. In other words, all of Ar⁴, Ar⁵, and Ar⁶ are not groups having the same structure.

In General Formula (2), the aromatic hydrocarbon ring group represented by each of Ar⁴, Ar⁵, and Ar⁶ has a total of 2 fluorine-containing groups (preferably fluoroalkyl groups).

Furthermore, only the aromatic hydrocarbon ring group represented by Ar⁶ may have 2 or more (for example, 2 or 3) fluorine-containing groups as a substituent, or the aromatic hydrocarbon ring group represented by Ar⁶ may have only one fluorine-containing group as a substituent and the aromatic hydrocarbon ring group represented by Ar⁴ and/or the aromatic hydrocarbon ring group represented by Ar⁵ may have one or more (for example, 1 to 3) fluorine-containing groups as a substituent.

Above all, the compound represented by General Formula (2) preferably satisfies the following condition 1 or condition 2. Furthermore, in a case where the condition 1 or the condition 2 is satisfied, only one of the condition 1 and the condition 2 may be satisfied, or the both may be satisfied.

Condition 1: In General Formula (2), the aromatic hydrocarbon ring group represented by each of Ar⁴, Ar⁵, and Ar⁶ has a total of 3 or more (preferably 3 to 6) fluorine-containing groups (preferably fluoroalkyl groups).

Condition 2: In General Formula (2), the aromatic hydrocarbon ring group represented by each of Ar⁴, Ar⁵, and Ar⁶ has a total of one or more (preferably 1 to 6) linear or branched organic groups other than an electron-withdrawing group, and a total number of carbon atoms included in the total of one or more linear or branched organic groups other than an electron-withdrawing group is 2 or more (preferably 3 or more, and more preferably 3 to 8).

Moreover, the compound represented by General Formula (2) is preferably different from the compound represented by General Formula (1).

For example, in the compound represented by General Formula (2), it is also preferable that the benzene ring group bonded to S⁺ does not have a fluorine-containing group at the ortho position with respect to the bonding position with S⁺.

### (Compound Represented by General Formula (3))

General Formula (3) is shown below.

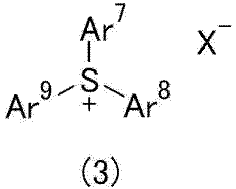

In General Formula (3), X⁻ represents an organic anion.

Examples of the organic anion represented by X⁻ in General Formula (3) include the organic anion represented by X⁻ described with respect to General Formula (1).

In General Formula (3), Ar⁷ and Ar⁸ each independently represent an aromatic hydrocarbon ring group which may have an organic group other than an electron-withdrawing group.

Examples of the aromatic hydrocarbon ring group include a benzene ring group, a naphthalene ring group, and an anthracene ring group. Among those, the aromatic hydrocarbon ring groups are each independently preferably the benzene ring group.

As described above, each of the aromatic hydrocarbon ring groups may each independently have an (preferably 1 to 5) organic group other than an electron-withdrawing group. In other words, the aromatic hydrocarbon ring group of each of Ar⁷ and Ar⁸ does not have a substituent other than the "organic group other than an electron-withdrawing group".

It should be noted that the aromatic hydrocarbon ring group represented by each of Ar⁷ and Ar⁸ has a total of one or more (preferably 1 to 8) organic groups other than an electron-withdrawing group.

Furthermore, examples of the organic group other than an electron-withdrawing group in General Formula (3) (including General Formula (3R)) include the same ones as the organic groups other than an electron-withdrawing group described with respect to General Formula (1) (including General Formula (1R)).

In General Formula (3), Ar⁹ represents an aromatic hydrocarbon ring group (benzene ring group) represented by General Formula (3R).

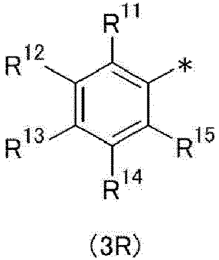

In General Formula (3R), ^{∗} represents a bonding position.

In General Formula (3R), R¹¹, R¹³, and R¹⁵ each independently represent a hydrogen atom, a fluorine atom, or an alkyl group which may have a substituent. The alkyl group which may have a substituent is preferably a fluoroalkyl group.

In General Formula (3R), R¹² and R¹⁴ each independently represent a fluorine-containing group selected from the group consisting of a fluorine atom and a fluoroalkyl group. The fluorine-containing group is preferably the fluoroalkyl group.

Furthermore, examples of the fluoroalkyl group in General Formula (3R) include the same ones as the fluoroalkyl groups which can serve as the fluorine-containing group described with respect to General Formula (1) (including General Formula (1R)).

Above all, in General Formula (3), the aromatic hydrocarbon ring group represented by each of Ar⁷, Ar⁸, and Ar⁹ has a total of one or more (preferably 2 to 5) organic groups other than an electron-withdrawing group, and a total number of carbon atoms included in the total of one or more organic groups other than an electron-withdrawing group is 2 or more (preferably 3 or more, more preferably 3 to 20, and still more preferably 3 to 12).

The aromatic hydrocarbon ring group represented by each of Ar⁷, Ar⁸, and Ar⁹ preferably has a total of 2 to 5 fluorine-containing groups (preferably fluoroalkyl groups).

Moreover, the compound represented by General Formula (3) is preferably different from the compound represented by General Formula (1) and/or the compound represented by General Formula (2).

For example, in the compound represented by General Formula (3), it is also preferable that the benzene ring group bonded to S⁺ does not have a fluorine-containing group at the ortho position and/or the para position with respect to the bonding position with S⁺.

The organic cations (portions other than X- in General Formulae (1) to (3)) which can be contained in the specific compound are exemplified below.

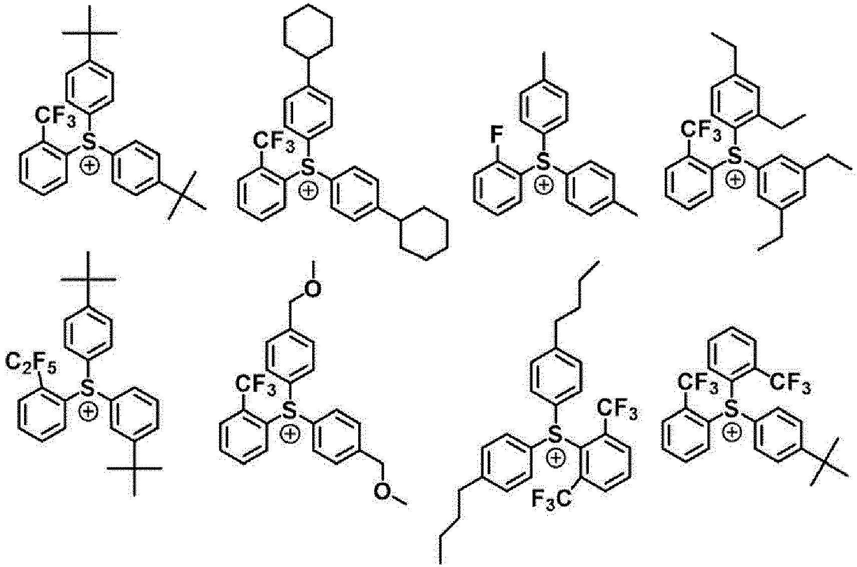

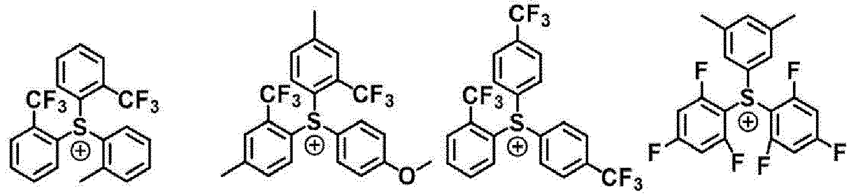

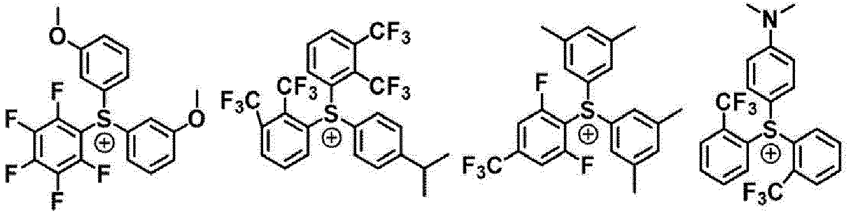

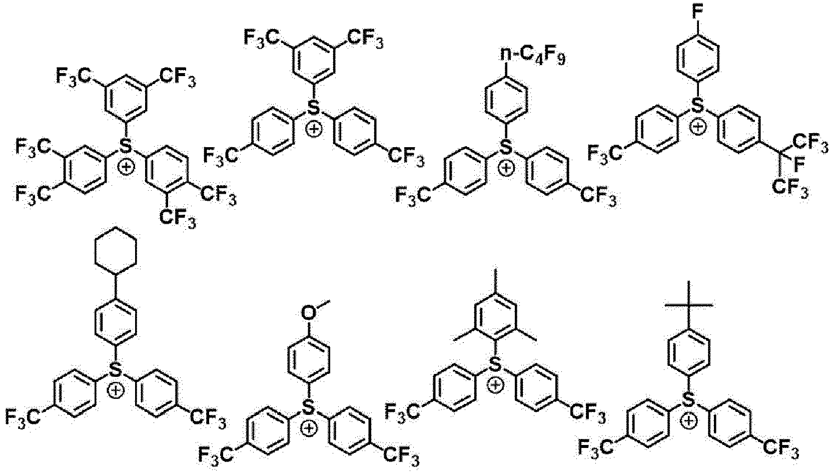

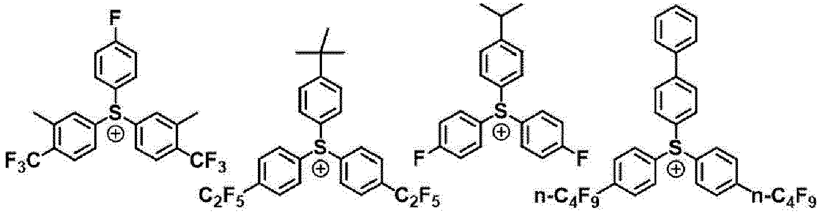

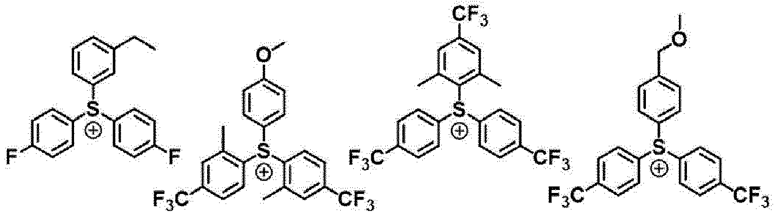

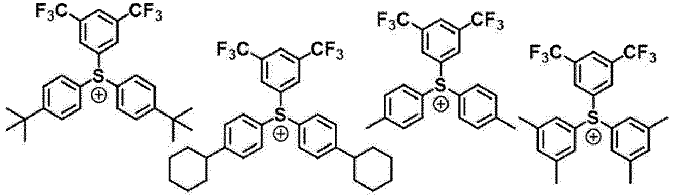

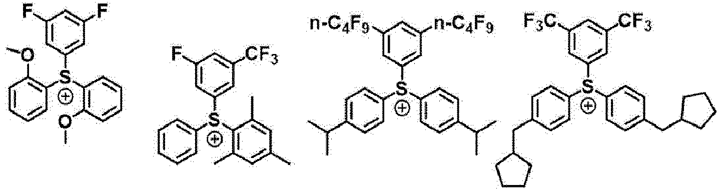

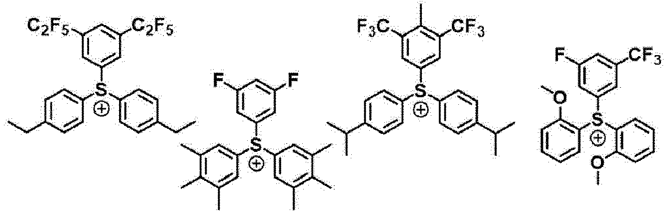

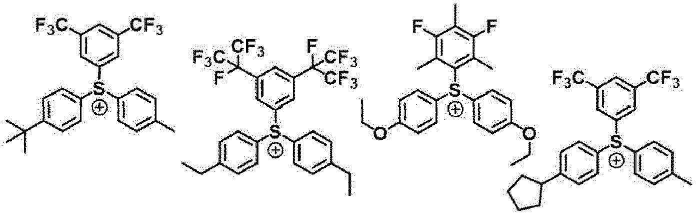

In the resist composition, a content of the specific compound is preferably 5% by mass or more, more preferably 9% by mass or more, and still more preferably 10% by mass with respect to a total solid content of the composition from the viewpoint that the effect of the present invention is more excellent. Moreover, the content is preferably 40% by mass or less, more preferably 35% by mass or less, and still more preferably 33% by mass or less.

In addition, in a case where the specific compound includes a specific compound having an anion represented by any of Formulae (d1-1) to (d1-3), a content of the specific compound is preferably 1% to 30% by mass, and more preferably 3% to 20% by mass in the total solid content.

In a case where a specific compound having an anion (for example, anions represented by Formulae (AN1) to (AN5), a disulfonamide anion, and a perfluoroaliphatic sulfonate anion) other than the anion represented by any of Formulae (d1-1) to (d1-3) is included as the specific compound, the content of the specific compound is preferably 3% to 35% by mass, and more preferably 10% to 25% by mass in the total solid content.

The specific compounds may be used alone or in combination of two or more kinds thereof.

Furthermore, the solid content means all the components excluding a solvent which will be described.

### <Acid-Decomposable Resin (Resin (A))>

The composition of the embodiment of the present invention includes a resin (hereinafter also referred to as an "acid-decomposable resin" or a "resin (A)") having a polarity that increases due to decomposition by the action of an acid.

That is, in the pattern forming method of an embodiment of the present invention which will be described later, typically, in a case where an alkali developer is adopted as the developer, a positive tone pattern is suitably formed, and in a case where an organic developer is adopted as the developer, a negative tone pattern is suitably formed.

The resin (A) usually includes a repeating unit having a group having a polarity that increases due to decomposition by the action of an acid (hereinafter also referred to as an "acid-decomposable group"), and preferably includes a repeating unit having an acid-decomposable group.

### (Repeating Unit Having Acid-Decomposable Group)

The acid-decomposable group is a group that decomposes by the action of an acid to produce a polar group. The acid-decomposable group preferably has a structure in which the polar group is protected by an eliminable group that is eliminated by the action of an acid. That is, the resin (A) has a repeating unit having a group that decomposes by the action of an acid to produce a polar group. A resin having this repeating unit has an increased polarity by the action of an acid, and thus has an increased solubility in an alkali developer, and a decreased solubility in an organic solvent.

As the polar group, an alkali-soluble group is preferable, and examples thereof include an acidic group such as a carboxyl group, a phenolic hydroxyl group, a fluorinated alcohol group, a sulfonic acid group, a phosphoric acid group, a sulfonamide group, a sulfonylimide group, an (alkylsulfonyl)(alkylcarbonyl)methylene group, an (alkylsulfonyl)(alkylcarbonyl)imide group, a bis(alkylcarbonyl)methylene group, a bis(alkylcarbonyl)imide group, a bis(alkylsulfonyl)methylene group, a bis(alkylsulfonyl)imide group, a tris(alkylcarbonyl)methylene group, and a tris(alkylsulfonyl)methylene group, and an alcoholic hydroxyl group.

Among those, as the polar group, the carboxyl group, the phenolic hydroxyl group, the fluorinated alcohol group (preferably a hexafluoroisopropanol group), or the sulfonic acid group is preferable.

Examples of the eliminable group that is eliminated by the action of an acid include groups represented by Formulae (Y1) to (Y4).

Formula (Y1): -C(Rx₁)(Rx₂)(Rx₃) Formula (Y2): -C(=O)OC(Rx₁)(Rx₂)(Rx₃) Formula (Y3): -C(R₃₆)(R₃₇)(OR₃₈) Formula (Y4): -C(Rn)(H)(Ar)

In Formula (Y1) and Formula (Y2), Rx₁ to Rx₃ each independently represent an (linear or branched) alkyl group or (monocyclic or polycyclic) cycloalkyl group, an (linear or branched) alkenyl group, or an (monocyclic or polycyclic) aryl group. Furthermore, in a case where all of Rxi to Rx₃ are (linear or branched) alkyl groups, it is preferable that at least two of Rx₁, Rx₂, or Rx₃ are methyl groups.

Above all, it is preferable that Rxi to Rx₃ each independently represent a linear or branched alkyl group, and it is more preferable that Rxi to Rx₃ each independently represent the linear alkyl group.

Two of Rxi to Rx₃ may be bonded to each other to form a monocycle or a polycycle.

As the alkyl group of each of Rxi to Rx₃, an alkyl group having 1 to 5 carbon atoms, such as a methyl group, an ethyl group, an n-propyl group, an isopropyl group, an n-butyl group, an isobutyl group, and a t-butyl group, is preferable.

As the cycloalkyl group of each of Rxi to Rx₃, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

As the aryl group of each of Rxi to Rx₃, an aryl group having 6 to 10 carbon atoms is preferable, and examples thereof include a phenyl group, a naphthyl group, and an anthryl group.

As the alkenyl group of each of Rx₁ to Rx₃, a vinyl group is preferable.

As a ring formed by the bonding of two of Rx₁ to Rx₃, a cycloalkyl group is preferable. As the cycloalkyl group formed by the bonding of two of Rx₁ to Rx₃, a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group is preferable, and a monocyclic cycloalkyl group having 5 or 6 carbon atoms is more preferable.

In the cycloalkyl group formed by the bonding of two of Rxi to Rx₃, for example, one of the methylene groups constituting the ring may be substituted with a heteroatom such as an oxygen atom, a group having a heteroatom, such as a carbonyl group, or a vinylidene group. In addition, in the cycloalkyl group, one or more of the ethylene groups constituting the cycloalkane ring may be substituted with a vinylene group.

With regard to the group represented by Formula (Y1) or Formula (Y2), for example, an aspect in which Rxi is a methyl group or an ethyl group, and Rx₂ and Rx₃ are bonded to each other to form a cycloalkyl group is preferable.

In Formula (Y3), R₃₆ to R₃₈ each independently represent a hydrogen atom or a monovalent organic group. R₃₇ and R₃₈ may be bonded to each other to form a ring. Examples of the monovalent organic group include an alkyl group, a cycloalkyl group, an aryl group, an aralkyl group, and an alkenyl group. It is also preferable that R₃₆ is the hydrogen atom.

Furthermore, the alkyl group, the cycloalkyl group, the aryl group, and the aralkyl group may include a heteroatom such as an oxygen atom, and/or a group having a heteroatom, such as a carbonyl group. For example, in the alkyl group, the cycloalkyl group, the aryl group, and the aralkyl group, one or more of the methylene groups may be substituted with a heteroatom such as an oxygen atom and/or a group having a heteroatom, such as a carbonyl group.

In addition, R₃₈ and another substituent contained in the main chain of the repeating unit may be bonded to each other to form a ring. A group formed by the mutual bonding of R₃₈ and another substituent on the main chain of the repeating unit is preferably an alkylene group such as a methylene group.

As Formula (Y3), a group represented by Formula (Y3-1) is preferable.

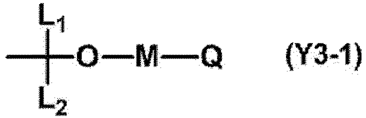

Here, L₁ and L₂ each independently represent a hydrogen atom, an alkyl group, a cycloalkyl group, an aryl group, or a group formed by combination thereof (for example, a group formed by combination of an alkyl group and an aryl group).

M represents a single bond or a divalent linking group.

Q represents an alkyl group which may include a heteroatom, a cycloalkyl group which may include a heteroatom, an aryl group which may include a heteroatom, an amino group, an ammonium group, a mercapto group, a cyano group, an aldehyde group, or a group formed by combination thereof (for example, a group formed by combination of an alkyl group and a cycloalkyl group).

In the alkyl group and the cycloalkyl group, for example, one of the methylene groups may be substituted with a heteroatom such as an oxygen atom or a group having a heteroatom, such as a carbonyl group.

In addition, it is preferable that one of L₁ or L₂ is a hydrogen atom, and the other is an alkyl group, a cycloalkyl group, an aryl group, or a group formed by combination of an alkylene group and an aryl group.

At least two of Q, M, or L₁ may be bonded to each other to form a ring (preferably a 5- or 6-membered ring).

From the viewpoint of pattern miniaturization, L₂ is preferably a secondary or tertiary alkyl group, and more preferably the tertiary alkyl group. Examples of the secondary alkyl group include an isopropyl group, a cyclohexyl group, and a norbornyl group, and examples of the tertiary alkyl group include a tert-butyl group and an adamantane group. In these aspects, since the glass transition temperature (Tg) and the activation energy are increased, it is possible to suppress fogging in addition to ensuring film hardness.

In Formula (Y4), Ar represents an aromatic ring group. Rn represents an alkyl group, a cycloalkyl group, or an aryl group. Rn and Ar may be bonded to each other to form a non-aromatic ring. Ar is more preferably the aryl group.

From the viewpoint that the acid decomposability of the repeating unit is excellent, in a case where a non-aromatic ring is directly bonded to a polar group (or a residue thereof) in an eliminable group that protects the polar group, it is also preferable that a ring member atom adjacent to the ring member atom directly bonded to the polar group (or a residue thereof) in the non-aromatic ring has no halogen atom such as a fluorine atom as a substituent.

In addition, the eliminable group that is eliminated by the action of an acid may be a 2-cyclopentenyl group having a substituent (an alkyl group and the like), such as a 3-methyl-2-cyclopentenyl group, and a cyclohexyl group having a substituent (an alkyl group and the like), such as a 1,1,4,4-tetramethylcyclohexyl group.

As the repeating unit having an acid-decomposable group, a repeating unit represented by Formula (A) is also preferable.

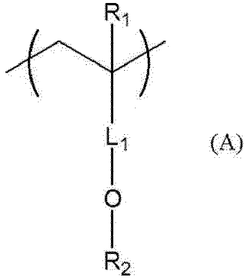

L₁ represents a divalent linking group which may have a fluorine atom or an iodine atom, R₁ represents a hydrogen atom, a fluorine atom, an iodine atom, a fluorine atom, an alkyl group which may have an iodine atom, or an aryl group which may have a fluorine atom or an iodine atom, and R₂ represents an eliminable group that is eliminated by the action of an acid and may have a fluorine atom or an iodine atom. It should be noted that at least one of L₁, R₁, or R₂ has a fluorine atom or an iodine atom.

L₁ represents a divalent linking group which may have a fluorine atom or an iodine atom. Examples of the divalent linking group which may have a fluorine atom or an iodine atom include -CO-, -O-, -S-, -SO-, -SO₂-, a hydrocarbon group which may have a fluorine atom or an iodine atom (for example, an alkylene group, a cycloalkylene group, an alkenylene group, and an arylene group), and a linking group formed by the linking of a plurality of these groups. Among those, as Li, -CO- or -arylene group-alkylene group having a fluorine atom or an iodine atom- is preferable.

As the arylene group, a phenylene group is preferable.

The alkylene group may be linear or branched. The number of carbon atoms of the alkylene group is not particularly limited, but is preferably 1 to 10, and more preferably 1 to 3.

The total number of fluorine atoms and iodine atoms included in the alkylene group having a fluorine atom or an iodine atom is not particularly limited, but is preferably 2 or more, more preferably 2 to 10, and still more preferably 3 to 6.

R₁ represents a hydrogen atom, a fluorine atom, an iodine atom, an alkyl group which may have a fluorine atom or an iodine atom, or an aryl group which may have a fluorine atom or an iodine atom.

The alkyl group may be linear or branched. The number of carbon atoms of the alkyl group is not particularly limited, but is preferably 1 to 10, and more preferably 1 to 3.

The total number of fluorine atoms and iodine atoms included in the alkyl group having a fluorine atom or an iodine atom is not particularly limited, but is preferably 1 or more, more preferably 1 to 5, and still more preferably 1 to 3.

The alkyl group may include a heteroatom such as an oxygen atom, other than a halogen atom.

R₂ represents an eliminable group that is eliminated by the action of an acid and may have a fluorine atom or an iodine atom.

Among those, examples of the eliminable group include groups represented by Formulae (Z1) to (Z4).

Formula (Z1): -C(Rx₁₁)(Rx₁₂)(Rx₁₃) Formula (Z2): -C(=O)OC(Rx₁₁)(Rx₁₂)(Rx₁₃) Formula (Z3): -C(R₁₃₆)(R₁₃₇)(OR₁₃₈) Formula (Z4): -C(Rni)(H)(An)

In Formulae (Z1) and (Z2), Rx₁₁ to Rx₁₃ each independently represent an (linear or branched) alkyl group which may have a fluorine atom or an iodine atom, a (monocyclic or polycyclic) cycloalkyl group which may have a fluorine atom or an iodine atom, an (linear or branched) alkenyl group which may have a fluorine atom or an iodine atom, or an (monocyclic or polycyclic) aryl group which may have a fluorine atom or an iodine atom. Furthermore, in a case where all of Rxn to Rx₁₃ are each an (linear or branched) alkyl group, it is preferable that at least two of Rx₁₁, Rx₁₂, or Rx₁₃ are methyl groups.

Rxn to Rx₁₃ are the same as Rx₁ to Rx₃ in formulae (Y1) and (Y2) described above, respectively, except that they may have a fluorine atom or an iodine atom, and have the same definitions and suitable ranges as those of the alkyl group, the cycloalkyl group, the alkenyl group, and the aryl group.

In Formula (Z3), R₁₃₆ to R₁₃₈ each independently represent a hydrogen atom, or a monovalent organic group which may have a fluorine atom or an iodine atom. R₁₃₇ and R₁₃₈ may be bonded to each other to form a ring. Examples of the monovalent organic group which may have a fluorine atom or an iodine atom include an alkyl group which may have a fluorine atom or an iodine atom, a cycloalkyl group which may have a fluorine atom or an iodine atom, an aryl group which may have a fluorine atom or an iodine atom, an aralkyl group which may have a fluorine atom or an iodine atom, and a group formed by combination thereof (for example, a group formed by combination of the alkyl group and the cycloalkyl group).

Incidentally, the alkyl group, the cycloalkyl group, the aryl group, and the aralkyl group may include a heteroatom such as an oxygen atom, in addition to the fluorine atom and the iodine atom. That is, in the alkyl group, the cycloalkyl group, the aryl group, and the aralkyl group, for example, one of the methylene groups may be substituted with a heteroatom such as an oxygen atom or a group having a heteroatom, such as a carbonyl group.

In addition, R₁₃₈ and another substituent contained in the main chain of the repeating unit may be bonded to each other to form a ring. In this case, a group formed by the mutual bonding of R₁₃₈ and another substituent on the main chain of the repeating unit is preferably an alkylene group such as a methylene group.

As Formula (Z3), a group represented by Formula (Z3-1) is preferable.

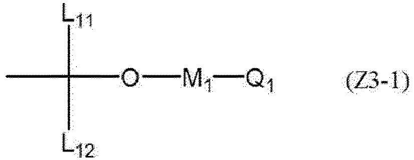

Here, L₁₁ and L₁₂ each independently represent a hydrogen atom; an alkyl group which may have a heteroatom selected from the group consisting of a fluorine atom, an iodine atom, and an oxygen atom; a cycloalkyl group which may have a heteroatom selected from the group consisting of a fluorine atom, an iodine atom, and an oxygen atom; an aryl group which may have a heteroatom selected from the group consisting of a fluorine atom, an iodine atom, and an oxygen atom; or a group formed by combination thereof (for example, a group formed by combination of an alkyl group and a cycloalkyl group, each of which may have a heteroatom selected from the group consisting of a fluorine atom, an iodine atom, and an oxygen atom).

Mi represents a single bond or a divalent linking group.

Qi represents an alkyl group which may have a heteroatom selected from the group consisting of a fluorine atom, an iodine atom, and an oxygen atom; a cycloalkyl group which may have a heteroatom selected from the group consisting of a fluorine atom, an iodine atom, and an oxygen atom; an aryl group which may have a heteroatom selected from the group consisting of a fluorine atom, an iodine atom, and an oxygen atom; an amino group; an ammonium group; a mercapto group; a cyano group; an aldehyde group; a group formed by combination thereof (for example, a group formed by combination of the alkyl group and the cycloalkyl group, each of which may have a heteroatom selected from the group consisting of a fluorine atom, an iodine atom, and an oxygen atom).

In Formula (Y4), An represents an aromatic ring group which may have a fluorine atom or an iodine atom. Rn₁ is an alkyl group which may have a fluorine atom or an iodine atom, a cycloalkyl group which may have a fluorine atom or an iodine atom, or an aryl group which may have a fluorine atom or an iodine atom. Rn₁ and Ar₁ may be bonded to each other to form a non-aromatic ring.

As the repeating unit having an acid-decomposable group, a repeating unit represented by General Formula (AI) is also preferable.

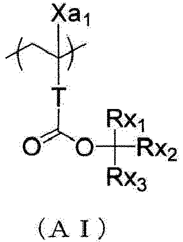

In General Formula (AI),

Xa₁ represents a hydrogen atom, or an alkyl group which may have a substituent.

T represents a single bond or a divalent linking group.

Rxi to Rx₃ each independently represent an (linear or branched) alkyl group, a (monocyclic or polycyclic) cycloalkyl group, an (linear or branched) alkenyl group, or an (monocyclic or polycyclic) aryl group. It should be noted that in a case where all of Rxi to Rx₃ are (linear or branched) alkyl groups, it is preferable that at least two of Rx₁, Rx₂, or Rx₃ are methyl groups.

Two of Rxi to Rx₃ may be bonded to each other to form a monocycle or polycycle (a monocyclic or polycyclic cycloalkyl group and the like).

Examples of the alkyl group which may have a substituent, represented by Xai, include a methyl group and a group represented by -CH₂-R₁₁. R₁₁ represents a halogen atom (a fluorine atom or the like), a hydroxyl group, or a monovalent organic group, examples thereof include an alkyl group having 5 or less carbon atoms, which may be substituted with a halogen atom, an acyl group having 5 or less carbon atoms, which may be substituted with a halogen atom, and an alkoxy group having 5 or less carbon atoms, which may be substituted with a halogen atom; and an alkyl group having 3 or less carbon atoms is preferable, and a methyl group is more preferable. Xai is preferably a hydrogen atom, a methyl group, a trifluoromethyl group, or a hydroxymethyl group.

Examples of the divalent linking group of T include an alkylene group, an aromatic ring group, a -COO-Rt- group, and an -O-Rt- group. In the formulae, Rt represents an alkylene group or a cycloalkylene group.

T is preferably the single bond or the -COO-Rt- group. In a case where T represents the -COO-Rt-group, Rt is preferably an alkylene group having 1 to 5 carbon atoms, and more preferably a -CH₂- group, a -(CH₂)₂- group, or a -(CH₂)₃- group.

As the alkyl group of each of Rxi to Rx₃, an alkyl group having 1 to 4 carbon atoms, such as a methyl group, an ethyl group, an n-propyl group, an isopropyl group, an n-butyl group, an isobutyl group, and a t-butyl group, is preferable.

As the cycloalkyl group of each of Rxi to Rx₃, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

As the aryl group of each of Rxi to Rx₃, an aryl group having 6 to 10 carbon atoms is preferable, and examples thereof include a phenyl group, a naphthyl group, and an anthryl group.

As the alkenyl group of each of Rxi to Rx₃, a vinyl group is preferable.

As the cycloalkyl group formed by the bonding of two of Rxi to Rx₃, a monocyclic cycloalkyl group such as a cyclopentyl group and a cyclohexyl group is preferable, and in addition, a polycyclic cycloalkyl group such as a norbornyl group, a tetracyclodecanyl group, a tetracyclododecanyl group, and an adamantyl group is also preferable. Among those, a monocyclic cycloalkyl group having 5 or 6 carbon atoms is preferable.

In the cycloalkyl group formed by the bonding of two of Rxi to Rx₃, for example, one of the methylene groups constituting the ring may be substituted with a heteroatom such as an oxygen atom, a group having a heteroatom, such as a carbonyl group, or a vinylidene group. In addition, in the cycloalkyl group, one or more of the ethylene groups constituting the cycloalkane ring may be substituted with a vinylene group.

With regard to the repeating unit represented by General Formula (AI), for example, an aspect in which Rxi is a methyl group or an ethyl group, and Rx₂ and Rx₃ are bonded to each other to form the above-mentioned cycloalkyl group is preferable.

In a case where each of the groups has a substituent, examples of the substituent include an alkyl group (having 1 to 4 carbon atoms), a halogen atom, a hydroxyl group, an alkoxy group (having 1 to 4 carbon atoms), a carboxyl group, and an alkoxycarbonyl group (having 2 to 6 carbon atoms). The substituent preferably has 8 or less carbon atoms.

The repeating unit represented by General Formula (AI) is preferably an acid-decomposable tertiary alkyl (meth)acrylate ester-based repeating unit (the repeating unit in which Xa₁ represents a hydrogen atom or a methyl group, and T represents a single bond).

A content of the repeating unit having an acid-decomposable group is preferably 15% to 80% by mole, more preferably 20% to 70% by mole, and still more preferably 20% to 65% by mole with respect to all repeating units in the resin (A).

Specific examples of the repeating unit having an acid-decomposable group are shown below, but the present invention is not limited thereto. Furthermore, in the formulae, Xai represents H, F, CH₃, CF₃, or CH₂OH, and Rxa and Rxb each represent a linear or branched alkyl group having 1 to 5 carbon atoms.

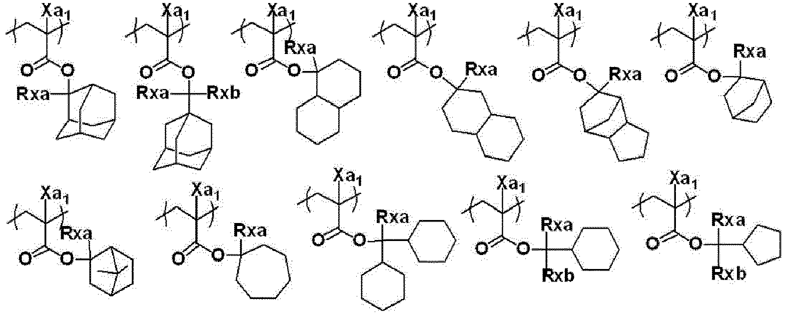

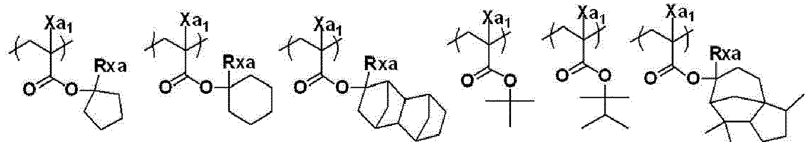

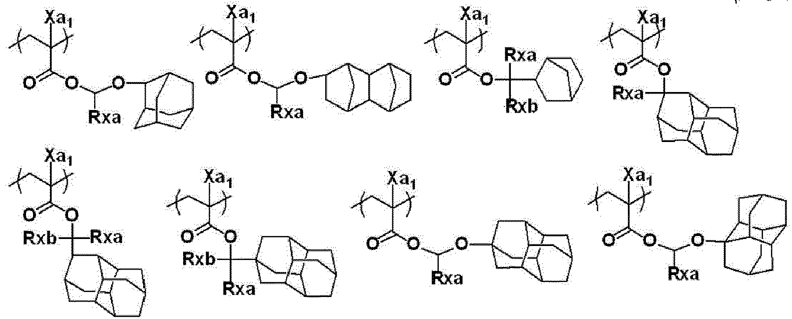

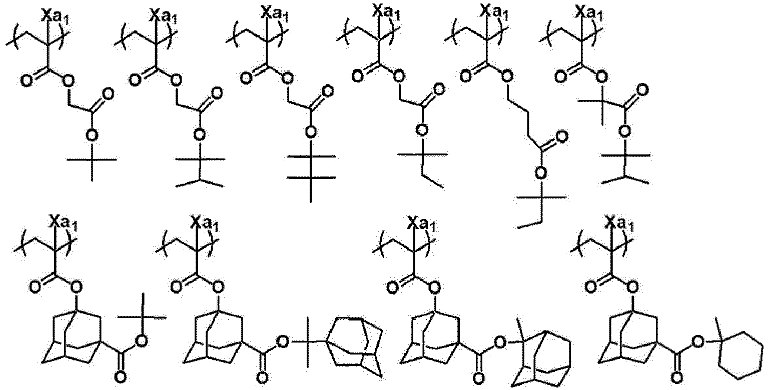

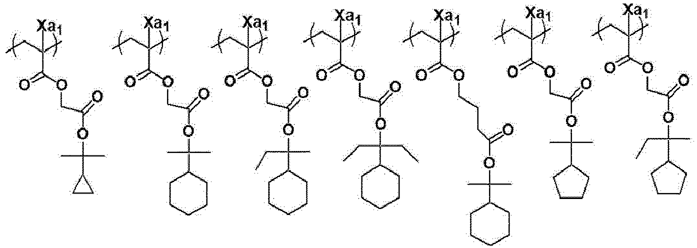

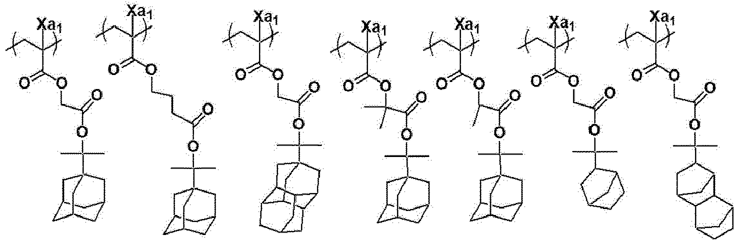

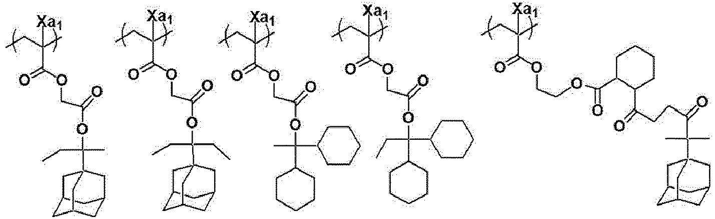

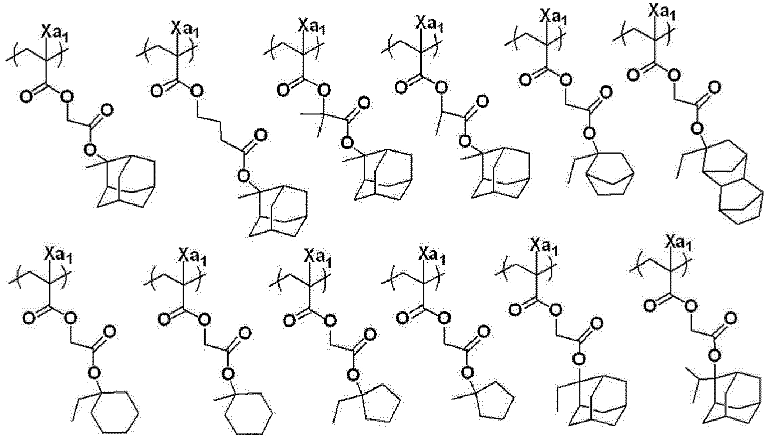

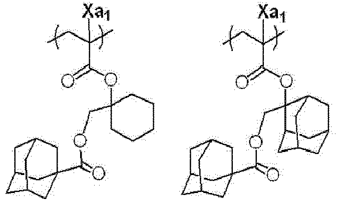

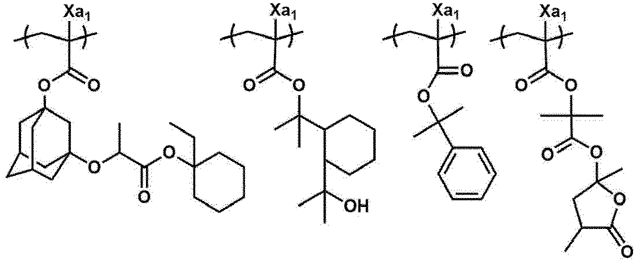

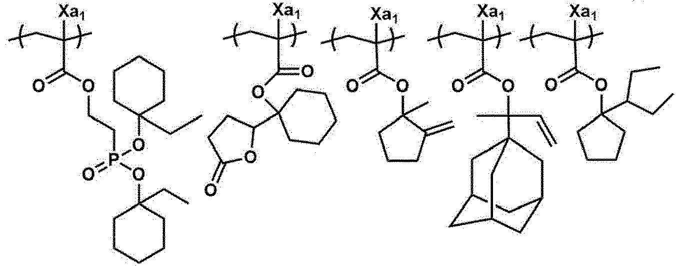

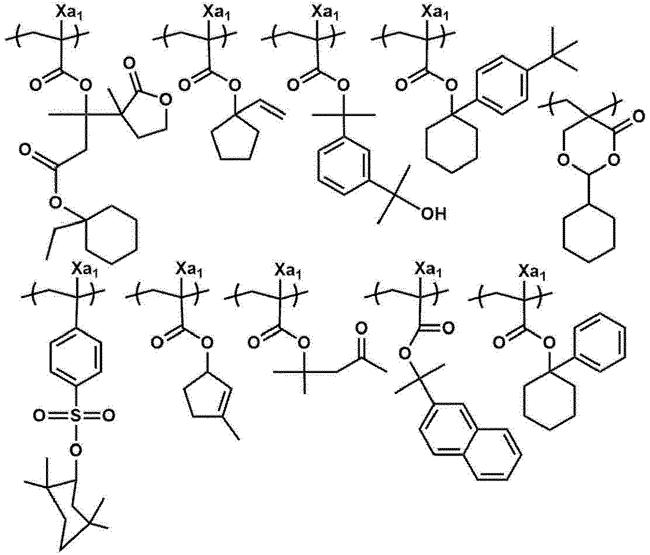

The resin (A) may include a repeating unit other than the above-mentioned repeating units.

For example, the resin (A) may include at least one repeating unit selected from the group consisting of the following group A and/or at least one repeating unit selected from the group consisting of the following group B.

Group A: A group consisting of the following repeating units (20) to (29).
(20) A repeating unit having an acid group, which will be described later
(21) A repeating unit having a fluorine atom or an iodine atom, which will be described later
(22) A repeating unit having a lactone group, a sultone group, or a carbonate group, which will be described later
(23) A repeating unit having a photoacid generating group, which will be described later
(24) A repeating unit represented by General Formula (V-1) or General Formula (V-2), which will be described later
(25) A repeating unit represented by Formula (A), which will be described later
(26) A repeating unit represented by Formula (B), which will be described later
(27) A repeating unit represented by Formula (C), which will be described later
(28) A repeating unit represented by Formula (D), which will be described later
(29) A repeating unit group B represented by Formula (E), which will be described later: a group consisting of the following repeating units (30) to (32).
(30) A repeating unit having at least one group selected from a lactone group, a sultone group, a carbonate group, a hydroxyl group, a cyano group, or an alkali-soluble group, which will be described later
(31) A repeating unit having an alicyclic hydrocarbon structure and not exhibiting acid decomposability described later
(32) A repeating unit represented by General Formula (III) having neither a hydroxyl group nor a cyano group, which will be described later

In a case where the composition of the embodiment of the present invention is used as an actinic ray-sensitive or radiation-sensitive resin composition for EUV, it is preferable that the resin (A) has at least one repeating unit selected from the group consisting of the group A.

Furthermore, in a case where the composition is used as the actinic ray-sensitive or radiation-sensitive resin composition for EUV, it is preferable that the resin (A) includes at least one of a fluorine atom or an iodine atom. In a case where the resin (A) includes both a fluorine atom and an iodine atom, the resin (A) may have one repeating unit including both a fluorine atom and an iodine atom, and the resin (A) may include two kinds of repeating units, that is, a repeating unit having a fluorine atom and a repeating unit having an iodine atom.

In addition, in a case where the composition is used as an actinic ray-sensitive or radiation-sensitive resin composition for EUV, it is also preferable that the resin (A) has a repeating unit having an aromatic group.

In a case where the composition of the embodiment of the present invention is used as an actinic ray-sensitive or radiation-sensitive resin composition for ArF, it is preferable that the resin (A) has at least one repeating unit selected from the group consisting of the group B.

Furthermore, in a case where the composition of the embodiment of the present invention is used as the actinic ray-sensitive or radiation-sensitive resin composition for ArF, it is preferable that the resin (A) includes neither a fluorine atom nor a silicon atom.

In addition, in a case where the composition is used as the actinic ray-sensitive or radiation-sensitive resin composition for ArF, it is preferable that the resin (A) does not have an aromatic group.

### (Repeating Unit Having Acid Group)

The resin (A) may have a repeating unit having an acid group.

As the acid group, an acid group having a pKa of 13 or less is preferable.

As the acid group, for example, a carboxyl group, a phenolic hydroxyl group, a fluorinated alcohol group (preferably a hexafluoroisopropanol group), a sulfonic acid group, a sulfonamide group, or an isopropanol group is preferable.

In addition, in the hexafluoroisopropanol group, one or more (preferably one or two) fluorine atoms may be substituted with a group (an alkoxycarbonyl group and the like) other than a fluorine atom. -C(CF₃)(OH)-CF₂- formed as above is also preferable as the acid group. In addition, one or more fluorine atoms may be substituted with a group other than a fluorine atom to form a ring including -C(CF₃)(OH)-CF₂-.

The repeating unit having an acid group is preferably a repeating unit different from a repeating unit having the structure in which a polar group is protected by the eliminable group that is eliminated by the action of an acid as described above, and a repeating unit having a lactone group, a sultone group, or a carbonate group which will be described later.

The repeating unit having an acid group may have a fluorine atom or an iodine atom.

As the repeating unit having an acid group, a repeating unit represented by Formula (B) is preferable.

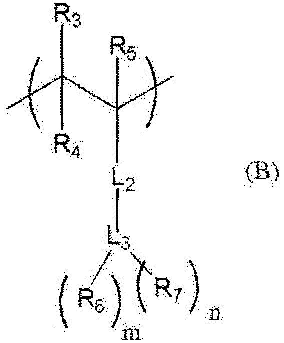

R₃ represents a hydrogen atom or a monovalent organic group which may have a fluorine atom or an iodine atom.

The monovalent organic group which may have a fluorine atom or an iodine atom is preferably a group represented by -L₄-R₈. L₄ represents a single bond or an ester group. R₈ is an alkyl group which may have a fluorine atom or an iodine atom, a cycloalkyl group which may have a fluorine atom or an iodine atom, an aryl group which may have a fluorine atom or an iodine atom, or a group formed by combination thereof.

R₄ and R₅ each independently represent a hydrogen atom, a fluorine atom, an iodine atom, or an alkyl group which may have a fluorine atom or an iodine atom.

L₂ represents a single bond or an ester group.

L₃ represents an (n + m + 1)-valent aromatic hydrocarbon ring group or an (n + m + 1)-valent alicyclic hydrocarbon ring group. Examples of the aromatic hydrocarbon ring group include a benzene ring group and a naphthalene ring group. The alicyclic hydrocarbon ring group may be either a monocycle or a polycycle, and examples thereof include a cycloalkyl ring group.

R₆ represents a hydroxyl group or a fluorinated alcohol group (preferably a hexafluoroisopropanol group). Furthermore, in a case where R₆ is a hydroxyl group, L₃ is preferably the (n + m + 1)-valent aromatic hydrocarbon ring group.

R₇ represents a halogen atom. Examples of the halogen atom include a fluorine atom, a chlorine atom, a bromine atom, or an iodine atom.

m represents an integer of 1 or more. m is preferably an integer of 1 to 3 and more preferably an integer of 1 or 2.

n represents 0 or an integer of 1 or more. n is preferably an integer of 1 to 4.

Furthermore, (n + m + 1) is preferably an integer of 1 to 5.

As the repeating unit having an acid group, a repeating unit represented by General Formula (I) is also preferable.

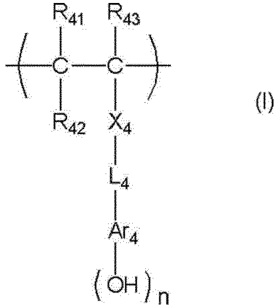

In General Formula (I),

R₄₁, R₄₂, and R₄₃ each independently represent a hydrogen atom, an alkyl group, a cycloalkyl group, a halogen atom, a cyano group, or an alkoxycarbonyl group. It should be noted that R₄₂ may be bonded to Ar₄ to form a ring, in which case R₄₂ represents a single bond or an alkylene group.

X₄ represents a single bond, -COO-, or -CONR₆₄-, and R₆₄ represents a hydrogen atom or an alkyl group.

L₄ represents a single bond or an alkylene group.

Ar₄ represents an (n + 1)-valent aromatic ring group, and in a case where Ar₄ is bonded to R₄₂ to form a ring, Ar₄ represents an (n + 2)-valent aromatic ring group.

n represents an integer of 1 to 5.

As the alkyl group represented by each of R₄₁, R₄₂, and R₄₃ in General Formula (I), an alkyl group having 20 or less carbon atoms, such as a methyl group, an ethyl group, a propyl group, an isopropyl group, an n-butyl group, a sec-butyl group, a hexyl group, a 2-ethylhexyl group, an octyl group, and a dodecyl group is preferable, an alkyl group having 8 or less carbon atoms is more preferable, and an alkyl group having 3 or less carbon atoms is still more preferable.

The cycloalkyl group of each of R₄₁, R₄₂, and R₄₃ in General Formula (I) may be monocyclic or polycyclic. Among those, a monocyclic cycloalkyl group having 3 to 8 carbon atoms, such as a cyclopropyl group, a cyclopentyl group, and a cyclohexyl group, is preferable.

Examples of the halogen atom of each of R₄₁, R₄₂, and R₄₃ in General Formula (I) include a fluorine atom, a chlorine atom, a bromine atom, and an iodine atom, and the fluorine atom is preferable.

As the alkyl group included in the alkoxycarbonyl group of each of R₄₁, R₄₂, and R₄₃ in General Formula (I), the same ones as the alkyl group in each of R₄₁, R₄₂, and R₄₃ are preferable.

Preferred examples of the substituent in each of the groups include an alkyl group, a cycloalkyl group, an aryl group, an amino group, an amide group, a ureide group, a urethane group, a hydroxyl group, a carboxyl group, a halogen atom, an alkoxy group, a thioether group, an acyl group, an acyloxy group, an alkoxycarbonyl group, a cyano group, and a nitro group. The substituent preferably has 8 or less carbon atoms.

Ar₄ represents an (n + 1)-valent aromatic ring group. The divalent aromatic ring group in a case where n is 1 is preferably for example, an arylene group having 6 to 18 carbon atoms, such as a phenylene group, a tolylene group, a naphthylene group, and an anthracenylene group, or a divalent aromatic ring group including a heterocyclic ring such as a thiophene ring, a furan ring, a pyrrole ring, a benzothiophene ring, a benzofuran ring, a benzopyrrole ring, a triazine ring, an imidazole ring, a benzimidazole ring, a triazole ring, a thiadiazole ring, and a thiazole ring. Furthermore, the aromatic ring group may have a substituent.

Specific examples of the (n + 1)-valent aromatic ring group in a case where n is an integer of 2 or more include groups formed by removing any (n - 1) hydrogen atoms from the above-described specific examples of the divalent aromatic ring group.

The (n + 1)-valent aromatic ring group may further have a substituent.

Examples of the substituent which can be contained in the alkyl group, the cycloalkyl group, the alkoxycarbonyl group, the alkylene group, and the (n + 1)-valent aromatic ring group, each mentioned above, include the alkyl groups; the alkoxy groups such as a methoxy group, an ethoxy group, a hydroxyethoxy group, a propoxy group, a hydroxypropoxy group, and a butoxy group; the aryl groups such as a phenyl group; and the like, as mentioned for each of R₄₁, R₄₂, and R₄₃ in General Formula (I).

Examples of the alkyl group of R₆₄ in -CONR₆₄- represented by X₄ (R₆₄ represents a hydrogen atom or an alkyl group) include an alkyl group having 20 or less carbon atoms, such as a methyl group, an ethyl group, a propyl group, an isopropyl group, an n-butyl group, a sec-butyl group, a hexyl group, a 2-ethylhexyl group, an octyl group, and a dodecyl group, and an alkyl group having 8 or less carbon atoms, is preferable.

As X₄, a single bond, -COO-, or -CONH- is preferable, and the single bond or -COO-is more preferable.

As the alkylene group in L₄, an alkylene group having 1 to 8 carbon atoms, such as a methylene group, an ethylene group, a propylene group, a butylene group, a hexylene group, and an octylene group, is preferable.

As Ar₄, an aromatic ring group having 6 to 18 carbon atoms is preferable, and a benzene ring group, a naphthalene ring group, and a biphenylene ring group are more preferable.

The repeating unit represented by General Formula (I) preferably comprises a hydroxystyrene structure. That is, Ar₄ is preferably the benzene ring group.

The repeating unit represented by General Formula (I) is preferably a repeating unit represented by General Formula (1).

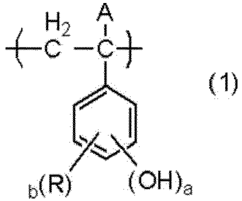

In General Formula (1),

A represents a hydrogen atom, an alkyl group, a cycloalkyl group, a halogen atom, or a cyano group.

R represents a halogen atom, an alkyl group, a cycloalkyl group, an aryl group, an alkenyl group, an aralkyl group, an alkoxy group, an alkylcarbonyloxy group, an alkylsulfonyloxy group, an alkyloxycarbonyl group, or an aryloxycarbonyl group, and in a case where a plurality of R's are present, R's may be the same as or different from each other. In a case where there are a plurality of R's, R's may be bonded to each other to form a ring. As R, the hydrogen atom is preferable.

a represents an integer of 1 to 3.

b represents an integer of 0 to (5-a).

The repeating unit having an acid group is exemplified below. In the formulae, a represents 1 or 2.

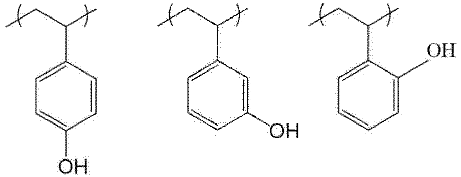

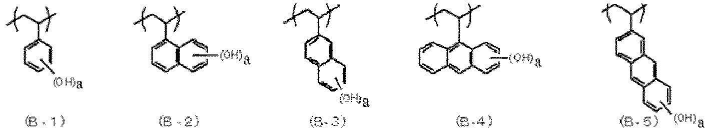

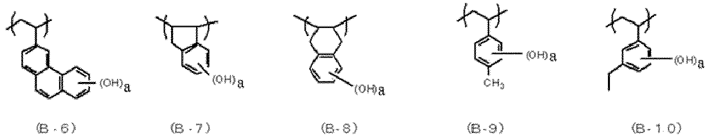

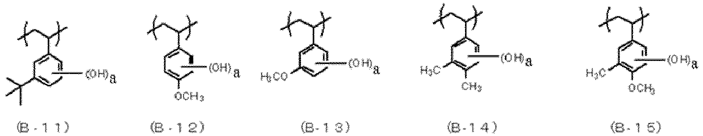

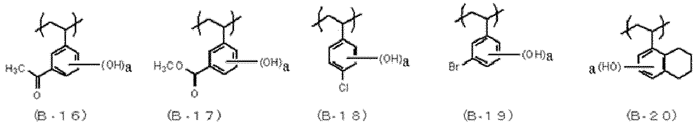

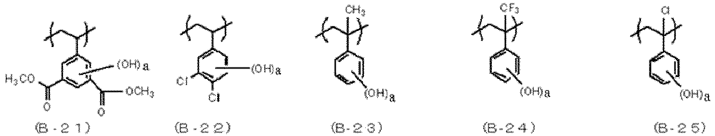

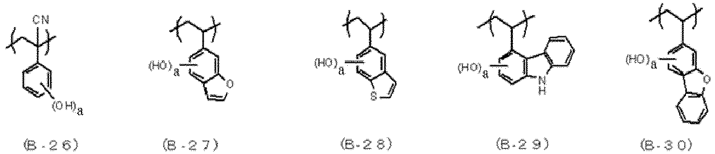

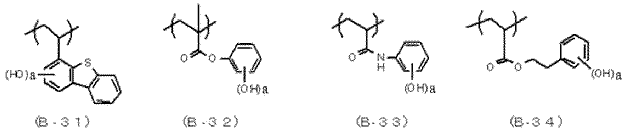

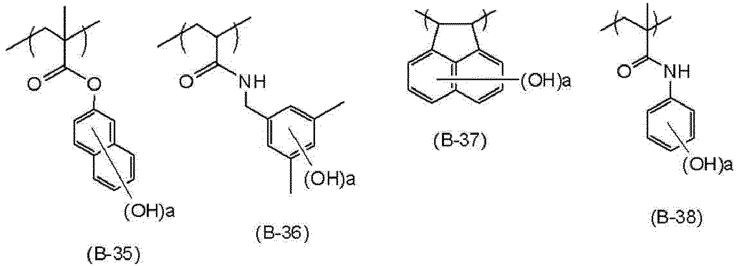

Moreover, among the repeating units, the repeating units specifically described below are preferable. In the formula, R represents a hydrogen atom or a methyl group, and a represents 2 or 3.

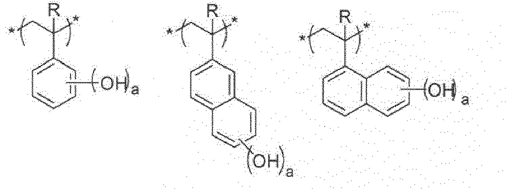

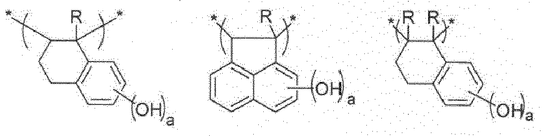

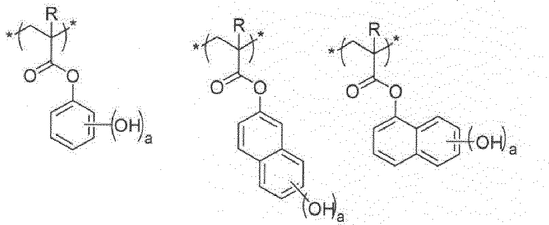

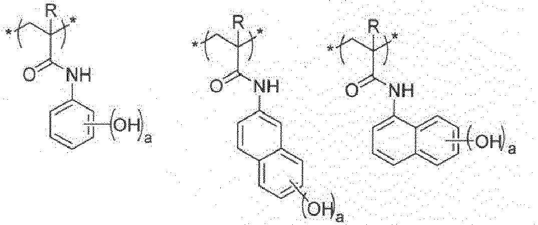

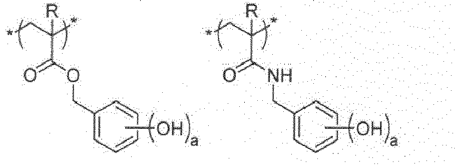

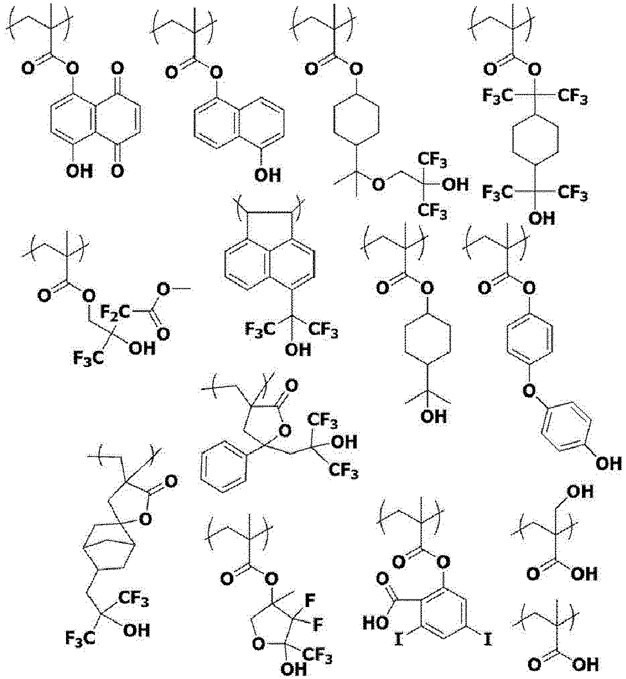

A content of the repeating unit having an acid group is preferably 10% to 70% by mole, more preferably 15% to 65% by mole, and still more preferably 20% to 60% by mole with respect to all repeating units in the resin (A).

### (Repeating Unit Having Fluorine Atom or Iodine Atom)

The resin (A) may have a repeating unit having a fluorine atom or an iodine atom in addition to the above-mentioned <Repeating Unit Having Acid-Decomposable Group> and <Repeating Unit Having Acid Group>. In addition, <Repeating Unit Having Fluorine Atom or Iodine Atom> as mentioned herein is preferably different from other kinds of repeating units belonging to the group A, such as <Repeating Unit Having Lactone Group, Sultone Group, or Carbonate Group> and <Repeating Unit Having Photoacid Generating Group>, which will be described later.

As the repeating unit having a fluorine atom or an iodine atom, a repeating unit represented by Formula (C) is preferable.

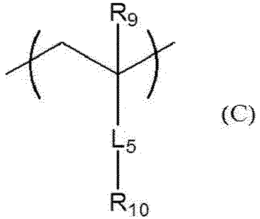

L₅ represents a single bond or an ester group.

R₉ represents a hydrogen atom, or an alkyl group which may have a fluorine atom or an iodine atom.

Rio represents a hydrogen atom, an alkyl group which may have a fluorine atom or an iodine atom, a cycloalkyl group which may have a fluorine atom or an iodine atom, an aryl group which may have a fluorine atom or an iodine atom, or a group formed by combination thereof.

The repeating unit having a fluorine atom or an iodine atom will be exemplified below.

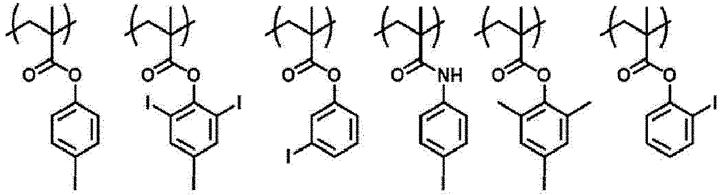

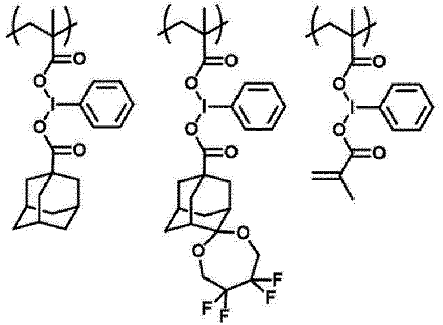

A content of the repeating unit having a fluorine atom or an iodine atom is preferably 0% to 50% by mole, more preferably 5% to 45% by mole, and still more preferably 10% to 40% by mole with respect to all repeating units in the resin (A).

Furthermore, since the repeating unit having a fluorine atom or an iodine atom does not include <Repeating Unit Having Acid-Decomposable Group> and <Repeating Unit Having Acid Group> as described above, the content of the repeating unit having a fluorine atom or an iodine atom is also intended to be the content of the repeating unit having a fluorine atom or an iodine atom excluding <Repeating Unit Having Acid-Decomposable Group> and <Repeating Unit Having Acid Group>.

Among the repeating units of the resin (A), the total content of the repeating units including at least one of a fluorine atom or an iodine atom is preferably 20% to 100% by mole, more preferably 30% to 100% by mole, and still more preferably 40% to 100% by mole with respect to all repeating units of the resin (A).

In addition, examples of the repeating unit including at least one of a fluorine atom or an iodine atom include a repeating unit which has a fluorine atom or an iodine atom, and has an acid-decomposable group, a repeating unit which has a fluorine atom or an iodine atom, and has an acid group, and a repeating unit having a fluorine atom or an iodine atom.

### (Repeating Unit Having Lactone Group, Sultone Group, or Carbonate Group)

The resin (A) may have a repeating unit having at least one selected from the group consisting of a lactone group, a sultone group, and a carbonate group (hereinafter also collectively referred to as a "repeating unit having a lactone group, a sultone group, or a carbonate group").

It is also preferable that the repeating unit having a lactone group, a sultone group, or a carbonate group has no acid group such as a hexafluoropropanol group.

The lactone group or the sultone group may have a lactone structure or a sultone structure. The lactone structure or the sultone structure is preferably a 5- to 7-membered ring lactone structure or a 5- to 7-membered ring sultone structure. Among those, the structure is more preferably a 5- to 7-membered ring lactone structure with which another ring structure is fused so as to form a bicyclo structure or a spiro structure or a 5- to 7-membered ring sultone structure with which another ring structure is fused so as to form a bicyclo structure or a spiro structure.

The resin (A) preferably has a repeating unit having a lactone group or a sultone group, formed by extracting one or more hydrogen atoms from a ring member atom of a lactone structure represented by any of General Formulae (LC1-1) to (LC1-21) or a sultone structure represented by any of General Formulae (SL1-1) to (SL1-3).

In addition, the lactone group or the sultone group may be bonded directly to the main chain. For example, a ring member atom of the lactone group or the sultone group may constitute the main chain of the resin (A).

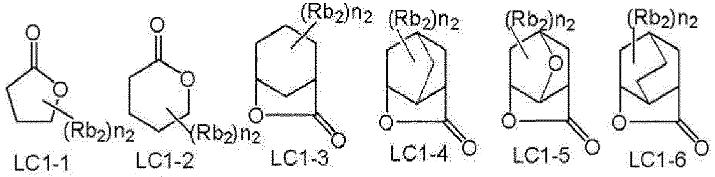

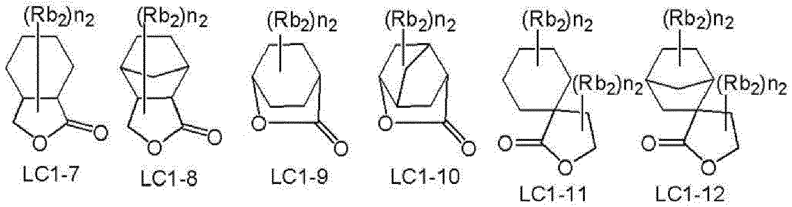

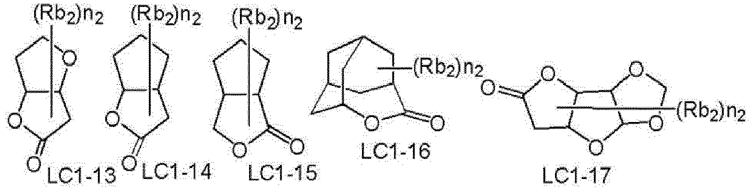

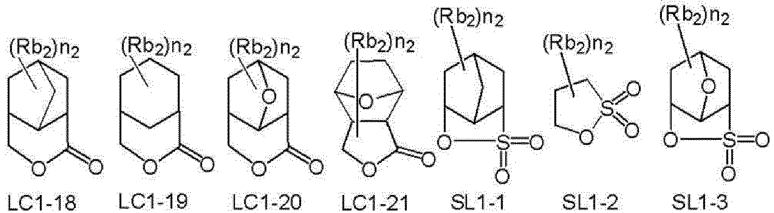

The moiety of the lactone structure or the sultone structure may have a substituent (Rb₂). Preferred examples of the substituent (Rb₂) include an alkyl group having 1 to 8 carbon atoms, a cycloalkyl group having 4 to 7 carbon atoms, an alkoxy group having 1 to 8 carbon atoms, an alkoxycarbonyl group having 1 to 8 carbon atoms, a carboxyl group, a halogen atom, a hydroxyl group, a cyano group, and an acid-decomposable group. n2 represents an integer of 0 to 4. In a case where n2 is 2 or more, Rb₂'s which are present in a plural number may be different from each other, and Rb₂'s which are present in a plural number may be bonded to each other to form a ring.

Examples of the repeating unit having a group having the lactone structure represented by any of General Formulae (LC1-1) to (LC1-21) or the sultone structure represented by any of General Formulae (SL1-1) to (SL1-3) include a repeating unit represented by General Formula (AI).

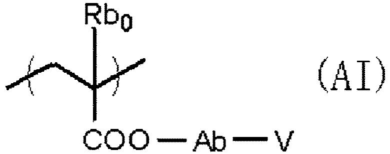

In General Formula (AI), Rbo represents a hydrogen atom, a halogen atom, or an alkyl group having 1 to 4 carbon atoms.

Preferred examples of the substituent which may be contained in the alkyl group of Rbo include a hydroxyl group and a halogen atom.

Examples of the halogen atom of Rbo include a fluorine atom, a chlorine atom, a bromine atom, and an iodine atom. Rbo is preferably the hydrogen atom or a methyl group.

Ab represents a single bond, an alkylene group, a divalent linking group having a monocyclic or polycyclic alicyclic hydrocarbon structure, an ether group, an ester group, a carbonyl group, a carboxyl group, or a divalent group formed by combination thereof. Among those, the single bond or a linking group represented by -Ab₁-CO₂- is preferable. Abi is a linear or branched alkylene group, or a monocyclic or polycyclic cycloalkylene group, and is preferably a methylene group, an ethylene group, a cyclohexylene group, an adamantylene group, or a norbornylene group.

V represents a group formed by extracting one hydrogen atom from a ring member atom of the lactone structure represented by any of General Formulae (LC1-1) to (LC1-21) or a group formed by extracting one hydrogen atom from a ring member atom of the sultone structure represented by any of General Formulae (SL1-1) to (SL1-3).

In a case where an optical isomer is present in the repeating unit having a lactone group or a sultone group, any of optical isomers may be used. In addition, one kind of optical isomers may be used alone or a plurality of kinds of optical isomers may be mixed and used. In a case where one kind of optical isomers is mainly used, an optical purity (ee) thereof is preferably 90 or more, and more preferably 95 or more.

As the carbonate group, a cyclic carbonic acid ester group is preferable.

As the repeating unit having a cyclic carbonic acid ester group, a repeating unit represented by General Formula (A-1) is preferable.

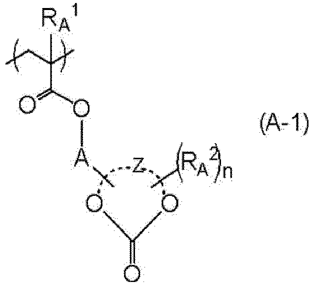

In General Formula (A-1), R_{A}¹ represents a hydrogen atom, a halogen atom, or a monovalent organic group (preferably a methyl group).

n represents an integer of 0 or more.

R_{A}² represents a substituent. In a case where n is 2 or more, R_{A}² which are present in a plural number may be the same as or different from each other.

A represents a single bond or a divalent linking group. As the divalent linking group, an alkylene group, a divalent linking group having a monocyclic or polycyclic alicyclic hydrocarbon structure, an ether group, an ester group, a carbonyl group, a carboxyl group, or a divalent group formed by combination thereof is preferable.

Z represents an atomic group that forms a monocycle or polycycle with a group represented by -O-CO-O- in the formula.

The repeating unit having a lactone group, a sultone group, or a carbonate group will be exemplified below.

(in the formulae, Rx represents H, CH₃, CH₂OH, or CF₃)

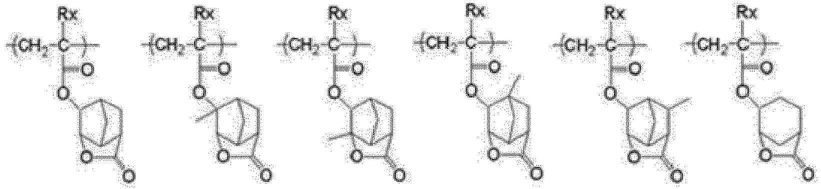

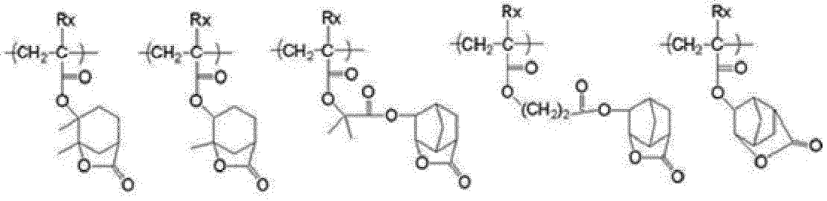

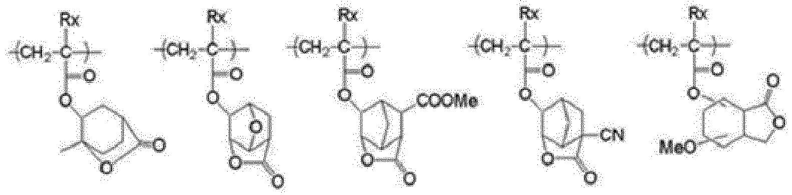

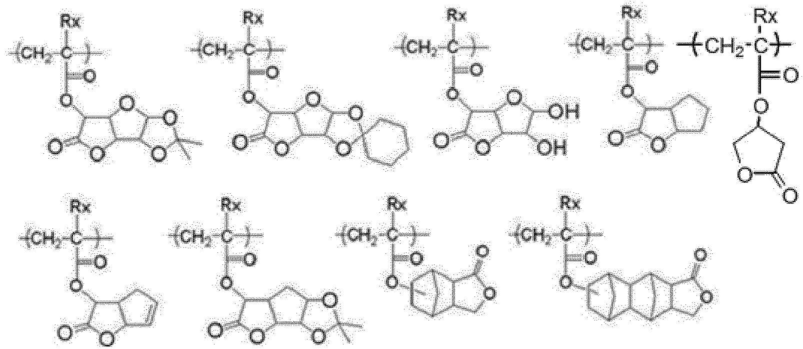

(in the formulae, Rx represents H, CH₃, CH₂OH, or CF₃)

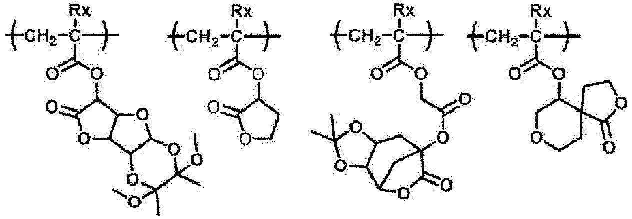

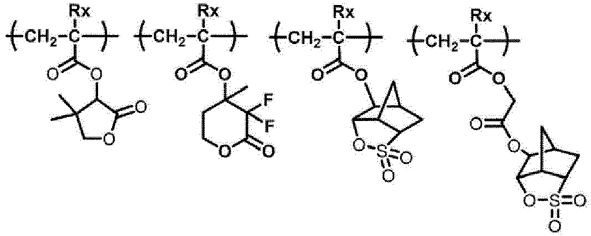

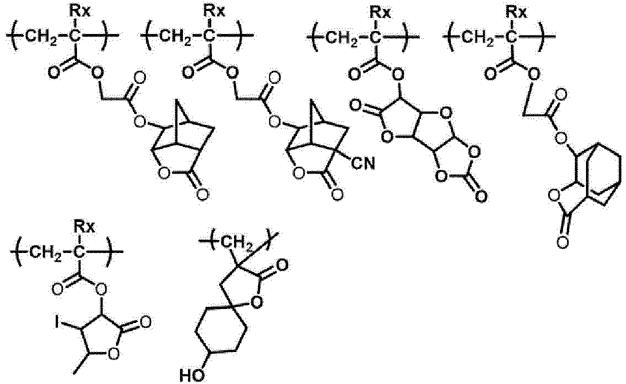

(in the formulae, Rx represents H, CH₃, CH₂OH, or CF₃)

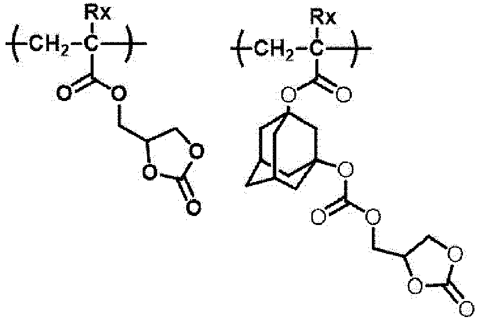

A content of the repeating unit having a lactone group, a sultone group, or a carbonate group is preferably 1% to 70% by mole, more preferably 5% to 65% by mole, and still more preferably 5% to 60% by mole with respect to all repeating units in the resin (A).

### (Repeating Unit Having Photoacid Generating Group)

The resin (A) may have, as a repeating unit other than those above, a repeating unit having a group that generates an acid upon irradiation with actinic rays or radiation (hereinafter also referred to as a "photoacid generating group").

In this case, it can be considered that the repeating unit having a photoacid generating group corresponds to a compound that generates an acid upon irradiation with actinic rays or radiation which will be described later (also referred to as a "photoacid generator").

Examples of such the repeating unit include a repeating unit represented by General Formula (4).

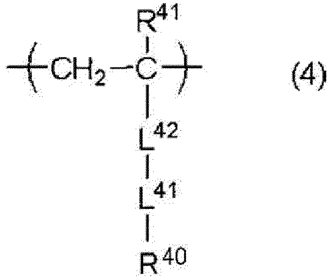

R⁴¹ represents a hydrogen atom or a methyl group. L⁴¹ represents a single bond or a divalent linking group. L⁴² represents a divalent linking group. R⁴⁰ represents a structural moiety that decomposes upon irradiation with actinic rays or radiation to generate an acid in a side chain.

The repeating unit having a photoacid generating group is exemplified below.

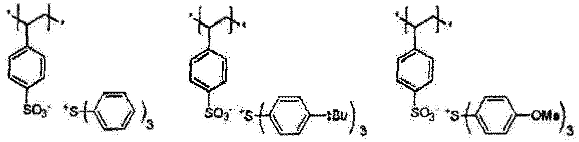

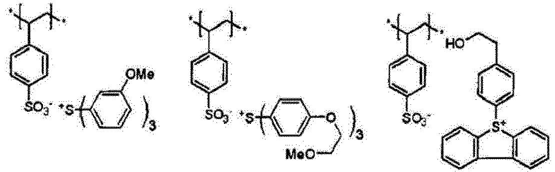

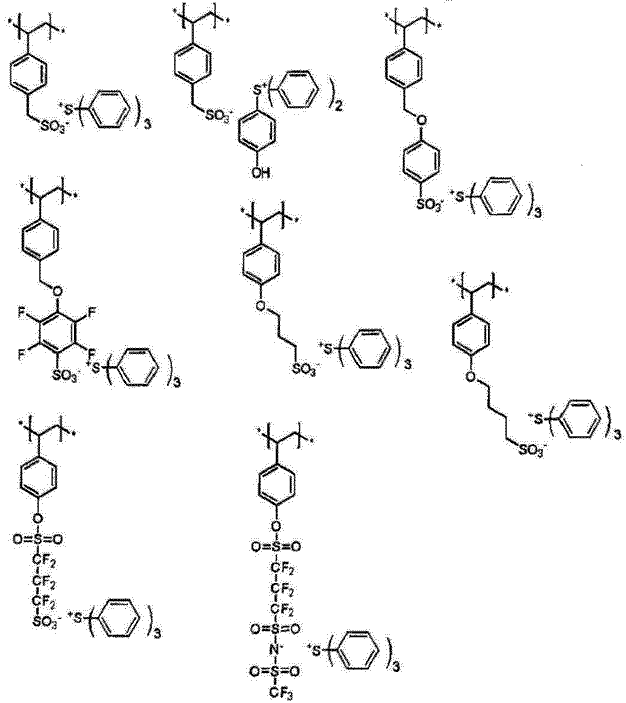

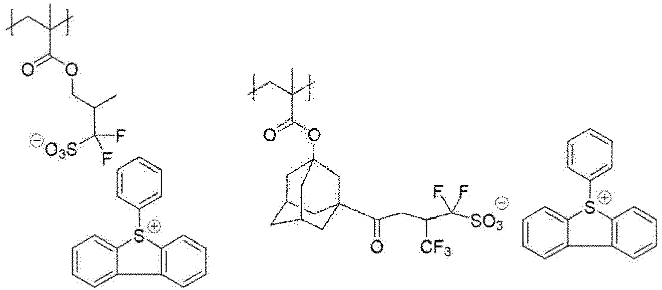

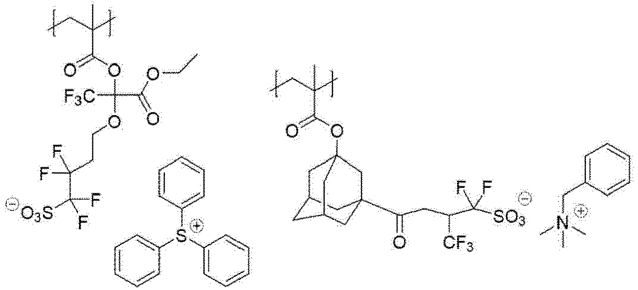

In addition, examples of the repeating unit represented by General Formula (4) include the repeating units described in paragraphs [0094] to [0105] of JP2014-041327A.

A content of the repeating unit having a photoacid generating group is preferably 1% to 40% by mole, more preferably 5% to 35% by mole, and still more preferably 5% to 30% by mole with respect to all repeating units in the resin (A).

### (Repeating Unit Represented by General Formula (V-1) or General Formula (V-2))

The resin (A) may have a repeating unit represented by General Formula (V-1) or General Formula (V-2).

The repeating unit represented by General Formula (V-1) and General Formula (V-2) is preferably a repeating unit different from the above-mentioned repeating units.

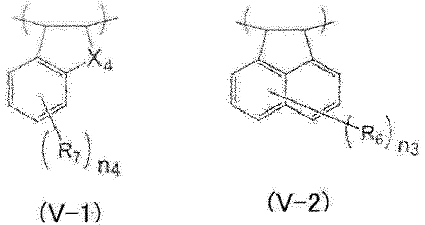

In the formulae,
R₆ and R₇ each independently represent a hydrogen atom, a hydroxyl group, an alkyl group, an alkoxy group, an acyloxy group, a cyano group, a nitro group, an amino group, a halogen atom, an ester group (-OCOR or -COOR: R is an alkyl group or fluorinated alkyl group having 1 to 6 carbon atoms), or a carboxyl group. As the alkyl group, a linear, branched, or cyclic alkyl group having 1 to 10 carbon atoms is preferable.
n₃ represents an integer of 0 to 6.
n₄ represents an integer of 0 to 4.
X₄ is a methylene group, an oxygen atom, or a sulfur atom.

The repeating unit represented by General Formula (V-1) or (V-2) will be exemplified below.

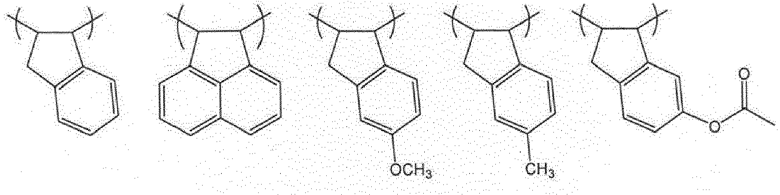

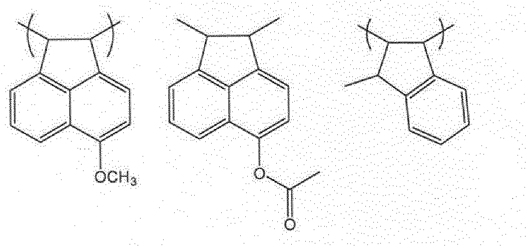

### (Repeating Unit for Reducing Motility of Main Chain)

The resin (A) preferably has a high glass transition temperature (Tg) from the viewpoint that excessive diffusion of an acid generated or pattern collapse during development can be suppressed. Tg is preferably higher than 90°C, more preferably higher than 100°C, still more preferably higher than 110°C, and particularly preferably higher than 125°C. In addition, since an excessive increase in Tg causes a decrease in the dissolution rate in a developer, Tg is preferably 400°C or lower, and more preferably 350°C or lower.

Furthermore, in the present specification, the glass transition temperature (Tg) of a polymer such as the resin (A) is calculated by the following method. First, the Tg of a homopolymer consisting only of each repeating unit included in the polymer is calculated by a Bicerano method. Hereinafter, the calculated Tg is referred to as the "Tg of the repeating unit". Next, the mass proportion (%) of each repeating unit to all repeating units in the polymer is calculated. Then, the Tg at each mass proportion is calculated using a Fox's equation (described in Materials Letters 62 (2008) 3152, and the like), and these are summed to obtain the Tg (°C) of the polymer.

The Bicerano method is described in Prediction of polymer properties, Marcel Dekker Inc., New York (1993), and the like. The calculation of a Tg by the Bicerano method can be carried out using MDL Polymer (MDL Information Systems, Inc.), which is software for estimating physical properties of a polymer.

In order to raise the Tg of the resin (A) (preferably to raise the Tg to higher than 90°C), it is preferable to reduce the motility of the main chain of the resin (A). Examples of a method for reducing the motility of the main chain of the resin (A) include the following (a) to (e) methods.
(a) Introduction of a bulky substituent into the main chain
(b) Introduction of a plurality of substituents into the main chain
(c) Introduction of a substituent that induces an interaction between the resins (A) near the main chain
(d) Formation of the main chain in a cyclic structure
(e) Linking of a cyclic structure to the main chain

Furthermore, the resin (A) preferably has a repeating unit having a Tg of a homopolymer exhibiting 130°C or higher.

In addition, the type of the repeating unit having a Tg of the homopolymer exhibiting 130°C or higher is not particularly limited, and may be any of repeating units having a Tg of a homopolymer of 130°C or higher calculated by the Bicerano method. Moreover, it corresponds to a repeating unit having a Tg of a homopolymer exhibiting 130°C or higher, depending on the type of a functional group in the repeating units represented by Formula (A) to Formula (E) which will be described later.

### (Repeating Unit Represented by Formula (A))

As an example of a specific unit for accomplishing (a) above, a method of introducing a repeating unit represented by Formula (A) into the resin (A) may be mentioned.

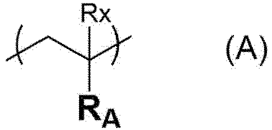

In Formula (A), R_{A} represents a group having a polycyclic structure. Rₓ represents a hydrogen atom, a methyl group, or an ethyl group. The group having a polycyclic structure is a group having a plurality of ring structures, and the plurality of ring structures may or may not be fused.

Specific examples of the repeating unit represented by Formula (A) include the following repeating units.

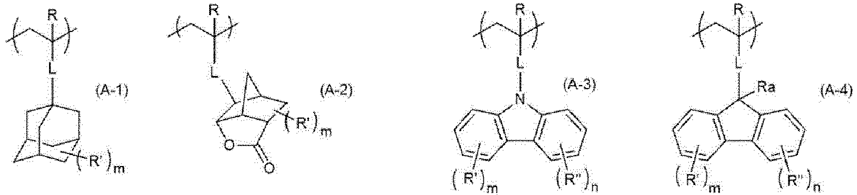

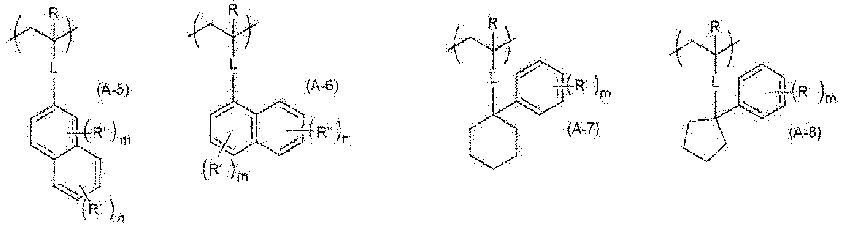

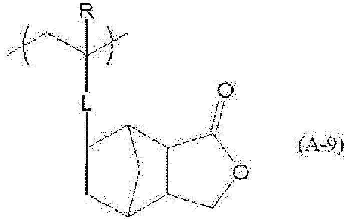

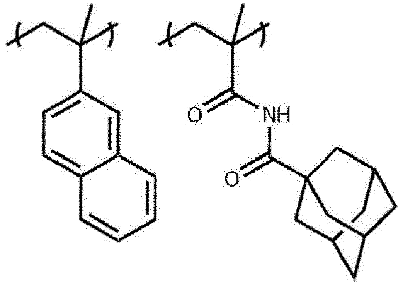

In the formulae, R represents a hydrogen atom, a methyl group, or an ethyl group.

Ra represents a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR'" or -COOR‴: R'" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by Ra may be substituted with a fluorine atom or an iodine atom.

Moreover, R' and R" each independently represent an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR'" or -COOR‴: R'" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by each of R' and R" may be substituted with a fluorine atom or an iodine atom.

L represents a single bond or a divalent linking group. Examples of the divalent linking group include -COO-, -CO-, -O-, -S-, -SO-, -SO₂-, an alkylene group, a cycloalkylene group, an alkenylene group, and a linking group in which a plurality of these groups are linked.

m and n each independently represent an integer of 0 or more. An upper limit of each of m and n is not particularly limited, but is 2 or less in many cases, and 1 or less in more cases.

### (Repeating Unit Represented by Formula (B))

As an example of a specific unit for accomplishing (b) above, a method of introducing a repeating unit represented by Formula (B) into the resin (A) may be mentioned.

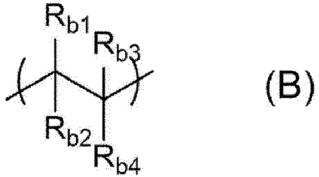

In Formula (B), R_{b1} to R_{b4} each independently represent a hydrogen atom or an organic group, and at least two or more of R_{b1},., or R_{b4} represent an organic group.

Furthermore, in a case where at least one of the organic groups is a group in which a ring structure is directly linked to the main chain in the repeating unit, the types of the other organic groups are not particularly limited.

In addition, in a case where none of the organic groups is a group in which a ring structure is directly linked to the main chain in the repeating unit, at least two or more of the organic groups are substituents having three or more constituent atoms excluding hydrogen atoms.

Specific examples of the repeating unit represented by Formula (B) include the following repeating units.

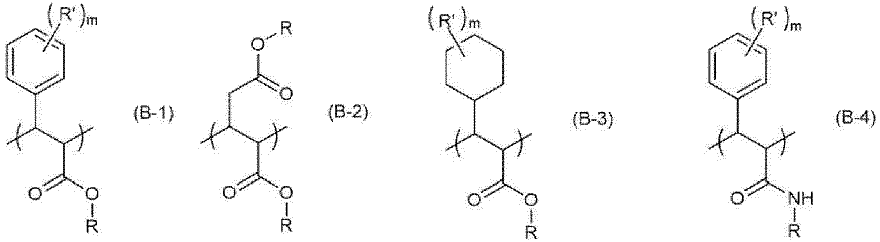

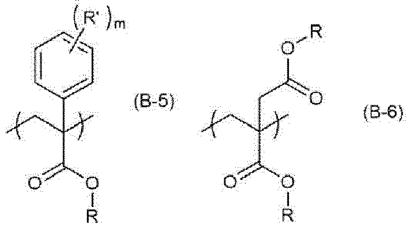

In the formula, R's each independently represent a hydrogen atom or an organic group. Examples of the organic group include an organic group such as an alkyl group, a cycloalkyl group, an aryl group, an aralkyl group, and an alkenyl group, each of which may have a substituent.

R"s each independently represent an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by R' may be substituted with a fluorine atom or an iodine atom.

m represents an integer of 0 or more. An upper limit of m is not particularly limited, but is 2 or less in many cases, and 1 or less in more cases.

### (Repeating Unit Represented by Formula (C))

As an example of a specific unit for accomplishing (c) above, a method of introducing a repeating unit represented by Formula (C) into the resin (A) may be mentioned.

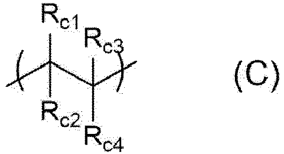

In Formula (C), R_{c1} to R_{c4} each independently represent a hydrogen atom or an organic group, and at least one of R_{c1},., or R_{c4} is a group having a hydrogen-bonding hydrogen atom with a number of atoms of 3 or less from the main chain carbon. Among those, it is preferable that the group has hydrogen-bonding hydrogen atoms with a number of atoms of 2 or less (on a side closer to the vicinity of the main chain) to induce an interaction between the main chains of the resin (A).

Specific examples of the repeating unit represented by Formula (C) include the following repeating units.

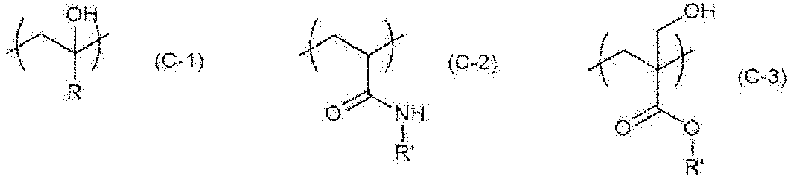

In the formula, R represents an organic group. Examples of the organic group include an alkyl group, a cycloalkyl group, an aryl group, an aralkyl group, an alkenyl group, and an ester group (-OCOR or -COOR: R represents an alkyl group or fluorinated alkyl group having 1 to 20 carbon atoms), each of which may have a substituent.

R' represents a hydrogen atom or an organic group. Examples of the organic group include an organic group such as an alkyl group, a cycloalkyl group, an aryl group, an aralkyl group, and an alkenyl group. In addition, a hydrogen atom in the organic group may be substituted with a fluorine atom or an iodine atom.

### (Repeating Unit Represented by Formula (D))

As an example of a specific unit for accomplishing (d) above, a method of introducing a repeating unit represented by Formula (D) into the resin (A) may be mentioned.

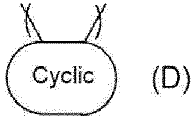

In Formula (D), "Cyclic" is a group that forms a main chain with a cyclic structure. The number of the ring-constituting atoms is not particularly limited.

Specific examples of the repeating unit represented by Formula (D) include the following repeating units.

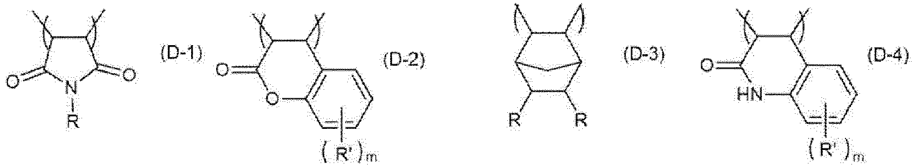

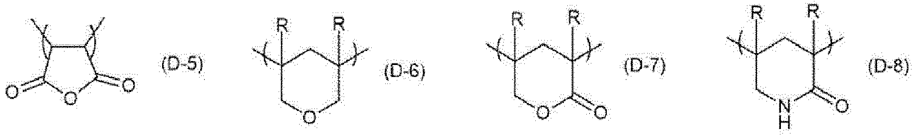

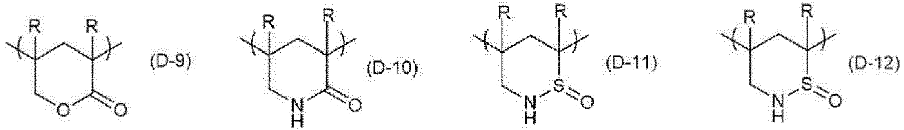

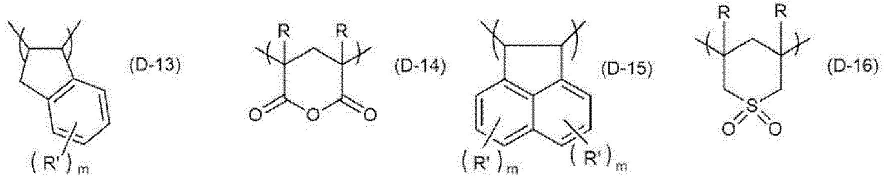

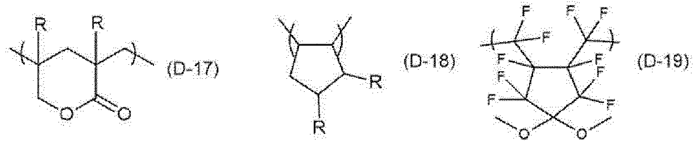

In the formula, R's each independently represent a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, the hydrogen atom bonded to the carbon atom in the group represented by R may be substituted with a fluorine atom or an iodine atom.

In the formula, R"s each independently represent an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by R' may be substituted with a fluorine atom or an iodine atom.

m represents an integer of 0 or more. An upper limit of m is not particularly limited, but is 2 or less in many cases, and 1 or less in more cases.

### (Repeating Unit Represented by Formula (E))

As an example of a specific unit for accomplishing (e) above, a method of introducing a repeating unit represented by Formula (E) into the resin (A) may be mentioned.

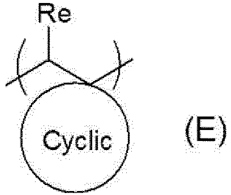

In Formula (E), Re's each independently represent a hydrogen atom or an organic group. Examples of the organic group include an alkyl group, a cycloalkyl group, an aryl group, an aralkyl group, and an alkenyl group, which may have a substituent.

"Cyclic" is a cyclic group including a carbon atom of the main chain. The number of atoms included in the cyclic group is not particularly limited.

Specific examples of the repeating unit represented by Formula (E) include the following repeating units.

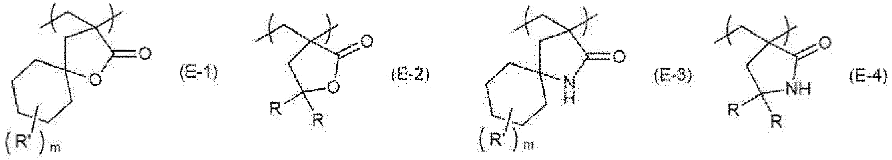

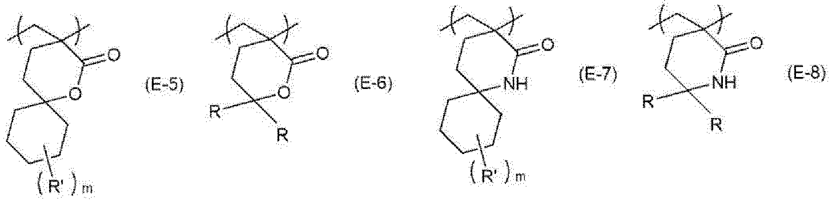

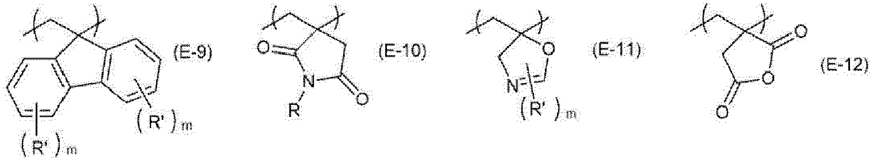

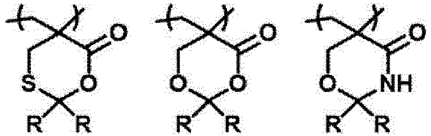

In the formula, R's each independently represent a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, the hydrogen atom bonded to the carbon atom in the group represented by R may be substituted with a fluorine atom or an iodine atom.

R"s each independently represent a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by R' may be substituted with a fluorine atom or an iodine atom.
m represents an integer of 0 or more. An upper limit of m is not particularly limited, but is 2 or less in many cases, and 1 or less in more cases.

In addition, two R's bonded to the same carbon atom may be bonded to each other to form a ring.

In Formula (E-2), Formula (E-4), Formula (E-6), and Formula (E-8), two R's may be combined to form an "=O".

A content of the repeating unit represented by Formula (E) is preferably 5% by mole or more, and more preferably 10% by mole or more with respect to all repeating units in the resin (A). In addition, an upper limit value thereof is preferably 60% by mole or less, and more preferably 55% by mole or less.

### (Repeating Unit Having at Least One Group selected from Lactone Group, Sultone Group, Carbonate Group, Hydroxyl Group, Cyano Group, or Alkali-Soluble Group)

The resin (A) may have a repeating unit having at least one group selected from a lactone group, a sultone group, a carbonate group, a hydroxyl group, a cyano group, or an alkali-soluble group.

Examples of the repeating unit having a lactone group, a sultone group, or a carbonate group contained in the resin (A) include the repeating units described in <Repeating Unit Having Lactone Group, Sultone Group, or Carbonate Group> mentioned above. A preferred content thereof is also the same as described in <Repeating Unit Having Lactone Group, Sultone Group, or Carbonate Group> mentioned above.

The resin (A) may have a repeating unit having a hydroxyl group or a cyano group. As a result, the adhesiveness to a substrate and the affinity for a developer are improved.

The repeating unit having a hydroxyl group or a cyano group is preferably a repeating unit having an alicyclic hydrocarbon structure substituted with a hydroxyl group or a cyano group.

The repeating unit having a hydroxyl group or a cyano group preferably has no acid-decomposable group.

Examples of the repeating unit having a hydroxyl group or a cyano group include repeating units represented by General Formulae (AIIa) to (AIId).

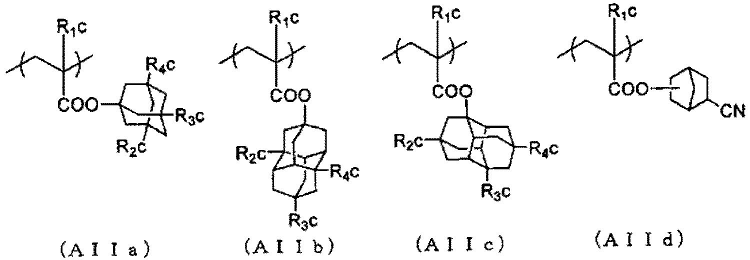

In General Formulae (AIIa) to (AIId),

R_{1c} represents a hydrogen atom, a methyl group, a trifluoromethyl group, or a hydroxymethyl group.

R_{2c} to R_{4c} each independently represent a hydrogen atom, a hydroxyl group, or a cyano group. It should be noted that at least one of R_{2c},., or R_{4c} represents a hydroxyl group or a cyano group. It is preferable that one or two of R_{2c} to R_{4c} are hydroxyl groups, and the rest are hydrogen atoms. It is more preferable that two of R_{2c} to R_{4c} are hydroxyl groups and the rest are hydrogen atoms.

A content of the repeating unit having a hydroxyl group or a cyano group is preferably 5% by mole or more, and more preferably 10% by mole or more with respect to all repeating units in the resin (A). In addition, an upper limit value thereof is preferably 60% by mole or less, more preferably 55% by mole or less, and still more preferably 50% by mole or less.

Specific examples of the repeating unit having a hydroxyl group or a cyano group are shown below, but the present invention is not limited thereto.

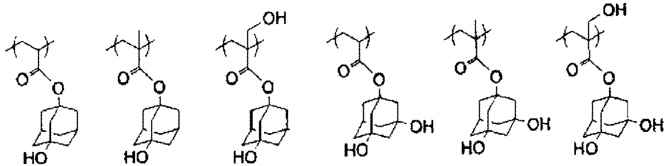

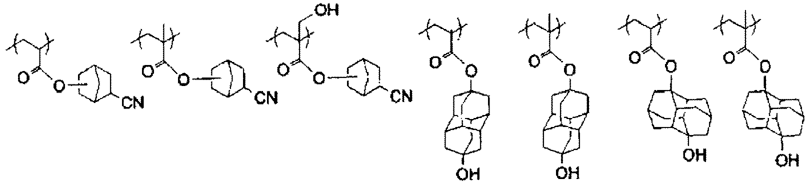

The resin (A) may have a repeating unit having an alkali-soluble group.

Examples of the alkali-soluble group include a carboxyl group, a sulfonamide group, a sulfonylimide group, a bissulfonylimide group, or an aliphatic alcohol group (for example, a hexafluoroisopropanol group) in which the α-position is substituted with an electron-withdrawing group, and the carboxyl group is preferable. In a case where the resin (A) includes a repeating unit having an alkali-soluble group, the resolution for use in contact holes increases.

Examples of the repeating unit having an alkali-soluble group include a repeating unit in which an alkali-soluble group is directly bonded to the main chain of a resin such as a repeating unit with acrylic acid and methacrylic acid, or a repeating unit in which an alkali-soluble group is bonded to the main chain of the resin through a linking group. Furthermore, the linking group may have a monocyclic or polycyclic cyclic hydrocarbon structure.

The repeating unit having an alkali-soluble group is preferably a repeating unit with acrylic acid or methacrylic acid.

A content of the repeating unit having an alkali-soluble group is preferably 0% by mole or more, more preferably 3% by mole or more, and still more preferably 5% by mole or more with respect to all repeating units in the resin (A). An upper limit value thereof is preferably 20% by mole or less, more preferably 15% by mole or less, and still more preferably 10% by mole or less.

Specific examples of the repeating unit having an alkali-soluble group are shown below, but the present invention is not limited thereto. In the specific examples, Rx represents H, CH₃, CH₂OH, or CF₃.

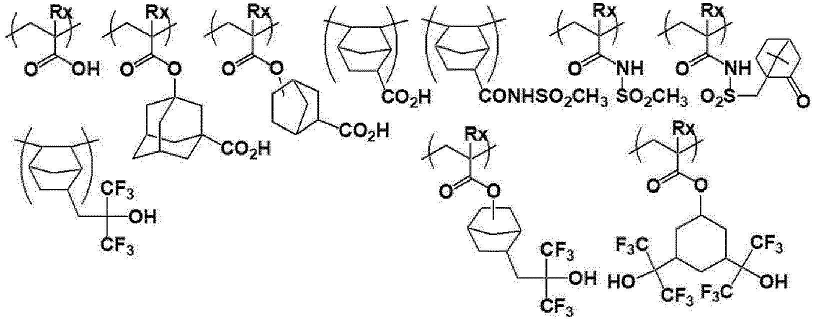

As the repeating unit having at least one group selected from a lactone group, a hydroxyl group, a cyano group, or an alkali-soluble group, a repeating unit having at least two selected from a lactone group, a hydroxyl group, a cyano group, or an alkali-soluble group is preferable, a repeating unit having a cyano group and a lactone group is more preferable, and a repeating unit having a structure in which a cyano group is substituted in the lactone structure represented by General Formula (LC1-4) is still more preferable.

### (Repeating Unit Having Alicyclic Hydrocarbon Structure and Not Exhibiting Acid Decomposability)

The resin (A) may have a repeating unit having an alicyclic hydrocarbon structure and not exhibiting acid decomposability. This can reduce the elution of low-molecular-weight components from the resist film into an immersion liquid during liquid immersion exposure. Examples of such the repeating unit include repeating units derived from 1-adamantyl (meth)acrylate, diadamantyl (meth)acrylate, tricyclodecanyl (meth)acrylate, and cyclohexyl (meth)acrylate.

### (Repeating Unit Represented by General Formula (III) Having Neither Hydroxyl Group Nor Cyano Group)

The resin (A) may have a repeating unit represented by General Formula (III), which has neither a hydroxyl group nor a cyano group.

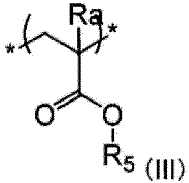

In General Formula (III), R₅ represents a hydrocarbon group having at least one cyclic structure and having neither a hydroxyl group nor a cyano group.

Ra represents a hydrogen atom, an alkyl group, or a -CH₂-O-Ra₂ group. In the formula, Ra₂ represents a hydrogen atom, an alkyl group, or an acyl group.

The cyclic structure contained in R₅ includes a monocyclic hydrocarbon group and a polycyclic hydrocarbon group. Examples of the monocyclic hydrocarbon group include a cycloalkyl group having 3 to 12 carbon atoms (more preferably having 3 to 7 carbon atoms) or a cycloalkenyl group having 3 to 12 carbon atoms.

Examples of the polycyclic hydrocarbon group include a ring-assembled hydrocarbon group and a crosslinked cyclic hydrocarbon group. Examples of the crosslinked cyclic hydrocarbon ring include a bicyclic hydrocarbon ring, a tricyclic hydrocarbon ring, and a tetracyclic hydrocarbon ring. Furthermore, examples of the crosslinked cyclic hydrocarbon ring also include a fused ring formed by fusing a plurality of 5- to 8-membered cycloalkane rings.

As the crosslinked cyclic hydrocarbon group, a norbornyl group, an adamantyl group, a bicyclooctanyl group, or a tricyclo[5,2,1,0^{2,6}]decanyl group is preferable, and the norbornyl group or the adamantyl group is more preferable.

The alicyclic hydrocarbon group may have a substituent, and examples of the substituent include a halogen atom, an alkyl group, a hydroxyl group protected by a protective group, and an amino group protected by a protective group.

The halogen atom is preferably a bromine atom, a chlorine atom, or a fluorine atom.

As the alkyl group, a methyl group, an ethyl group, a butyl group, or a t-butyl group is preferable. The alkyl group may further have a substituent, and examples of the substituent include a halogen atom, an alkyl group, a hydroxyl group protected by a protective group, and an amino group protected by a protective group.

Examples of the protective group include an alkyl group, a cycloalkyl group, an aralkyl group, a substituted methyl group, a substituted ethyl group, an alkoxycarbonyl group, and an aralkyloxycarbonyl group.

As the alkyl group, an alkyl group having 1 to 4 carbon atoms is preferable.

As the substituted methyl group, a methoxymethyl group, a methoxythiomethyl group, a benzyloxymethyl group, a t-butoxymethyl group, or a 2-methoxyethoxymethyl group is preferable.

The substituted ethyl group is preferably a 1-ethoxyethyl group or a 1-methyl-1-methoxyethyl group.

As the acyl group, an aliphatic acyl group having 1 to 6 carbon atoms, such as a formyl group, an acetyl group, a propionyl group, a butyryl group, an isobutyryl group, a valeryl group, and a pivaloyl group, is preferable.

As the alkoxycarbonyl group, an alkoxycarbonyl group having 1 to 4 carbon atoms is preferable.

A content of the repeating unit represented by General Formula (III), which has neither a hydroxyl group nor a cyano group, is preferably 0% to 40% by mole, and more preferably 0% to 20% by mole with respect to all repeating units in the resin (A).

Specific examples of the repeating unit represented by General Formula (III) are shown below, but the present invention is not limited thereto. In the formulae, Ra represents H, CH₃, CH₂OH, or CF₃.

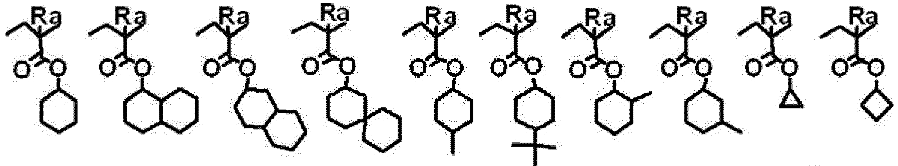

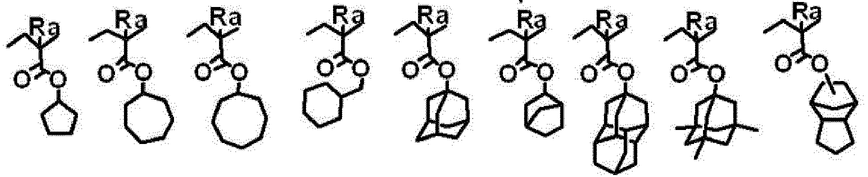

### (Other Repeating Units)

The resin (A) may further have a repeating unit other than the above-mentioned repeating units.

For example, the resin (A) may have a repeating unit selected from the group consisting of a repeating unit having an oxathiane ring group, a repeating unit having an oxazolone ring group, a repeating unit having a dioxane ring group, and a repeating unit having a hydantoin ring group.

Such repeating units will be exemplified below.

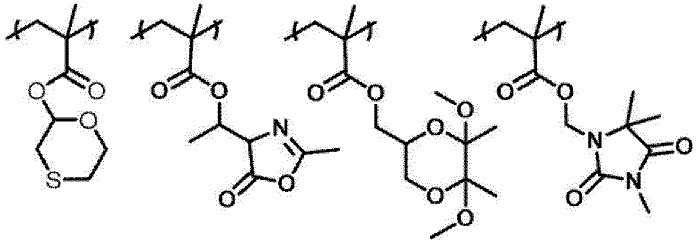

The resin (A) may have a variety of repeating structural units, in addition to the repeating structural units described above, for the purpose of adjusting dry etching resistance, suitability for a standard developer, adhesiveness to a substrate, a resist profile, resolving power, heat resistance, sensitivity, and the like.

As the resin (A), all repeating units is also preferably composed of (meth)acrylate-based repeating units (particularly in a case where the composition is used as an actinic ray-sensitive or radiation-sensitive resin composition for ArF). In this case, any of a resin in which all of the repeating units are methacrylate-based repeating units, a resin in which all of the repeating units are acrylate-based repeating units, and a resin in which all of the repeating units are methacrylate-based repeating units and acrylate-based repeating units can be used, and it is preferable that the amount of the acrylate-based repeating units is 50% by mole or less with respect to all repeating units.

The resin (A) can be synthesized in accordance with an ordinary method (for example, radical polymerization).

The weight-average molecular weight of the resin (A) as a value expressed in terms of polystyrene by a GPC method is preferably 1,000 to 200,000, more preferably 3,000 to 20,000, and still more preferably 5,000 to 15,000. By setting the weight-average molecular weight of the resin (A) to 1,000 to 200,000, deterioration of heat resistance and dry etching resistance can be further suppressed. In addition, deterioration of developability and deterioration of film forming property due to high viscosity can also be further suppressed.

The dispersity (molecular weight distribution) of the resin (A) is usually 1 to 5, preferably 1 to 3, more preferably 1.2 to 3.0, and still more preferably 1.2 to 2.0. The smaller the dispersity, the more excellent the resolution and the resist shape, and the smoother the side wall of the resist pattern, the more excellent the roughness.

In the composition of the embodiment of the present invention, a content of the resin (A) is preferably 50% to 99.9% by mass, and more preferably 60% to 99.0% by mass with respect to the total solid content of the composition.

Furthermore, the solid content is intended to be components excluding the solvent in the composition, and any of components other than the solvent are regarded as the solid content even in a case where they are liquid components.

In addition, the resin (A) may be used alone or in combination of a plurality thereof.

### <Another Photoacid Generator>

The resist composition may include another photoacid generator (a compound which does not correspond to the specific compound and generates an acid upon irradiation with actinic rays or radiation) which does not correspond to the specific compound. Such another photoacid generator is a compound which generates an acid upon exposure (preferably exposure to EUV light and/or ArF).

Such another photoacid generator may be in a form of a low-molecular-weight compound or a form incorporated into a part of a polymer. Furthermore, a combination of the form of a low-molecular-weight compound and the form incorporated into a part of a polymer may also be used.

In a case where such another photoacid generator is in the form of a low-molecular-weight compound, the molecular weight is preferably 3,000 or less, more preferably 2,000 or less, and still more preferably 1,000 or less.

In a case where such another photoacid generator is in the form incorporated into a part of a polymer, it may be incorporated into a part of the resin (A) or into a resin that is different from the resin (A).

In the present invention, the photoacid generator is preferably in the form of the low-molecular-weight compound.

Such another photoacid generator is not particularly limited, and above all, a compound which generates an organic acid is preferable, and examples of the organic acid include the same ones as the organic acids described as the organic acid which can be generated by the specific compound.

Examples of such another photoacid generator include a compound (onium salt) represented by "M⁺X⁻".

In the compound represented by "M⁺X⁻", X⁻ represents an organic anion.

As X⁻ in "M⁺X⁻", the organic anion represented by X⁻ described with respect to General Formula (1) in the specific compound can be similarly used.

In the compound represented by "M⁺X⁻", M⁺ represents an organic cation.

The organic cations are each independently preferably a cation represented by General Formula (ZaI) (cation (ZaI)) or a cation represented by General Formula (ZaII) (cation (ZaII)).

It should be noted that the cation represented by General Formula (ZaI) is different from the cation (a portion other than X⁻) in the specific compound (the compound represented by each of General Formulae (1) to (3)).

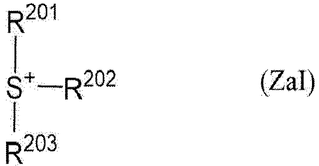

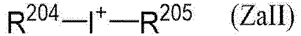

In General Formula (ZaI),

R²⁰¹, R²⁰², and R²⁰³ each independently represent an organic group.

The organic group as each of R²⁰¹, R²⁰², and R²⁰³ usually has 1 to 30 carbon atoms, and preferably has 1 to 20 carbon atoms. In addition, two of R²⁰¹ to R²⁰³ may be bonded to each other to form a ring structure, and the ring may include an oxygen atom, a sulfur atom, an ester group, an amide group, or a carbonyl group. Examples of the group formed by the bonding of two of R²⁰¹ to R²⁰³ include an alkylene group (for example, a butylene group and a pentylene group), and -CH₂-CH₂-O-CH₂-CH₂-.

Examples of the cation in General Formula (ZaI) include a cation (ZaI-1) which will be described later.

The cation (ZaI-1) is an arylsulfonium cation in which at least one of R²⁰¹, R²⁰², or R²⁰³ of General Formula (ZaI) is an aryl group.

In the arylsulfonium cation, all of R²⁰¹ to R²⁰³ may be aryl groups, or some of R²⁰¹ to R²⁰³ may be an aryl group, and the rest may be an alkyl group or a cycloalkyl group.

In addition, one of R²⁰¹ to R²⁰³ may be an aryl group, two of R²⁰¹ to R²⁰³ may be bonded to each other to form a ring structure, and an oxygen atom, a sulfur atom, an ester group, an amide group, or a carbonyl group may be included in the ring. Examples of the group formed by the bonding of two of R²⁰¹ to R²⁰³ include an alkylene group (for example, a butylene group, a pentylene group, or -CH₂-CH₂-O-CH₂-CH₂-) in which one or more methylene groups may be substituted with an oxygen atom, a sulfur atom, an ester group, an amide group, and/or a carbonyl group.

Examples of the arylsulfonium cation include a triarylsulfonium cation, a diarylalkylsulfonium cation, an aryldialkylsulfonium cation, a diarylcycloalkylsulfonium cation, and an aryldicycloalkylsulfonium cation.

The aryl group included in the arylsulfonium cation is preferably a phenyl group or a naphthyl group, and more preferably the phenyl group. The aryl group may be an aryl group which has a heterocyclic structure having an oxygen atom, a nitrogen atom, a sulfur atom, or the like. Examples of the heterocyclic structure include a pyrrole residue, a furan residue, a thiophene residue, an indole residue, a benzofuran residue, and a benzothiophene residue. In a case where the arylsulfonium cation has two or more aryl groups, the two or more aryl groups may be the same as or different from each other.

The alkyl group or the cycloalkyl group contained in the arylsulfonium cation, as necessary, is preferably a linear alkyl group having 1 to 15 carbon atoms, a branched alkyl group having 3 to 15 carbon atoms, or a cycloalkyl group having 3 to 15 carbon atoms, and examples thereof include a methyl group, an ethyl group, a propyl group, an n-butyl group, a sec-butyl group, a t-butyl group, a cyclopropyl group, a cyclobutyl group, and a cyclohexyl group.

The substituents which may be contained in the aryl group, the alkyl group, and the cycloalkyl group of each of R²⁰¹ to R²⁰³ are each independently preferably an alkyl group (for example, having 1 to 15 carbon atoms), a cycloalkyl group (for example, having 3 to 15 carbon atoms), an aryl group (for example, having 6 to 14 carbon atoms), an alkoxy group (for example, having 1 to 15 carbon atoms), a cycloalkylalkoxy group (for example, having 1 to 15 carbon atoms), a halogen atom, a hydroxyl group, or a phenylthio group.

The substituent may further have a substituent as possible, and may be in the form of an alkyl halide group such as a trifluoromethyl group, for example, in which an alkyl group has a halogen atom as a substituent.

In a case where the resist composition includes such another photoacid generator, a content thereof is not particularly limited, but from the viewpoint that the effect of the present invention is more excellent, the content is preferably 0.5% by mass or more, more preferably 1% by mass or more, and still more preferably 2% by mass or more with respect to a total solid content of the composition. Moreover, the content is preferably 40% by mass or less, more preferably 35% by mass or less, and still more preferably 30% by mass or less.

The photoacid generators may be used alone or in combination of two or more kinds thereof.

### <Solvent>

The resist composition may include a solvent.

The solvent preferably includes at least one solvent of (M1) propylene glycol monoalkyl ether carboxylate, or (M2) at least one selected from the group consisting of a propylene glycol monoalkyl ether, a lactic acid ester, an acetic acid ester, an alkoxypropionic acid ester, a chain ketone, a cyclic ketone, a lactone, and an alkylene carbonate as a solvent. Furthermore, this solvent may further include components other than the components (M1) and (M2).

The present inventors have found that by using such a solvent and the above-mentioned resin in combination, a pattern having a small number of development defects can be formed while improving the coating property of the composition. A reason therefor is not necessarily clear, but the present inventors have considered that since these solvents have a good balance among the solubility, the boiling point, and the viscosity of the resin, the unevenness of the film thickness of a composition film, the generation of precipitates during spin coating, and the like can be suppressed.

As the component (M1), at least one selected from the group consisting of propylene glycol monomethyl ether acetate (PGMEA), propylene glycol monomethyl ether propionate, and propylene glycol monoethyl ether acetate is preferable, and propylene glycol monomethyl ether acetate (PGMEA) is more preferable.

The component (M2) is preferably the following solvent.

The propylene glycol monoalkyl ether is preferably propylene glycol monomethyl ether (PGME) or propylene glycol monoethyl ether (PGEE).

The lactic acid ester is preferably ethyl lactate, butyl lactate, or propyl lactate.

The acetic acid ester is preferably methyl acetate, ethyl acetate, butyl acetate, isobutyl acetate, propyl acetate, isoamyl acetate, methyl formate, ethyl formate, butyl formate, propyl formate, or 3-methoxybutyl acetate.

In addition, butyl butyrate is also preferable.

The alkoxypropionic acid ester is preferably methyl 3-methoxypropionate (MMP), or ethyl 3-ethoxypropionate (EEP).

The chain ketone is preferably 1-octanone, 2-octanone, 1-nonanone, 2-nonanone, acetone, 2-heptanone, 4-heptanone, 1-hexanone, 2-hexanone, diisobutyl ketone, phenyl acetone, methyl ethyl ketone, methyl isobutyl ketone, acetyl acetone, acetonyl acetone, ionone, diacetonyl alcohol, acetyl carbinol, acetophenone, methyl naphthyl ketone, or methyl amyl ketone.

The cyclic ketone is preferably methylcyclohexanone, isophorone, cyclopentanone, or cyclohexanone.

The lactone is preferably γ-butyrolactone.

The alkylene carbonate is preferably propylene carbonate.

The component (M2) is more preferably propylene glycol monomethyl ether (PGME), ethyl lactate, ethyl 3-ethoxypropionate, methyl amyl ketone, cyclohexanone, butyl acetate, pentyl acetate, γ-butyrolactone, or propylene carbonate.

In addition to the components, it is preferable to use an ester-based solvent having 7 or more carbon atoms (preferably 7 to 14 carbon atoms, more preferably 7 to 12 carbon atoms, and still more preferably 7 to 10 carbon atoms) and 2 or less heteroatoms.

Examples of the ester-based solvent having 7 or more carbon atoms and 2 or less heteroatoms include 2-methylbutyl acetate, 1-methylbutyl acetate, hexyl acetate, pentyl propionate, hexyl propionate, butyl propionate, isobutyl isobutyrate, heptyl propionate, and butyl butanoate, with isoamyl acetate being preferable.

The component (M2) is preferably a solvent having a flash point (hereinafter also referred to as fp) of 37°C or higher. Such a component (M2) is preferably propylene glycol monomethyl ether (fp: 47°C), ethyl lactate (fp: 53°C), ethyl 3-ethoxypropionate (fp: 49°C), methyl amyl ketone (fp: 42°C), cyclohexanone (fp: 44°C), pentyl acetate (fp: 45°C), methyl 2-hydroxyisobutyrate (fp: 45°C), γ-butyrolactone (fp: 101°C), or propylene carbonate (fp: 132°C). Among those, propylene glycol monoethyl ether, ethyl lactate, pentyl acetate, or cyclohexanone is more preferable, and propylene glycol monoethyl ether or ethyl lactate is still more preferable.

In addition, "flash point" herein means the value described in a reagent catalog of Tokyo Chemical Industry Co., Ltd. or Sigma-Aldrich Co. LLC.

It is preferable that the solvent includes the component (M1). The solvent is more preferably formed of substantially only the component (M1) or is a mixed solvent of the component (M1) and other components. In a case where the solvent is the mixed solvent, it is still more preferable that the solvent includes both the component (M1) and the component (M2).

A mass ratio (M1/M2) of the component (M1) to the component (M2) is preferably "100/0" to "0/10", more preferably "100/0" to "15/85", still more preferably "100/0" to "40/60", and particularly preferably "100/0" to "60/40". That is, in a case where the solvent includes both the component (M1) and the component (M2), the mass ratio of the component (M1) to the component (M2) is preferably 15/85 or more, more preferably 40/60 or more, and still more preferably 60/40 or more. In a case where such a configuration is adopted and used, the number of development defects is reduced.

Moreover, in a case where both of the component (M1) and the component (M2) are included in the solvent, a mass ratio of the component (M1) to the component (M2) is set to, for example, 99/1 or less.

As described above, the solvent may further include components other than the components (M1) and (M2). In this case, a content of the components other than the components (M1) and (M2) is preferably 5% to 30% by mass with respect to the total mass of the solvent.

A content of the solvent in the resist composition is preferably set so that the concentration of solid contents is 0.5% to 30% by mass, and more preferably set so that the concentration of solid contents is 1% to 20% by mass. With this content, the coating property of the resist composition can be further improved.

Furthermore, the solid content means all the components excluding the solvent.

### <Acid Diffusion Control Agent>

The resist composition may further include an acid diffusion control agent. The acid diffusion control agent acts as a quencher that traps an acid generated from a photoacid generator and functions to control the phenomenon of acid diffusion in the resist film.

The acid diffusion control agent may be, for example, a basic compound.

The basic compound is preferably a compound having a structure represented by each of General Formula (A) to General Formula (E).

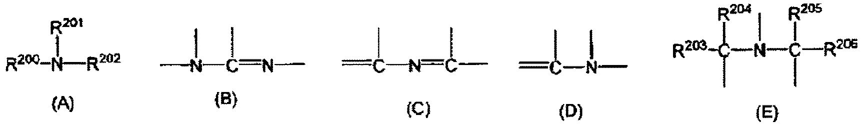

In General Formula (A) and General Formula (E), R²⁰⁰, R²⁰¹, and R²⁰² may be the same as or different from each other, and each represent a hydrogen atom, an alkyl group (preferably having 1 to 20 carbon atoms), a cycloalkyl group (preferably having 3 to 20 carbon atoms), or an aryl group (preferably having 6 to 20 carbon atoms), in which R²⁰¹ and R²⁰² may be bonded to each other to form a ring.

With regard to the alkyl group, the alkyl group having a substituent is preferably an aminoalkyl group having 1 to 20 carbon atoms, a hydroxyalkyl group having 1 to 20 carbon atoms, or a cyanoalkyl group having 1 to 20 carbon atoms.

R²⁰³, R²⁰⁴, R²⁰⁵, and R²⁰⁶ may be the same as or different from each other, and each represent an alkyl group having 1 to 20 carbon atoms.

It is more preferable that the alkyl groups in General Formula (A) and General Formula (E) are unsubstituted.

As a basic compound, guanidine, aminopyrrolidine, pyrazole, pyrazoline, piperazine, aminomorpholine, aminoalkylmorpholine (in which an alkyl group moiety may be linear or branched, and may be partly substituted with an ether group and/or an ester group, and a total number of all atoms other than hydrogen atoms in the alkyl group moiety is preferably 1 to 17), piperidine, or the like is preferable. Among these, a compound having an imidazole structure, a diazabicyclo structure, an onium hydroxide structure, an onium carboxylate structure, a trialkylamine structure, an aniline structure, or a pyridine structure; an alkylamine derivative having a hydroxyl group and/or an ether bond; an aniline derivative having a hydroxyl group and/or an ether bond; or the like is more preferable.

Examples of the compound having an imidazole structure include imidazole, 2,4,5-triphenylimidazole, and benzimidazole. Examples of the compound having a diazabicyclo structure include 1,4-diazabicyclo[2,2,2]octane, 1,5-diazabicyclo[4,3,0]non-5-ene, and 1,8-diazabicyclo[5,4,0]undec-7-ene. Examples of the compound having an onium hydroxide structure include triarylsulfonium hydroxide, phenacylsulfonium hydroxide, and sulfonium hydroxide having a 2-oxoalkyl group. Specific examples thereof include triphenylsulfonium hydroxide, tris(t-butylphenyl)sulfonium hydroxide, bis(t-butylphenyl)iodonium hydroxide, phenacylthiophenium hydroxide, and 2-oxopropylthiophenium hydroxide. The compound having an onium carboxylate structure is formed by carboxylation of an anionic moiety of a compound having an onium hydroxide structure, and examples thereof include acetate, adamantane-1-carboxylate, and perfluoroalkyl carboxylate. Examples of the compound having a trialkylamine structure include tri(n-butyl)amine and tri(n-octyl)amine. Examples of the aniline compound include 2,6-diisopropylaniline, N,N-dimethylaniline, N,N-dibutylaniline, and N,N-dihexylaniline. Examples of the alkylamine derivative having a hydroxyl group and/or an ether bond include ethanolamine, diethanolamine, triethanolamine, tris(methoxyethoxyethyl)amine, and "(HO-C₂H₄-O-C₂H₄)₂N(-C₃H₆-O-CH₃)". Examples of the aniline derivative having a hydroxyl group and/or an ether bond include N,N-bis(hydroxyethyl)aniline.

Preferred examples of the basic compound include an amine compound having a phenoxy group and an ammonium salt compound having a phenoxy group.

As the amine compound, for example, a primary, secondary, or tertiary amine compound can be used, and an amine compound in which at least one alkyl group is bonded to a nitrogen atom is preferable. The amine compound is more preferably a tertiary amine compound. Any amine compound is available as long as at least one alkyl group (preferably having 1 to 20 carbon atoms) is bonded to a nitrogen atom, and a cycloalkyl group (preferably having 3 to 20 carbon atoms) or an aryl group (preferably having 6 to 12 carbon atoms), in addition to the alkyl group, may be bonded to the nitrogen atom.

In addition, the amine compound preferably has an oxyalkylene group. The number of the oxyalkylene groups is preferably 1 or more, more preferably 3 to 9, and still more preferably 4 to 6, within the molecule. Among those, as the oxyalkylene group, an oxyethylene group (-CH₂CH₂O-) or an oxypropylene group (-CH(CH₃)CH₂O- or -CH₂CH₂CH₂O-) is preferable, and the oxyethylene group is more preferable.

Examples of the ammonium salt compound include primary, secondary, tertiary, and quaternary ammonium salt compounds, and an ammonium salt compound in which at least one alkyl group is bonded to a nitrogen atom is preferable. Any ammonium salt compound is available as long as at least one alkyl group (preferably having 1 to 20 carbon atoms) is bonded to a nitrogen atom, and a cycloalkyl group (preferably having 3 to 20 carbon atoms) or an aryl group (preferably having 6 to 12 carbon atoms) may be bonded to the nitrogen atom, in addition to the alkyl group.

It is preferable that the ammonium salt compound has an oxyalkylene group. The number of the oxyalkylene groups is preferably 1 or more, more preferably 3 to 9, and still more preferably 4 to 6, within the molecule. Among oxyalkylene groups, an oxyethylene group (-CH₂CH₂O-) or an oxypropylene group (-CH(CH₃)CH₂O- or -CH₂CH₂CH₂O-) is preferable, and the oxyethylene group is more preferable.

Examples of the anion of the ammonium salt compound include a halogen atom, a sulfonate, a borate, and a phosphate, and among these, the halogen atom or the sulfonate is preferable. The halogen atom is preferably a chlorine atom, a bromine atom, or an iodine atom. The sulfonate is preferably an organic sulfonate having 1 to 20 carbon atoms. Examples of the organic sulfonate include alkyl sulfonate and aryl sulfonate, having 1 to 20 carbon atoms. The alkyl group of the alkyl sulfonate may have a substituent, and examples of the substituent include a fluorine atom, a chlorine atom, a bromine atom, an alkoxy group, an acyl group, and an aromatic ring group. Examples of the alkyl sulfonate include methanesulfonate, ethanesulfonate, butanesulfonate, hexanesulfonate, octanesulfonate, benzyl sulfonate, trifluoromethanesulfonate, pentafluoroethanesulfonate, and nonafluorobutanesulfonate. Examples of the aryl group of the aryl sulfonate include a benzene ring group, a naphthalene ring group, and an anthracene ring group. The substituent which can be contained in the benzene ring group is preferably the naphthalene ring group, and the anthracene ring group, a linear or branched alkyl group having 1 to 6 carbon atoms, or a cycloalkyl group having 3 to 6 carbon atoms. Examples of the linear or branched alkyl group and the cycloalkyl group include a methyl group, an ethyl group, an n-propyl group, an isopropyl group, an n-butyl group, an i-butyl group, a t-butyl group, an n-hexyl group, and a cyclohexyl group. Examples of other substituents include an alkoxy group having 1 to 6 carbon atoms, a halogen atom, a cyano group, a nitro group, an acyl group, and an acyloxy group.

The amine compound having a phenoxy group and the ammonium salt compound having a phenoxy group are each a compound having a phenoxy group at the terminal on the opposite side to the nitrogen atom of the alkyl group which is contained in the amine compound or the ammonium salt compound.

Examples of a substituent of the phenoxy group include an alkyl group, an alkoxy group, a halogen atom, a cyano group, a nitro group, a carboxylic acid group, a carboxylic acid ester group, a sulfonic acid ester group, an aryl group, an aralkyl group, an acyloxy group, and an aryloxy group. The substitution position of the substituent may be any of 2- to 6-positions. The number of the substituents may be any of 1 to 5.

This compound preferably has at least one oxyalkylene group between the phenoxy group and the nitrogen atom. The number of the oxyalkylene groups is preferably 1 or more, more preferably 3 to 9, and still more preferably 4 to 6, within the molecule. Among the oxyalkylene groups, an oxyethylene group (-CH₂CH₂O-) or an oxypropylene group (-CH(CH₃)CH₂O- or -CH₂CH₂CH₂O-) is preferable, and the oxyethylene group is more preferable.

The amine compound having a phenoxy group can be obtained by heating a mixture of a primary or secondary amine having a phenoxy group and a haloalkyl ether to perform a reaction, then adding an aqueous solution of a strong base (for example, sodium hydroxide, potassium hydroxide, and tetraalkylammonium) to a reaction system, and extracting the reaction product with an organic solvent (for example, ethyl acetate and chloroform). Alternatively, the amine compound having a phenoxy group can also be obtained by heating a mixture of a primary or secondary amine and a haloalkyl ether having a phenoxy group at the terminal to perform a reaction, then adding an aqueous solution of a strong base to the reaction system, and extracting the reaction product with an organic solvent.

### (Compound (PA) Which Has Proton-Accepting Functional Group and Generates Compound That Decomposes upon Irradiation with Actinic Rays or Radiation to Exhibit Deterioration in Proton-Accepting Properties, No Proton-Accepting Properties, or Change from Proton-Accepting Properties to Acidic Properties)

The resist composition may include a compound (hereinafter also referred to as a "compound (PA)") which has a proton-accepting functional group and generates a compound that decomposes upon irradiation with actinic rays or radiation to exhibit deterioration in proton-accepting properties, no proton-accepting properties, or a change from the proton-accepting properties to acidic properties as an acid diffusion control agent.

The proton-accepting functional group refers to a functional group having a group or electron capable of electrostatically interacting with a proton, and for example, means a functional group with a macrocyclic structure, such as a cyclic polyether, or a functional group having a nitrogen atom having an unshared electron pair not contributing to π-conjugation. The nitrogen atom having an unshared electron pair not contributing to π-conjugation is, for example, a nitrogen atom having a partial structure represented by the following general formula. 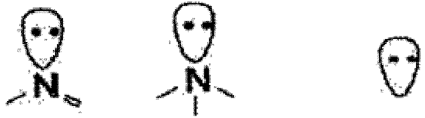 Unshared electron pair

Preferred examples of the partial structure of the proton-accepting functional group include a crown ether structure, an azacrown ether structure, primary to tertiary amine structures, a pyridine structure, an imidazole structure, and a pyrazine structure.

The compound (PA) decomposes upon irradiation with actinic rays or radiation to generate a compound exhibiting deterioration in proton-accepting properties, no proton-accepting properties, or a change from the proton-accepting properties to acidic properties. Here, an expression of generating a compound which exhibits deterioration in proton-accepting properties, no proton-accepting properties, or a change from the proton-accepting properties to acidic properties is a change of proton-accepting properties due to the proton being added to the proton-accepting functional group. Specifically, the expression means a decrease in the equilibrium constant at chemical equilibrium in a case where a proton adduct is generated from the compound (PA) having the proton-accepting functional group and the proton.

With regard to the compound (PA), reference can be made to those described in paragraphs [0421] to [0428] of JP2014-41328A or paragraphs [0108] to [0116] of JP2014-134686A, the contents of which are incorporated herein by reference.

A low-molecular-weight compound having a nitrogen atom and a group that is eliminated by the action of an acid can also be used as an acid diffusion control agent. The low-molecular-weight compound is preferably an amine derivative having, on the nitrogen atom, a group that is eliminated by the action of an acid.

The group that is eliminated by the action of an acid is preferably an acetal group, a carbonate group, a carbamate group, a tertiary ester group, a tertiary hydroxyl group, or a hemiaminal ether group, and more preferably the carbamate group or the hemiaminal ether group.

The molecular weight of the low-molecular-weight compound is preferably 100 to 1,000, more preferably 100 to 700, and still more preferably 100 to 500.

The low-molecular-weight compound may have a carbamate group having a protective group on the nitrogen atom.

Specific examples of the acid diffusion control agent are shown below, but the present invention is not limited.

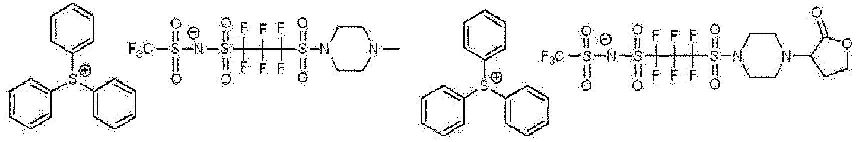

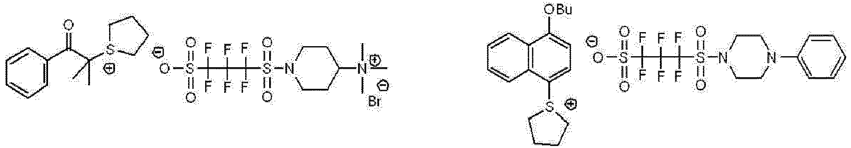

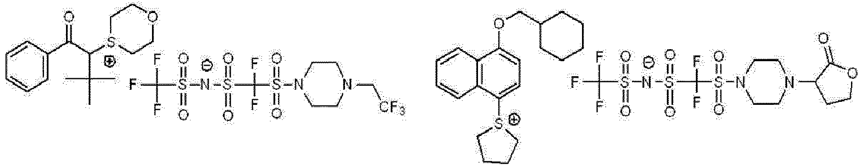

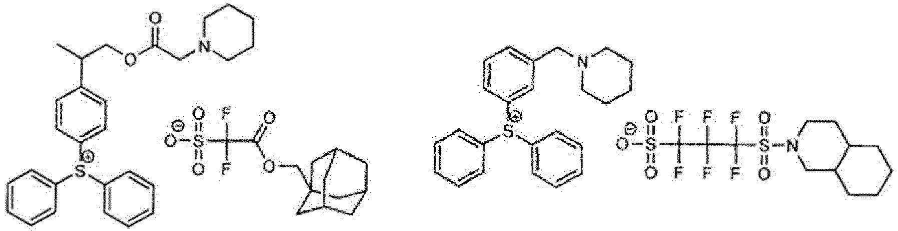

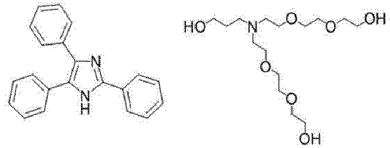

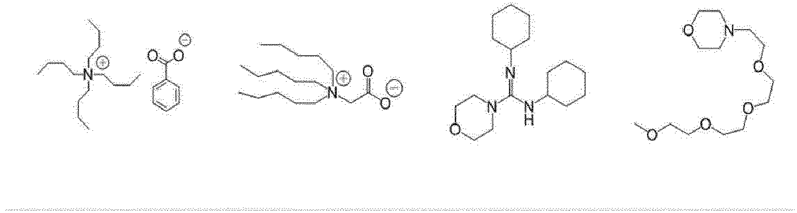

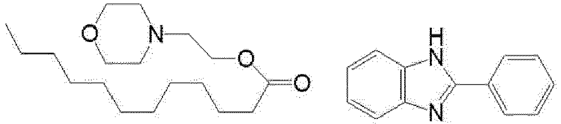

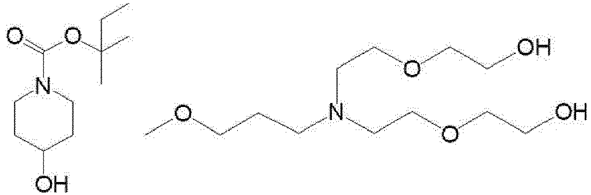

In a case where the resist composition includes an acid diffusion control agent, a content of the acid diffusion control agent is preferably 0.001% to 15% by mass, and more preferably 0.01% to 8% by mass with respect to a total solid content of the resist composition.

The acid diffusion control agents may be used alone or in combination of two or more kinds thereof.

In addition, in a case where the resist composition includes a specific compound having an anion represented by any of Formulae (d1-1) to (d1-3) and/or another photoacid generator having an anion represented by any of Formulae (d1-1) to (d1-3) (which are collectively referred to as a "d1-based photoacid generator"), the d1-based photoacid generator can also serve as an acid diffusion control agent. In a case where the resist composition includes the d1-based photoacid generator, it is also preferable that the resist composition does not substantially include an acid diffusion control agent. Here, the expression that the acid diffusion control agent is not substantially included means that a content of the acid diffusion control agent is 5% by mass or less with respect to a total content of the d1-based photoacid generator.

In addition, in a case where the resist composition includes both the d1-based photoacid generator and the acid diffusion control agent, a total content thereof is preferably 1% to 30% by mass, and more preferably 3% to 20% by mass.

It is preferable that a proportion of the photoacid generator and the acid diffusion control agent to be used in the resist composition, that is, the photoacid generator/the acid diffusion control agent (molar ratio) is 2.5 to 300. From the viewpoints of the sensitivity and the resolution, the molar ratio is preferably 2.5 or more, and from the viewpoint of suppressing a reduction in the resolution due to an increase in the thickness of a resist pattern over time after exposure until a heating treatment, the molar ratio is preferably 300 or less. The photoacid generator/the acid diffusion control agent (molar ratio) is more preferably 5.0 to 200, and still more preferably 7.0 to 150.

Examples of the acid diffusion control agent include the compounds (amine compounds, amide group-containing compounds, urea compounds, nitrogen-containing heterocyclic compounds, and the like) described in paragraphs [0140] to [0144] of JP2013-11833A.

### <Hydrophobic Resin>

The resist composition may include a hydrophobic resin different from the resin (A), in addition to the resin (A).

Although it is preferable that the hydrophobic resin is designed to be unevenly distributed on a surface of the resist film, it does not necessarily need to have a hydrophilic group in the molecule as different from the surfactant, and does not need to contribute to uniform mixing of polar materials and non-polar materials.

Examples of the effect caused by the addition of the hydrophobic resin include a control of static and dynamic contact angles of a surface of the resist film with respect to water and suppression of out gas.

The hydrophobic resin preferably has any one or more of a "fluorine atom", a "silicon atom", and a "CH₃ partial structure which is contained in a side chain moiety of a resin" from the viewpoint of uneven distribution on the film surface layer, and more preferably has two or more kinds thereof. In addition, the hydrophobic resin preferably has a hydrocarbon group having 5 or more carbon atoms. These groups may be contained in the main chain of the resin or may be substituted in a side chain.

In a case where hydrophobic resin includes a fluorine atom and/or a silicon atom, the fluorine atom and/or the silicon atom in the hydrophobic resin may be included in the main chain or a side chain of the resin.

In a case where the hydrophobic resin includes a fluorine atom, as a partial structure having a fluorine atom, an alkyl group having a fluorine atom, a cycloalkyl group having a fluorine atom, or an aryl group having a fluorine atom is preferable.

The alkyl group having a fluorine atom (preferably having 1 to 10 carbon atoms, and more preferably having 1 to 4 carbon atoms) is a linear or branched alkyl group in which at least one hydrogen atom is substituted with a fluorine atom, and the alkyl group may further have a substituent other than a fluorine atom.

The cycloalkyl group having a fluorine atom is a monocyclic or polycyclic cycloalkyl group in which at least one hydrogen atom is substituted with a fluorine atom, and may further have a substituent other than a fluorine atom.

Examples of the aryl group having a fluorine atom include an aryl group such as a phenyl group and a naphthyl group, in which at least one hydrogen atom is substituted with a fluorine atom, and the aryl group may further have a substituent other than a fluorine atom.

Examples of the repeating unit having a fluorine atom or a silicon atom include the repeating units exemplified in paragraph [0519] of US2012/0251948A1.

Furthermore, as described above, it is also preferable that the hydrophobic resin includes a CH₃ partial structure in a side chain moiety.

Here, the CH₃ partial structure contained in the side chain moiety in the hydrophobic resin includes a CH₃ partial structure contained in an ethyl group, a propyl group, and the like.

On the other hand, a methyl group bonded directly to the main chain of the hydrophobic resin (for example, an α-methyl group in the repeating unit having a methacrylic acid structure) makes only a small contribution of uneven distribution on the surface of the hydrophobic resin due to the effect of the main chain, and it is therefore not included in the CH₃ partial structure in the present invention.

The hydrophobic resin may have a repeating unit having an acid-decomposable group.

With regard to the hydrophobic resin, reference can be made to the description in paragraphs [0348] to [0415] of JP2014-010245A, the contents of which are incorporated herein by reference.

Furthermore, for the hydrophobic resin, the resins described in JP2011-248019A, JP2010-175859A, and JP2012-032544A can also be preferably used, in addition to the resins described above.

Preferred examples of a monomer corresponding to the repeating unit constituting the hydrophobic resin are shown below.

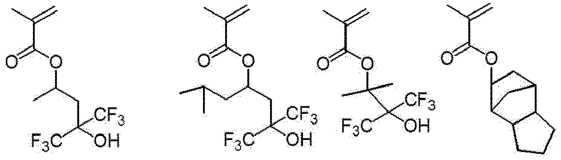

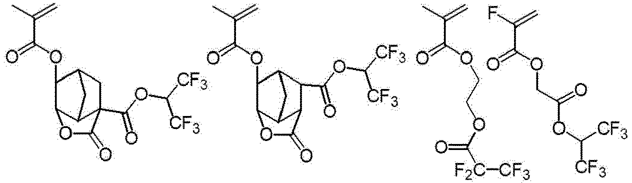

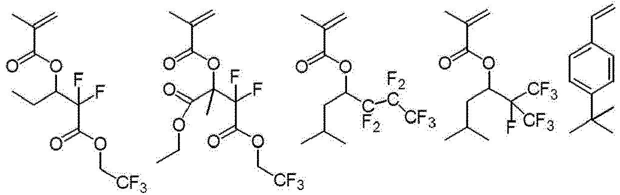

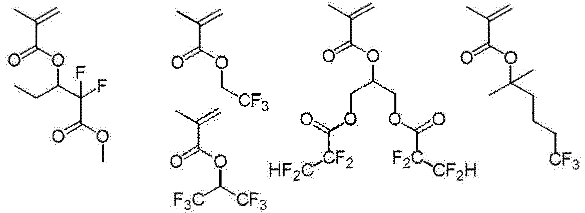

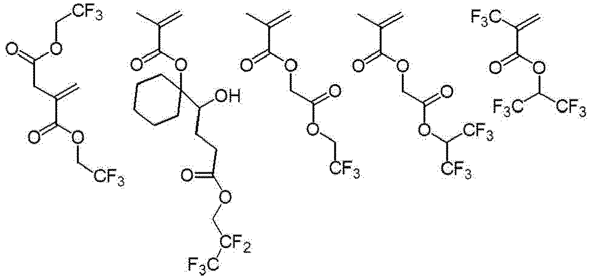

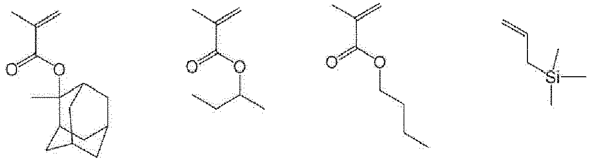

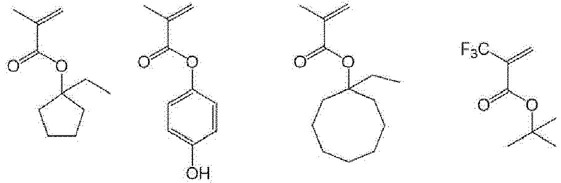

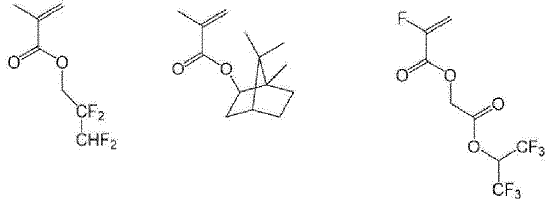

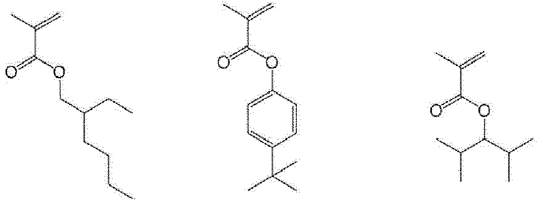

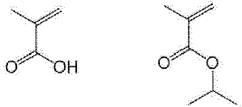

In a case where the resist composition includes a hydrophobic resin, a content of the hydrophobic resin is preferably 0.01% to 20% by mass, and more preferably 0.1% to 15% by mass with respect to the total solid content of the resist composition.

### <Surfactant>

The resist composition may include a surfactant. In a case where the surfactant is included, it is possible to form a pattern having more excellent adhesiveness and fewer development defects.

The surfactant is preferably a fluorine-based and/or silicon-based surfactant.

Examples of the fluorine-based and/or silicon-based surfactant include the surfactants described in paragraph [0276] of the specification of US2008/0248425A. In addition, EFTOP EF301 or EF303 (manufactured by Shin-Akita Chemical Co., Ltd.); FLUORAD FC430, 431, and 4430 (manufactured by Sumitomo 3M inc.); MEGAFACE F171, F173, F176, F189, F113, F110, F177, F120, and R08 (manufactured by DIC Corporation); SURFLON S-382, SC101, 102, 103, 104, 105, or 106 (manufactured by Asahi Glass Co., Ltd.); TROYSOL S-366 (manufactured by Troy Corporation); GF-300 or GF-150 (manufactured by Toagosei Co., Ltd.); SURFLON S-393 (manufactured by AGC Seimi Chemical Co., Ltd.); EFTOP EF121, EF122A, EF122B, RF122C, EF125M, EF135M, EF351, EF352, EF801, EF802, or EF601 (manufactured by JEMCO Inc.); PF636, PF656, PF6320, and PF6520 (manufactured by OMNOVA Solutions Inc.); KH-20 (manufactured by Asahi Kasei Corporation); or FTX-204G, 208G, 218G, 230G, 204D, 208D, 212D, 218D, and 222D (manufactured by NEOS COMPANY LIMITED) may be used. In addition, a polysiloxane polymer KP-341 (manufactured by Shin-Etsu Chemical Co., Ltd.) can also be used as the silicon-based surfactant.

Moreover, in addition to the known surfactants as shown above, a surfactant may be synthesized using a fluoroaliphatic compound manufactured using a telomerization method (also referred to as a telomer method) or an oligomerization method (also referred to as an oligomer method). Specifically, a polymer including a fluoroaliphatic group derived from fluoroaliphatic compound may be used as the surfactant. This fluoroaliphatic compound can be synthesized, for example, by the method described in JP2002-90991A.

In addition, a surfactant other than the fluorine-based surfactant and/or the silicon-based surfactants described in paragraph [0280] of the specification of US2008/0248425A may be used.

The surfactants may be used alone or in combination of two or more kinds thereof.

In a case where the resist composition includes a surfactant, a content of the surfactant is preferably 0.0001% to 2% by mass, and more preferably 0.0005% to 1% by mass with respect to the total solid content of the composition.

### <Other Additives>

The resist composition may further include, in addition to the components, a dissolution inhibiting compound, a dye, a plasticizer, a photosensitizer, a light absorber, and/or a compound promoting a solubility in a developer (for example, a phenol compound having a molecular weight of 1,000 or less or an alicyclic or aliphatic compound including a carboxylic acid group), or the like.

The resist composition may further include a dissolution inhibiting compound. Here, the "dissolution inhibiting compound" is intended to be a compound having a molecular weight of 3,000 or less, whose solubility in an organic developer decreases by decomposition by the action of an acid.

### [Resist Film and Pattern Forming Method]

The procedure of the pattern forming method using the resist composition is not particularly limited, but preferably has the following steps.
Step 1: A step of forming a resist film on a substrate, using a resist composition
Step 2: A step of exposing the resist film
Step 3: A step of developing the exposed resist film, using a developer, to form a pattern

Hereinafter, the procedure of each of the steps will be described in detail.

### <Step 1: Resist Film Forming Step>

The step 1 is a step of forming a resist film on a substrate, using a resist composition.

The definition of the resist composition is as described above.

Examples of a method in which a resist film is formed on a substrate, using a resist composition, include a method in which a resist composition is applied onto a substrate.

In addition, it is preferable that the resist composition before the application is filtered through a filter, as desired. A pore size of the filter is preferably 0.1 µm or less, more preferably 0.05 µm or less, and still more preferably 0.03 µm or less. In addition, the filter is preferably a polytetrafluoroethylene-, polyethylene-, or nylon-made filter.

The resist composition can be applied onto a substrate (for example, silicon and silicon dioxide coating) as used in the manufacture of integrated circuit elements by a suitable application method such as ones using a spinner or a coater. The application method is preferably spin application using a spinner. A rotation speed upon the spin application using a spinner is preferably 1,000 to 3,000 rpm.

After the application of the resist composition, the substrate may be dried to form a resist film. In addition, various underlying films (an inorganic film, an organic film, or an antireflection film) may be formed on the underlayer of the resist film.

Examples of the drying method include a method of heating and drying. The heating can be carried out using a unit included in an ordinary exposure machine and/or an ordinary development machine, and may also be carried out using a hot plate or the like. A heating temperature is preferably 80°C to 150°C, more preferably 80°C to 140°C, and still more preferably 80°C to 130°C. A heating time is preferably 30 to 1,000 seconds, more preferably 60 to 800 seconds, and still more preferably 60 to 600 seconds.

A film thickness of the resist film is not particularly limited, but is preferably 10 to 65 nm, and more preferably 15 to 50 nm from the viewpoint that a fine pattern having higher accuracy can be formed.

Moreover, a topcoat may be formed on the upper layer of the resist film, using the topcoat composition.

It is preferable that the topcoat composition is not mixed with the resist film and can be uniformly applied onto the upper layer of the resist film.

The topcoat composition includes, for example, a resin, an additive, and a solvent.

As the resin, the same resin as the above-mentioned hydrophobic resin can be used. A content of the resin is preferably 50% to 99.9% by mass, and more preferably 60% to 99.7% by mass with respect to a total solid content of the topcoat composition.

As the additive, the above-mentioned acid diffusion control agent and d1-based photoacid generator can be used. In addition, a compound having a radical trapping group such as a compound having an N-oxy free radical group can also be used. Examples of such a compound include a [4-(benzoyloxy)-2,2,6,6-tetramethylpiperidinooxy] radical. A content of the additive is preferably 0.01% to 20% by mass, and more preferably 0.1% to 15% by mass with respect to the total solid content of the topcoat composition.

It is preferable that the solvent does not dissolve a resist film, and examples of the solvent include an alcohol-based solvent (4-methyl-2-pentanol and the like), an ether-based solvent (diisoamyl ether and the like), an ester-based solvent, a fluorine-based solvent, and a hydrocarbon-based solvent (n-decane and the like).

A content of the solvent in the topcoat composition is preferably set so that the concentration of solid contents is 0.5% to 30% by mass, and more preferably set so that the concentration of solid contents is 1% to 20% by mass.

In addition, the topcoat composition may include a surfactant in addition to the above-mentioned additive, and as the surfactant, a surfactant which may be included in the composition of the embodiment of the present invention can be used. A content of the surfactant is preferably 0.0001% to 2% by mass, and more preferably 0.0005% to 1% by mass with respect to the total solid content of the topcoat composition.

In addition, the topcoat is not particularly limited, a topcoat known in the related art can be formed by the methods known in the related art, and the topcoat can be formed, based on the description in paragraphs [0072] to [0082] of JP2014-059543A, for example.

It is preferable that a topcoat including a basic compound as described in JP2013-61648A, for example, is formed on a resist film. Specific examples of the basic compound which can be included in the topcoat include a basic compound which may be included in the resist composition.

In addition, it is also preferable that the topcoat includes a compound which includes at least one group or bond selected from the group consisting of an ether bond, a thioether bond, a hydroxyl group, a thiol group, a carbonyl bond, and an ester bond.

### <Step 2: Exposing Step>

The step 2 is a step of exposing the resist film.

Examples of an exposing method include a method in which a resist film formed is irradiated with EUV light through a predetermined mask.

It is preferable to perform baking (heating) before performing development after the exposure. The baking accelerates a reaction in the exposed area, and the sensitivity and the pattern shape are improved.

A heating temperature is preferably 80°C to 150°C, more preferably 80°C to 140°C, and still more preferably 80°C to 130°C.

A heating time is preferably 10 to 1,000 seconds, more preferably 10 to 180 seconds, and still more preferably 30 to 120 seconds.

The heating can be carried out using a unit included in an ordinary exposure machine and/or an ordinary development machine, and may also be performed using a hot plate or the like.

This step is also referred to as a post-exposure baking.

### <Step 3: Developing Step>

The step 3 is a step of developing the exposed resist film, using a developer, to form a pattern.

The developer may be either an alkali developer or a developer containing an organic solvent (hereinafter also referred to as an organic developer).

Examples of the developing method include a method in which a substrate is immersed in a tank filled with a developer for a certain period of time (a dip method), a method in which development is performed by heaping a developer up onto the surface of a substrate by surface tension, and then leaving it to stand for a certain period of time (a puddle method), a method in which a developer is sprayed on the surface of a substrate (a spray method), and a method in which a developer is continuously jetted onto a substrate rotating at a constant rate while scanning a developer jetting nozzle at a constant rate (a dynamic dispense method).

Furthermore, after the step of performing development, a step of stopping the development may be carried out while substituting the solvent with another solvent.

A developing time is not particularly limited as long as it is a period of time where the unexposed area of a resin is sufficiently dissolved, and is preferably 10 to 300 seconds, and more preferably 20 to 120 seconds.

The temperature of the developer is preferably 0°C to 50°C, and more preferably 15°C to 35°C.

As the alkali developer, it is preferable to use an aqueous alkali solution including an alkali. The type of the aqueous alkali solution is not particularly limited, but examples thereof include an aqueous alkali solution including a quaternary ammonium salt typified by tetramethylammonium hydroxide, an inorganic alkali, a primary amine, a secondary amine, a tertiary amine, an alcoholamine, a cyclic amine, or the like. Among those, the aqueous solutions of the quaternary ammonium salts typified by tetramethylammonium hydroxide (TMAH) are preferable as the alkali developer. An appropriate amount of an alcohol, a surfactant, or the like may be added to the alkali developer. The alkali concentration of the alkali developer is usually 0.1% to 20% by mass. Furthermore, the pH of the alkali developer is usually 10.0 to 15.0.

The organic developer is preferably a developer containing at least one organic solvent selected from the group consisting of a ketone-based solvent, an ester-based solvent, an alcohol-based solvent, an amide-based solvent, an ether-based solvent, and a hydrocarbon-based solvent.

Examples of the ketone-based solvent include 1-octanone, 2-octanone, 1-nonanone, 2-nonanone, acetone, 2-heptanone (methyl amyl ketone), 4-heptanone, 1-hexanone, 2-hexanone, diisobutyl ketone, cyclohexanone, methylcyclohexanone, phenyl acetone, methyl ethyl ketone, methyl isobutyl ketone, acetyl acetone, acetonyl acetone, ionone, diacetonyl alcohol, acetyl carbinol, acetophenone, methyl naphthyl ketone, isophorone, and propylene carbonate.

Examples of the ester-based solvent include methyl acetate, butyl acetate, ethyl acetate, isopropyl acetate, pentyl acetate, isopentyl acetate, amyl acetate, propylene glycol monomethyl ether acetate, ethylene glycol monoethyl ether acetate, diethylene glycol monobutyl ether acetate, diethylene glycol monoethyl ether acetate, ethyl-3-ethoxypropionate, 3-methoxybutyl acetate, 3-methyl-3-methoxybutyl acetate, methyl formate, ethyl formate, butyl formate, propyl formate, ethyl lactate, butyl lactate, propyl lactate, butyl butyrate, methyl 2-hydroxyisobutyrate, isoamyl acetate, isobutyl isobutyrate, and butyl propionate.

As the alcohol-based solvent, the amide-based solvent, the ether-based solvent, and the hydrocarbon-based solvent, for example, the solvents disclosed in paragraphs [0715] to [0718] of the specification of US2016/0070167A1 can be used.

A plurality of the solvents may be mixed or the solvent may be used in admixture with a solvent other than those described above or water. The moisture content in the entire developer is preferably less than 50% by mass, more preferably less than 20% by mass, and still more preferably less than 10% by mass, and particularly preferably moisture is not substantially contained.

A content of the organic solvent with respect to the organic developer is preferably from 50% by mass to 100% by mass, more preferably from 80% by mass to 100% by mass, still more preferably from 90% by mass to 100% by mass, and particularly preferably from 95% by mass to 100% by mass with respect to the total amount of the developer.

### <Other Steps>

It is preferable that the pattern forming method includes a step of performing washing using a rinsing liquid after the step 3.

Examples of the rinsing liquid used in the rinsing step after the step of performing development using an alkali developer include pure water. Furthermore, an appropriate amount of a surfactant may be added to pure water.

An appropriate amount of a surfactant may be added to the rinsing liquid.

The rinsing liquid used in the rinsing step after the developing step with an organic developer is not particularly limited as long as the rinsing liquid does not dissolve the pattern, and a solution including a common organic solvent can be used. As the rinsing liquid, a rinsing liquid containing at least one organic solvent selected from the group consisting of a hydrocarbon-based solvent, a ketone-based solvent, an ester-based solvent, an alcohol-based solvent, an amide-based solvent, and an ether-based solvent is preferably used.

Examples of the hydrocarbon-based solvent, the ketone-based solvent, the ester-based solvent, the alcohol-based solvent, the amide-based solvent, and the ether-based solvent include the same as those described for the developer including an organic solvent.

A method for the rinsing step is not particularly limited, and examples thereof include a method in which a rinsing liquid is continuously jetted on a substrate rotated at a constant rate (a rotation application method), a method in which a substrate is immersed in a tank filled with a rinsing liquid for a certain period of time (a dip method), and a method in which a rinsing liquid is sprayed on a substrate surface (a spray method).

Furthermore, the pattern forming method of the embodiment of the present invention may include a heating step (postbaking) after the rinsing step. By the present step, the developer and the rinsing liquid remaining between and inside the patterns are removed by baking. In addition, the present step also has an effect that a resist pattern is annealed and the surface roughness of the pattern is improved. The heating step after the rinsing step is usually performed at 40°C to 250°C (preferably 90°C to 200°C) for usually 10 seconds to 3 minutes (preferably 30 seconds to 120 seconds).

In addition, an etching treatment on the substrate may be carried out using a pattern formed as a mask. That is, the substrate (or the underlayer film and the substrate) may be processed using the pattern thus formed in the step 3 as a mask to form a pattern on the substrate.

A method for processing the substrate (or the underlayer film and the substrate) is not particularly limited, but a method in which a pattern is formed on a substrate by subjecting the substrate (or the underlayer film and the substrate) to dry etching using the pattern thus formed in the step 3 as a mask is preferable.

The dry etching may be one-stage etching or multi-stage etching. In a case where the etching is etching including a plurality of stages, the etchings at the respective stages maybe the same treatment or different treatments.

For etching, any of known methods can be used, and various conditions and the like are appropriately determined according to the type of a substrate, usage, and the like. Etching can be carried out, for example, in accordance with Journal of The International Society for Optical Engineering (Proc. of SPIE), Vol. 6924, 692420 (2008), JP2009-267112A, and the like. In addition, the etching can also be carried out in accordance with "Chapter 4 Etching" in "Semiconductor Process Text Book, 4th Ed., published in 2007, publisher: SEMI Japan".

Among those, oxygen plasma etching is preferable as the dry etching.

It is preferable that various materials (for example, a solvent, a developer, a rinsing liquid, a composition for forming an antireflection film, and a composition for forming a topcoat) used in the resist composition and the pattern forming method of the embodiment of the present invention do not include impurities such as metals. A content of the impurities included in these materials is preferably 1 ppm by mass or less, more preferably 10 ppb by mass or less, still more preferably 100 ppt by mass or less, particularly preferably 10 ppt by mass or less, and most preferably 1 ppt by mass or less. Here, examples of the metal impurities include Na, K, Ca, Fe, Cu, Mg, Al, Li, Cr, Ni, Sn, Ag, As, Au, Ba, Cd, Co, Pb, Ti, V, W, and Zn.

Examples of a method for removing impurities such as metals from the various materials include filtration using a filter. As for the filter pore diameter, the pore size is preferably less than 100 nm, more preferably 10 nm or less, and still more preferably 5 nm or less. As the filter, a polytetrafluoroethylene-made, polyethylene-made, or nylon-made filter is preferable. The filter may include a composite material in which the filter material is combined with an ion exchange medium. As the filter, a filter which has been washed with an organic solvent in advance may be used. In the step of filter filtration, plural kinds of filters connected in series or in parallel may be used. In a case of using the plural kinds of filters, a combination of filters having different pore diameters and/or materials may be used. In addition, various materials may be filtered plural times, and the step of filtering plural times may be a circulatory filtration step.

In the production of a resist composition, for example, it is preferable to dissolve the respective components such as a resin and a photoacid generator in a solvent, and then perform circulatory filtration using a plurality of filters having different materials. For example, it is preferable to connect a polyethylene-made filter with a pore diameter of 50 nm, a nylon-made filter with a pore diameter of 10 nm, and a polyethylene-made filter with a pore diameter of 3 nm in permuted connection, and then perform circulatory filtration ten times or more. A smaller pressure difference among the filters is more preferable, and the pressure difference is generally 0.1 MPa or less, preferably 0.05 MPa or less, and more preferably 0.01 MPa or less. A smaller pressure difference between the filter and the charging nozzle is more preferable, and the pressure difference is generally 0.5 MPa or less, preferably 0.2 MPa or less, and more preferably 0.1 MPa or less.

It is preferable to subject the inside of a device for producing the resist composition to gas replacement with an inert gas such as nitrogen. This makes it possible to suppress the dissolution of an active gas such as oxygen in the resist composition.

After being filtered by a filter, the resist composition is charged into a clean container. It is preferable that the resist composition charged in the container is subjected to cold storage. This enables performance deterioration caused by the lapse of time to be suppressed. A shorter time from completion of charging the composition into the container to initiation of cold storage is more preferable, and the time is generally 24 hours or shorter, preferably 16 hours or shorter, more preferably 12 hours or shorter, and still more preferably 10 hours or shorter. The storage temperature is preferably 0°C to 15°C, more preferably 0°C to 10°C, and still more preferably 0°C to 5°C.

In addition, examples of a method for reducing impurities such as metals included in various materials include a method of selecting raw materials having a low content of metals as raw materials constituting various materials, a method of subjecting raw materials constituting various materials to filter filtration, and a method of performing distillation under the condition for suppressing the contamination as much as possible by, for example, lining the inside of a device with TEFLON (registered trademark).

In addition to the filter filtration, removal of impurities by an adsorbing material may be performed, or a combination of filter filtration and an adsorbing material may be used. As the adsorbing material, known adsorbing materials may be used, and for example, inorganic adsorbing materials such as silica gel and zeolite, and organic adsorbing materials such as activated carbon can be used. It is necessary to prevent the incorporation of impurities such as metals in the production process in order to reduce the metal impurities included in the various materials. Sufficient removal of metal impurities from a production device can be confirmed by measuring the content of metal components included in a washing liquid used to wash the production device. A content of the metal components included in the washing liquid after the use is preferably 100 parts per trillion (ppt) by mass or less, more preferably 10 ppt by mass or less, and still more preferably 1 ppt by mass or less.

A conductive compound may be added to an organic treatment liquid such as a rinsing liquid in order to prevent breakdown of chemical liquid pipes and various parts (a filter, an O-ring, a tube, or the like) due to electrostatic charging, and subsequently generated electrostatic discharging. The conductive compound is not particularly limited, but examples thereof include methanol. The addition amount is not particularly limited, but from the viewpoint that preferred development characteristics or rinsing characteristics are maintained, the addition amount is preferably 10% by mass or less, and more preferably 5% by mass or less.

For members of the chemical liquid pipe, for example, various pipes coated with stainless steel (SUS), or a polyethylene, polypropylene, or fluorine resin (a polytetrafluoroethylene or perfluoroalkoxy resin, or the like) that has been subjected to an antistatic treatment can be used. In the same manner, for the filter or the O-ring, polyethylene, polypropylene, or a fluorine resin (a polytetrafluoroethylene or perfluoroalkoxy resin, or the like) that has been subjected to an antistatic treatment can be used.

A method for improving the surface roughness of a pattern may be applied to a pattern formed by the method of the embodiment of the present invention. Examples of the method for improving the surface roughness of the pattern include the method of treating a pattern by a plasma of a hydrogen-containing gas disclosed in WO2014/002808A. Additional examples of the method include known methods as described in JP2004-235468A, US2010/0020297A, JP2008-83384A, and Proc. of SPIE Vol. 8328 83280N-1 "EUV Resist Curing Technique for LWR Reduction and Etch Selectivity Enhancement".

In a case where a pattern formed is in the form of a line, an aspect ratio determined by dividing the height of the pattern with the line width is preferably 2.5 or less, more preferably 2.1 or less, and still more preferably 1.7 or less.

In a case where a pattern formed is in the form of a trench (groove) pattern or a contact hole pattern, an aspect ratio determined by dividing the height of the pattern with the trench width or the hole diameter is preferably 4.0 or less, more preferably 3.5 or less, and still more preferably 3.0 or less.

The pattern forming method of the embodiment of the present invention can also be used for forming a guide pattern in a directed self-assembly (DSA) (see, for example, ACS Nano Vol. 4, No. 8, Pages 4815-4823).

In addition, a pattern formed by the method can be used as a core material (core) of the spacer process disclosed in, for example, JP1991-270227A (JP-H03-270227A) and JP2013-164509A.

Moreover, the present invention further relates to a method for manufacturing an electronic device, including the pattern forming method, and an electronic device manufactured by the manufacturing method.

The electronic device of an embodiment of the present invention is suitably mounted on electric and electronic equipment (for example, home appliances, office automation (OA)-related equipment, media-related equipment, optical equipment, telecommunication equipment, and the like).

### Examples

Hereinbelow, the present invention will be described in more detail with reference to Examples. The materials, the amounts of materials used, the proportions, the treatment details, the treatment procedure, and the like shown in Examples below may be modified as appropriate as long as the modifications do not depart from the spirit of the present invention. Therefore, the scope of the present invention should not be construed as being limited to Examples shown below.

### [Production of Composition]

Components included in actinic ray-sensitive or radiation-sensitive resin composition (hereinafter also referred to as a "resist composition") used in Examples or Comparative Examples, and production procedure therefor are shown below.

### <Photoacid Generator (Specific Compound and Comparative Compound)>

### (Synthesis of Specific Compound A-3)

A specific compound A-3 was synthesized based on the following scheme.

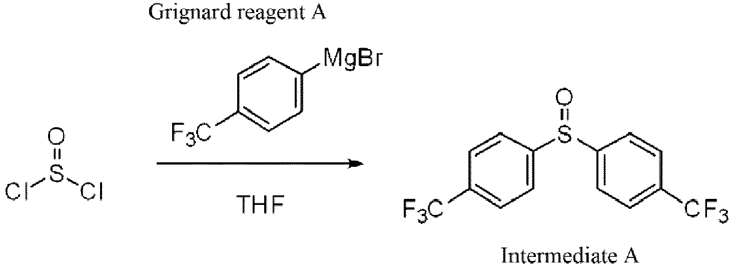

Magnesium (18.0 g) was added to tetrahydrofuran (500 mL) to obtain a mixture. 4-Bromobenzotrifluoride (151.5 g) was added dropwise to the obtained mixture. Then, the mixture was stirred for 1 hour to prepare a Grignard reagent A.

Thionyl chloride (37.7 g) was added to tetrahydrofuran (500 mL) to obtain a mixed liquid. The obtained mixed liquid was cooled to 0°C, and the Grignard reagent A prepared above was added dropwise to the mixed liquid. After stirring the mixed liquid for 1 hour, 1 N hydrochloric acid (600 mL) was added to the mixed liquid while maintaining the temperature of the mixed liquid at 0°C.

A reaction product produced in the mixed liquid was extracted with ethyl acetate (600 mL). The obtained organic phase was washed with a saturated aqueous sodium hydrogen carbonate solution (500 mL) and water (500 mL), and then the solvent was evaporated from the organic phase. The obtained concentrate was washed with hexane (300 mL) and filtered to obtain an intermediate A (60 g) as a filtrate (yield 56%).

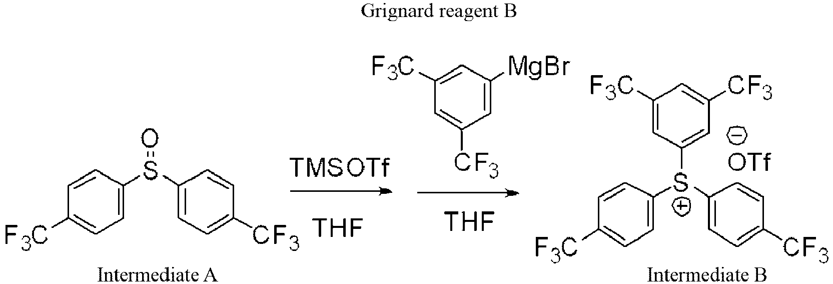

Magnesium (5.0 g) was added to tetrahydrofuran (170 mL) to obtain a mixture. 1-Bromo-3,5-bis(trifluoromethyl)benzene (52.0 g) was added dropwise to the obtained mixture. Then, the mixture was stirred for 1 hour to prepare a Grignard reagent B.

The intermediate A (20.0 g) was added to tetrahydrofuran (170 mL) to obtain a mixed liquid. The obtained mixed liquid was cooled to 0°C, and trimethylsilyl trifluoromethanesulfonate (91.9 g) was added dropwise to the mixed liquid. Subsequently, the Grignard reagent B prepared above was added dropwise to the mixed liquid, and then the mixed liquid was stirred for 1 hour.

Water (500 mL) was added to the mixed liquid while maintaining the temperature of the mixed liquid at 0°C, and then a reaction product produced in the mixed liquid was extracted with methylene chloride (500 mL). The obtained organic phase was washed with water (500 mL), and then the solvent was evaporated from the organic phase. The obtained concentrate was washed with diisopropyl ether (300 mL) and filtered to obtain an intermediate B (20 g) as a filtrate (yield 58%).

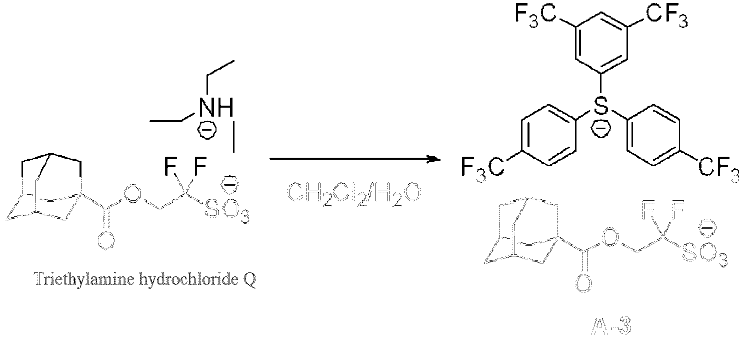

Methylene chloride (100 mL) and water (100 mL) were mixed to obtain a mixed liquid. Triethylamine hydrochloride Q (10.0 g) and the intermediate B (16.1 g) were added to the mixed liquid. After stirring the mixed liquid for 1 hour, the aqueous phase was removed from the mixed liquid. The remaining organic phase was washed with a 1%-by-mass aqueous potassium carbonate solution (100 mL), 0.01 N hydrochloric acid (100 mL), and water (100 mL). The solvent was evaporated from the organic phase to obtain a specific compound A-3 (20 g) (yield 99%).

Other specific compounds were synthesized with reference to the synthesis method. Photoacid generators used in Examples are shown below.

In addition, A-1 to A-13 and B-2 to B-5 correspond to the specific compounds, and B-1 and Z-1 to Z-2 do not correspond to the specific compounds.

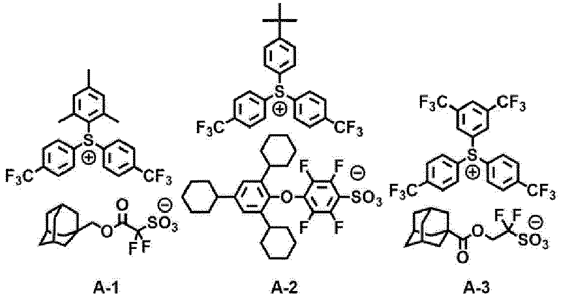

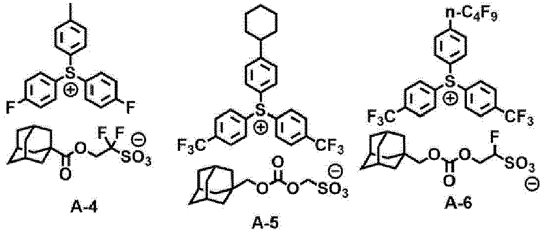

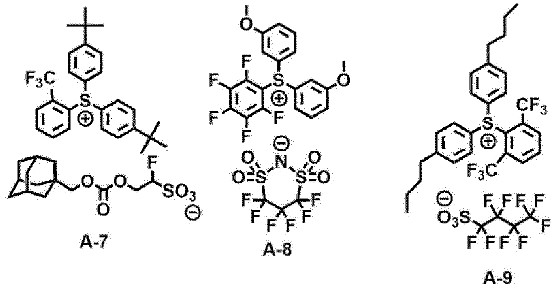

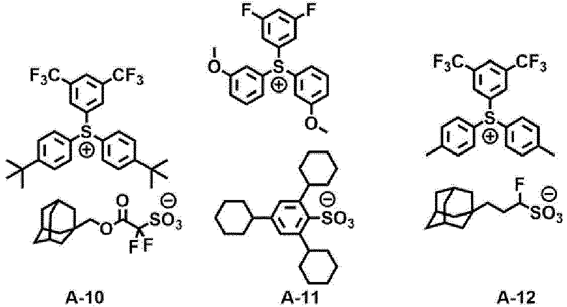

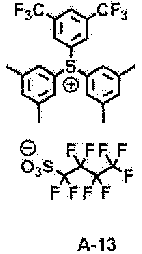

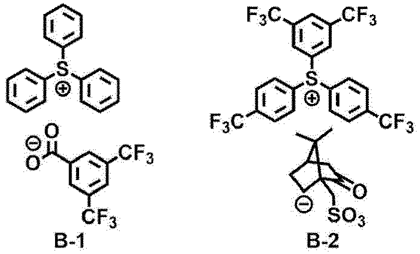

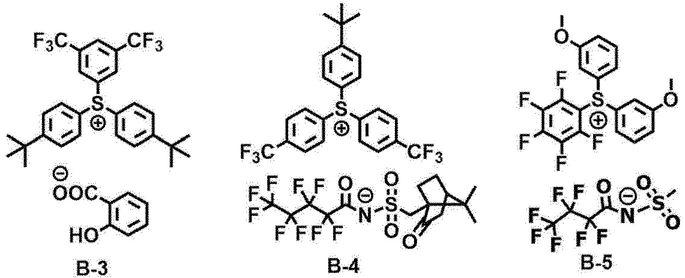

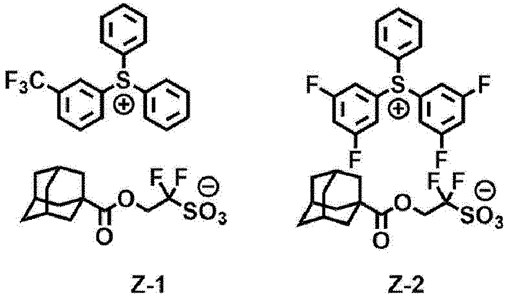

### <Acid-Decomposable Resin (Resin (A))>

Acid-decomposable resins (resins (A)) used for producing a resist composition are shown below.

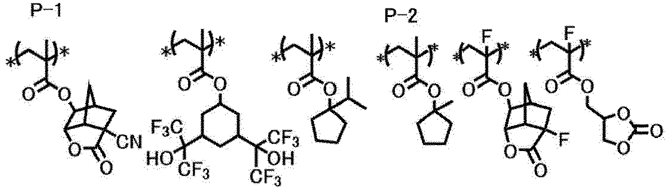

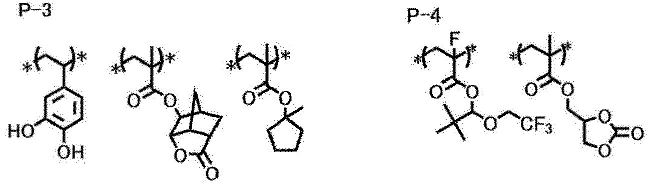

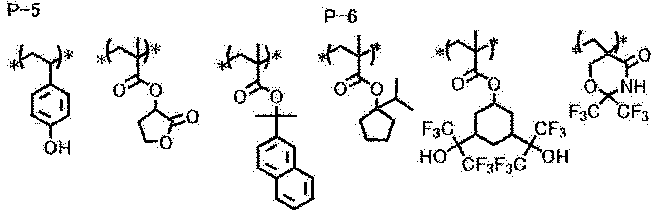

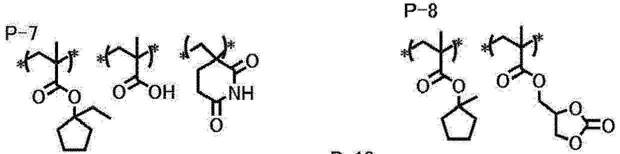

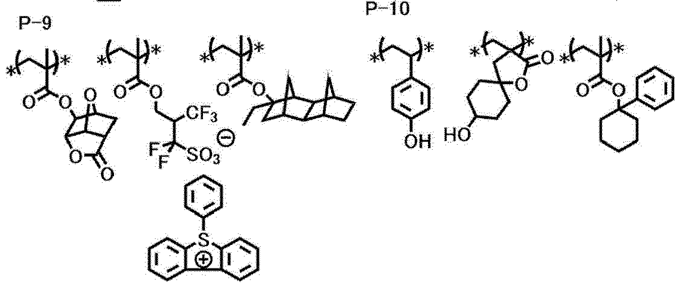

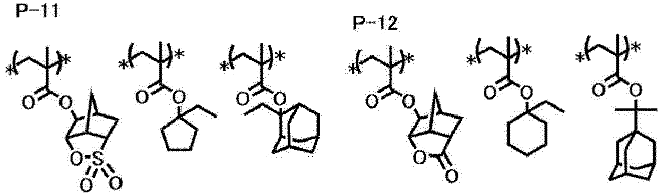

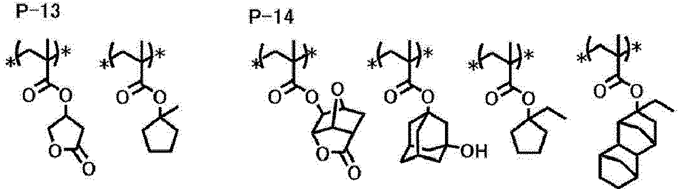

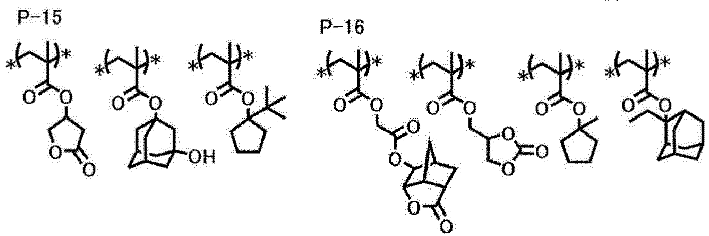

The molar ratios of the repeating units constituting each resin shown above (corresponding in order from the left), and the weight-average molecular weight (Mw) and the dispersity (Mw/Mn) of each resin are shown in the following table.

**[Table 1]**

| Table 1 | Molar ratio of repeating unit | | | | Mw | Mw/Mn |
|---|---|---|---|---|---|---|
| Resin P-1 | 25 | 30 | 45 | - | 6,000 | 1.71 |
| Resin P-2 | 40 | 15 | 45 | - | 10,200 | 1.64 |
| Resin P-3 | 40 | 20 | 40 | - | 7,500 | 1.54 |
| Resin P-4 | 40 | 60 | - | - | 6,800 | 1.52 |
| Resin P-5 | 40 | 10 | 50 | - | 6,500 | 1.63 |
| Resin P-6 | 45 | 40 | 15 | - | 5,900 | 1.59 |
| Resin P-7 | 40 | 20 | 40 | - | 5,100 | 1.51 |
| Resin P-8 | 60 | 40 | - | - | 6,200 | 1.39 |
| Resin P-9 | 40 | 20 | 40 | - | 7,000 | 1.73 |
| Resin P-10 | 40 | 15 | 45 | - | 11,300 | 1.51 |
| Resin P-11 | 15 | 45 | 40 | - | 6,400 | 1.51 |
| Resin P-12 | 50 | 30 | 20 | - | 8,000 | 1.65 |
| Resin P-13 | 50 | 50 | - | - | 7,600 | 1.49 |
| Resin P-14 | 30 | 50 | 10 | 10 | 5,000 | 1.61 |
| Resin P-15 | 40 | 10 | 50 | - | 12,000 | 1.49 |
| Resin P-16 | 25 | 30 | 30 | 15 | 8,500 | 1.61 |

### <Acid Diffusion Control Agent>

In a case where the resist composition included an acid diffusion control agent, the following acid diffusion control agent was used.

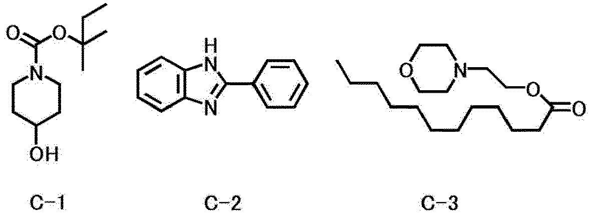

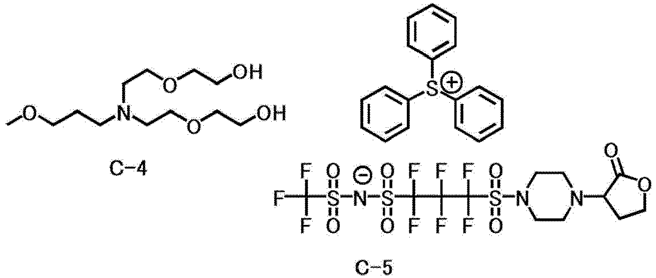

### <Hydrophobic Resin>

In a case where the resist composition included a hydrophobic resin, a hydrophobic resin having a repeating unit based on the following monomer was used.

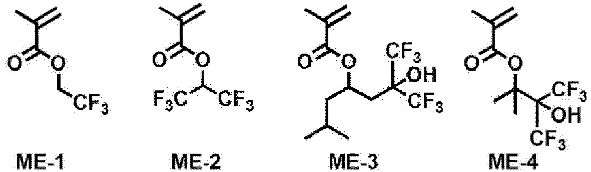

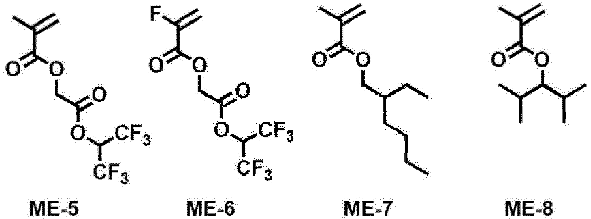

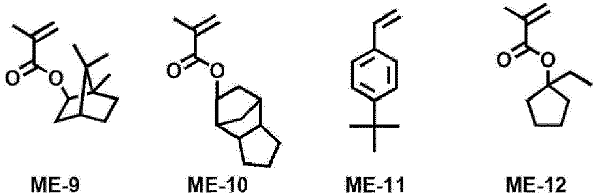

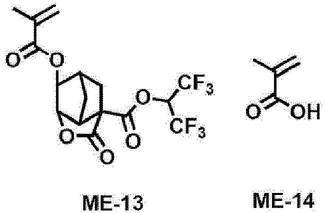

The molar ratios of the repeating units based on the respective monomers, and the weight-average molecular weight (Mw) and the dispersity (Mw/Mn) of each resin in the hydrophobic resin used in the composition are shown in the following table.

**[Table 2]**

| Table 2 | Molar ratio of repeating unit 1 | | Molar ratio of repeating unit 2 | | Molar ratio of repeating unit 3 | | Molar ratio of repeating unit 4 | | Mw | Mw/Mn |
|---|---|---|---|---|---|---|---|---|---|---|
| Resin D-1 | ME-12 | 50 | ME-1 | 50 | - | - | - | - | 12,000 | 1.5 |
| Resin D-2 | ME-2 | 40 | ME-11 | 50 | ME-7 | 5 | ME-14 | 5 | 6,000 | 1.3 |
| Resin D-3 | ME-8 | 50 | ME-2 | 50 | - | - | - | - | 15,000 | 1.5 |
| Resin D-4 | ME-5 | 100 | - | - | - | - | - | - | 23,000 | 1.7 |
| Resin D-5 | ME-11 | 10 | ME-13 | 85 | ME-7 | 5 | - | - | 11,000 | 1.4 |
| Resin D-6 | ME-6 | 80 | ME-9 | 20 | - | - | - | - | 13,000 | 1.4 |

### <Surfactant>

In a case where the composition included a surfactant, the following surfactants were used.

E-1: MEGAFACE F176 (manufactured by DIC Corporation, fluorine-based surfactant) E-2: MEGAFACE R08 (manufactured by DIC Corporation, a fluorine- and silicon-based surfactant) E-3: PF656 (manufactured by OMNOVA Solutions Inc., a fluorine-based surfactant)

### <Solvent>

Solvents included in the composition are shown below.
F-1: Propylene glycol monomethyl ether acetate (PGMEA)
F-2: Propylene glycol monomethyl ether (PGME)
F-3: Propylene glycol monoethyl ether (PGEE)
F-4: Cyclohexanone
F-5: Cyclopentanone
F-6: 2-Heptanone
F-7: Ethyl lactate
F-8: γ-Butyrolactone
F-9: Propylene carbonate

### <Preparation of Composition>

### (Preparation of Composition for EUV Exposure Test (Re-1 to Re-22))

The respective components shown in the following table were mixed so that the concentration of solid contents was 2% by mass. Next, the obtained mixed liquid was filtered initially through a polyethylene-made filter having a pore diameter of 50 nm, then through a nylon-made filter having a pore diameter of 10 nm, and lastly through a polyethylene-made filter having a pore diameter of 5 nm in this order to prepare a resist composition used for a test by EUV exposure (actinic ray-sensitive or radiation-sensitive resin composition).

In addition, in the resist composition, the solid content means all the components excluding the solvent. The obtained resist composition was used in Examples and Comparative Examples.

In addition, in the following table, the content (% by mass) of each component means a content with respect to the total solid content.

### (Preparation of Composition for ArF Exposure Test (Re-23 to Re-34))

The respective components shown in the following table were mixed so that the concentration of solid contents was 4% by mass. Next, the obtained mixed liquid was filtered initially through a polyethylene-made filter having a pore diameter of 50 nm, then through a nylon-made filter having a pore diameter of 10 nm, and lastly through a polyethylene-made filter having a pore diameter of 5 nm in this order to prepare a resist composition used for a test by ArF exposure (actinic ray-sensitive or radiation-sensitive resin composition).

The formulation of each composition is shown below.

**[Table 3]**

| Table 3 | Solid content | | | | | | | | | | | | Solvent | |
|---|---|---|---|---|---|---|---|---|---|---|---|---|---|---|
| | Resin | | Photoacid generator 1 | | Photoacid generator 2 | | Acid diffusion control agent | | Hydrophobic resin | | Surfactant | | | |
| | Type | % by mass | Type | % by mass | Type | % by mass | Type | % by mass | Type | % by mass | Type | % by mass | Type | Mixing ratio |
| Re-1 | P-1 | 84.0 | A-1 | 14.5 | - | - | C-1 | 1.5 | | | - | - | F-1/F-2 | 70/30 |
| Re-2 | P-6 | 82.0 | A-2 | 16.2 | - | - | C-1 | 1.6 | | | E-1 /E-2 | 0.1 /0.1 | F-1/F-3 | 90/10 |
| Re-3 | P-2 | 77.8 | A-3 | 20.1 | - | - | C-2 | 2.1 | | | - | - | F-1 | 100 |
| Re-4 | P-2 | 81.8 | A-4 | 14.4 | - | - | C-3 | 3.8 | | | - | - | F-1/F-6 | 40/60 |
| Re-5 | P-3 | 73.8 | A-5 | 24.0 | - | - | C-4 | 2.1 | | | E-3 | 0.1 | F-1/F-2 | 70/30 |
| Re-6 | P-4 | 79.2 | A-6 | 15.4 | - | - | C-5 | 5.4 | | | - | - | F-4 | 100 |
| Re-7 | P-5 | 78.7 | A-7 | 17.5 | B-3 | 3.8 | - | - | | | - | - | F-1/F-5 | 50/50 |
| Re-8 | P-10 | 75.0 | A-8 | 15.2 | B-1 | 9.8 | - | - | | | - | - | F-1/F-2 | 70/30 |
| Re-9 | P-9 | 84.1 | A-9 | 13.0 | - | - | C-3 | 2.9 | | | - | - | F-1/F-6 | 40/60 |
| Re-10 | P-7 | 78.7 | A-10 | 18.7 | - | - | C-4 | 2.6 | | | - | - | F-1/F-2 | 70/30 |
| Re-11 | P-8 | 79.7 | A-11 | 17.8 | - | - | C-4 | 2.4 | | | E-1 | 0.1 | F-7 | 100 |
| Re-12 | P-6 | 79.3 | A-12 | 18.9 | - | - | C-1 | 1.8 | | | - | - | F-1/F-2 | 70/30 |
| Re-13 | P-1/ P-2 | 38.9 /38.9 | A-13 | 19.9 | - | - | C-1 | 2.3 | | | - | - | F-1/F-3 | 90/10 |
| Re-14 | P-6 | 69.4 | A-6 | 15.3 | B-2 | 15.3 | - | - | | | - | - | F-1/F-7 | 80/20 |
| Re-15 | P-5 | 77.1 | A-13 | 15.3 | B-4 | 7.6 | - | - | | | - | - | F-1/F-8 | 85/15 |
| Re-16 | P-1 | 80.3 | A-2 /A-10 | 8.8 /8.8 | - | - | C-2 | 1.9 | | | E-1 /E-2 | 0.1 /0.1 | F-4 | 100 |
| Re-17 | P-2 | 75.9 | A-1 /A-6 | 8.8 /8.8 | B-4 | 6.5 | - | - | | | - | - | F-1/F-5 | 50/50 |
| Re-18 | P-3/ P-4 | 38.5 /38.5 | A-4 | 10.0 | B-1 | 10.0 | C-2 | 3.0 | | | - | - | F-1 | 100 |
| Re-19 | P-2/ P-7 | 39.5 /39.5 | A-5 | 18.8 | - | - | C-3 | 2.2 | | | - | - | F-1/F-2/F-8 | 70/25/5 |
| Re-20 | P-3 | 74.2 | A-8 | 18.2 | B-5 | 7.6 | - | - | | | - | - | F-1/F-6 | 40/60 |
| Re-21 | P-1 | 84.0 | Z-1 | 14.5 | - | - | C-1 | 1.5 | | | - | - | F-1/F-2 | 70/30 |
| Re-22 | P-6 | 82.0 | Z-2 | 16.2 | - | - | C-1 | 1.6 | | | E-1 /E-2 | 0.1/0.1 | F-1/F-3 | 90/10 |
| Re-23 | P-11 | 79.2 | A-2 | 14.3 | - | - | C-2 | 3.0 | D-1 | 3.5 | - | - | F-1/F-8 | 90/10 |
| Re-24 | P-12 | 79.3 | A-3 | 18.0 | - | - | C-1 | 2.2 | D-5 | 0.5 | - | - | F-1/F-2 | 70/30 |
| Re-25 | P-13 | 77.1 | A-4 /A-8 | 8.5 /8.5 | - | - | C-3 | 3.4 | D-4 | 2.5 | - | - | F-1/F-7 | 80/20 |
| Re-26 | P-11/ P-14 | 35.7 /35.7 | A-5 | 24.1 | - | - | C-4 | 3.1 | D-6 | 1.2 | E-1 /E-2 | 0.1 /0.1 | F-4 | 100 |
| Re-27 | P-15 | 75.0 | A-7 | 16.2 | - | - | C-5 | 4.5 | D-2 | 4.2 | E-3 | 0.1 | F-1/F-9 | 85/15 |
| Re-28 | P-16 | 72.1 | A-8 | 19.5 | B-1 | 6.9 | - | - | D-3 | 1.5 | - | - | F-1/F-6 | 40/60 |
| Re-29 | P-12 | 68.3 | A-10 | 18.1 | B-5 | 12.1 | - | - | D-2 | 1.5 | - | - | F-1/F-5 | 50/50 |
| Re-30 | P-14 | 76.0 | A-11 | 20.3 | B-1 | 3.7 | - | - | - | - | - | - | F-1 | 100 |
| Re-31 | P-13 | 79.3 | A-10 | 18.1 | - | - | C-1 | 2.6 | - | - | - | - | F-1/F-2/F-8 | 70/25/5 |
| Re-32 | P-16 | 77.1 | A-4 | 16.8 | B-1 | 6.1 | - | - | - | - | - | - | F-3 | 100 |
| Re-33 | P-11 | 80.0 | Z-1 | 14.3 | - | - | C-2 | 2.2 | D-1 | 3.5 | - | - | F-1/F-8 | 90/10 |
| Re-34 | P-12 | 79.3 | Z-2 | 18.0 | - | - | C-1 | 2.2 | D-5 | 0.5 | - | - | F-1/F-2 | 70/30 |

### [Production of Topcoat Composition]

In the present Example, in a case where a resist film was manufactured using the resist composition, a topcoat prepared on the resist film was further manufactured as desired.

The components used in the topcoat composition used to form the topcoat and a production procedure therefor are shown below.

### <Resin>

The molar fractions of the repeating units based on the respective monomers, and the weight-average molecular weight (Mw) and the dispersity (Mw/Mn) of each resin of the resin used in the topcoat composition are shown in the following table.

Furthermore, with regard to the structures of the monomers corresponding to the repeating units shown in the table, reference can be made to the above-mentioned monomers shown in the description of <Hydrophobic Resin>.

**[Table 4]**

| Table 4 | Molar ratio of repeating unit 1 | | Molar ratio of repeating unit 2 | | Molar ratio of repeating unit 3 | | Mw | Mw/Mn |
|---|---|---|---|---|---|---|---|---|
| Resin PT-1 | ME-2 | 20 | ME-9 | 50 | ME-7 | 30 | 8,000 | 1.6 |
| Resin PT-2 | ME-2 | 15 | ME-6 | 75 | ME-3 | 10 | 5,000 | 1.5 |
| Resin PT-3 | ME-3 | 30 | ME-4 | 20 | ME-10 | 50 | 8,500 | 1.7 |

### <Additive>

The additives included in the topcoat composition are shown below.

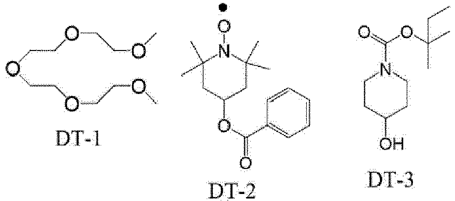

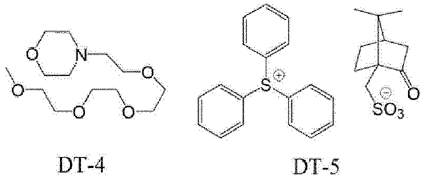

### <Surfactant>

In a case where the topcoat composition included a surfactant, the following surfactant was used.

E-3: PF656 (manufactured by OMNOVA Solutions Inc., fluorine-based surfactant)

### <Solvent>

Solvents included in the topcoat composition are shown below. FT-1: 4-Methyl-2-pentanol (MIBC) FT-2: n-Decane FT-3: Diisoamyl ether

### <Preparation of Topcoat Composition>

A solution was prepared by dissolving the respective components in a solvent such that a formulation shown in the following table was satisfied and a concentration of solid contents of 3.8% by mass was obtained.

Then, the obtained solution was filtered through a polyethylene filter having a pore size of 0.1 µm to prepare a topcoat composition.

**[Table 5]**

| Table 5 | Resin | | Additive | | Surfactant | | Solvent | |
|---|---|---|---|---|---|---|---|---|
| | Type | Mass (g) | Type | Mass (g) | Type | Mass (g) | Type | Mixing ratio (mass) |
| TC-1 | PT-1 | 10.0 | DT-1/DT-2 | 1.3/0.06 | - | - | FT-1/FT-2 | 70/30 |
| TC-2 | PT-2 | 10.0 | DT-3/DT-4 | 0.04/0.06 | E-3 | 0.005 | FT-1/FT-3 | 75/25 |
| TC-3 | PT-3 | 10.0 | DT-5 | 0.05 | - | - | FT-1/FT-3 | 10/90 |

### [Test]

Using the resist composition prepared as mentioned above, the LWR of a pattern developed under each of the following conditions was evaluated.

Furthermore, in any of the tests, a resist composition which had been left in an environment of 4°C for 3 months after production thereof was used as the resist composition used for pattern formation (actinic ray-sensitive or radiation-sensitive resin composition).

### <EUV Exposure and Organic Solvent Development

### (Pattern Formation)

A composition for forming an underlayer film, AL412 (manufactured by Brewer Science, Inc.), was applied onto a silicon wafer and baked at 205°C for 60 seconds to form an underlying film having a film thickness of 20 nm. A resist composition shown in Table 6 was applied thereon and baked at 100°C for 60 seconds to form a resist film having a film thickness of 30 nm.

The silicon wafer having the obtained resist film was subjected to patternwise irradiation using an EUV exposure device (manufactured by Exitech Ltd., Micro Exposure Tool, NA 0.3, Quadrupol, outer sigma 0.68, inner sigma 0.36). Furthermore, as the reticle, a mask having a line size = 20 nm and a line: space = 1:1 was used.

The resist film after the exposure was baked at 90°C for 60 seconds, developed with n-butyl acetate for 30 seconds, and spin-dried to obtain a negative tone pattern.

### (Evaluation)

In a case where a 20 nm (1:1) line-and-space pattern resolved with an optimum exposure amount upon resolving a line pattern having an average line width of 20 nm was observed from the upper part of the pattern using a critical dimension scanning electron microscope (SEM (S-9380II manufactured by Hitachi, Ltd.)). The line width of the pattern was observed at any points (100 points), and a measurement deviation thereof was evaluated with 3σ and taken as an LWR. A smaller value of LWR indicates better LWR performance. LWR (nm) is preferably 4.5 nm or less, more preferably 3.9 nm or less, and still more preferably 3.5 nm or less.

The results are shown in the following table.

The "Formula" column in the table indicates which of General Formulae (1) to (3) the specific compound used corresponds to.

The "F-AL" column indicates whether or not a cation in the specific compound used has a fluorine-containing group which is a fluoroalkyl group. A case where the present requirement is satisfied is evaluated as "A", and a case where the present requirement is not satisfied is evaluated as "B".

The "Condition X" column indicates whether or not the specific compound used satisfies any of the following conditions X1 to X3. A case where the present requirement is satisfied is evaluated as "A", and a case where the present requirement is not satisfied is evaluated as "B".

Condition X1: The specific compound used is a compound represented by General Formula (1), and the aromatic hydrocarbon ring group represented by each of Ar¹, Ar², and Ar³ has a total of three or more fluoroalkyl groups, or
the aromatic hydrocarbon ring group has a total of one or more organic groups other than an electron-withdrawing group, and a total number of carbon atoms included in the total of one or more organic groups other than an electron-withdrawing group is 3 or more.

Condition X2: The specific compound used is a compound represented by General Formula (2), and the aromatic hydrocarbon ring group represented by each of Ar⁴, Ar⁵, and Ar⁶ has a total of three or more fluorine-containing groups, or
the aromatic hydrocarbon ring group represented by each of Ar⁴, Ar⁵, and Ar⁶ has a total of one or more organic groups other than a linear or branched electron-withdrawing group, and a total number of carbon atoms included in the total of one or more organic groups other than a linear or branched electron-withdrawing group is 3 or more.

Condition X3: The specific compound used is a compound represented by General Formula (3), and a total number of carbon atoms included in an organic group other than an electron-withdrawing group, contained in the aromatic hydrocarbon ring group represented by each of Ar⁷, Ar⁸, and Ar⁹, is 3 or more.

**[Table 6]**

| Table 6 | Characteristics | | | | | | | | | Evaluation results |
|---|---|---|---|---|---|---|---|---|---|---|
| | Resist composition | Photoacid generator 1 | | | | Photoacid generator 2 | | | | LWR (nm) after time passage (organic solvent development) |
| | | Type | Formula | F-AL | Condition X | Type | Formula | F-AL | Condition X | |
| Example 1-1 | Re-1 | A-1 | (2) | A | A | - | - | - | - | 2.9 |
| Example 1-2 | Re-2 | A-2 | (2) | A | A | - | - | - | - | 3.1 |
| Example 1-3 | Re-3 | A-3 | (2) | A | A | - | - | - | - | 3.3 |
| Example 1-4 | Re-4 | A-4 | (2) | B | B | - | - | - | - | 4.2 |
| Example 1-5 | Re-5 | A-5 | (2) | A | B | - | - | - | - | 3.7 |
| Example 1-6 | Re-6 | A-6 | (2) | A | A | - | - | - | - | 3.4 |
| Example 1-7 | Re-7 | A-7 | (1) | A | A | B-3 | (3) | A | A | 2.9 |
| Example 1-8 | Re-8 | A-8 | (1) | B | B | B-1 | - | - | - | 4.4 |
| Example 1-9 | Re-9 | A-9 | (1) | A | A | - | - | - | - | 3.2 |
| Example 1-10 | Re-10 | A-10 | (3) | A | A | - | - | - | - | 3.3 |
| Example 1-11 | Re-11 | A-11 | (3) | B | B | - | - | - | - | 4.1 |
| Example 1-12 | Re-12 | A-12 | (3) | A | B | - | - | - | - | 3.8 |
| Example 1-13 | Re-13 | A-13 | (3) | A | A | - | - | - | - | 3 |
| Example 1-14 | Re-14 | A-6 | (2) | A | A | B-2 | (2) | A | A | 2.9 |
| Example 1-15 | Re-15 | A-13 | (3) | A | A | B-4 | (2) | A | A | 3.4 |
| Example 1-16 | Re-16 | A-2 /A-10 | (2)/(3) | A/A | A/A | - | - | - | - | 3.3 |
| Example 1-17 | Re-17 | A-1 /A-6 | (2)/(2) | A/A | A/A | B-4 | (2) | A | A | 3.2 |
| Example 1-18 | Re-18 | A-4 | (2) | B | B | B-1 | - | - | - | 4.3 |
| Example 1-19 | Re-19 | A-5 | (2) | A | B | - | - | - | - | 3.9 |
| Example 1-20 | Re-20 | A-8 | (1) | B | B | B-5 | (1) | B | B | 4.3 |
| Comparative Example 1-1 | Re-21 | Z-1 | - | - | - | - | - | - | - | 5.8 |
| Comparative Example 1-2 | Re-22 | Z-2 | - | - | - | - | - | - | - | 5.4 |

As shown in the table, it was confirmed that in a case where a pattern is obtained by performing EUV exposure and organic solvent development, the resist composition of the embodiment of the present invention is capable of forming a pattern having excellent LWR performance even with a use of the resist composition that has been stored for a long period of time.

In addition, it was confirmed that in a case where a specific compound in which the fluorine-containing group is a fluoroalkyl group is used, the effect of the present invention is more excellent (see the comparison between Examples in which the specific compounds satisfying the requirements in the "F-AL" column were used and Examples in which the specific compounds not satisfying the requirements were used).

It was confirmed that in a case where a specific compound satisfying the condition X is used, the effect of the present invention is more excellent (see the comparison between Examples in which the specific compounds satisfying the requirements in the "Condition X" column were used and Examples in which the specific compounds not satisfying the requirements were used).

### <EUV Exposure and Alkali Development>

### (Pattern Formation)

A composition for forming an underlayer film, AL412 (manufactured by Brewer Science, Inc.), was applied onto a silicon wafer and baked at 205°C for 60 seconds to form an underlying film having a film thickness of 20 nm. A resist composition shown in Table 7 was applied thereon and baked at 100°C for 60 seconds to form a resist film having a film thickness of 30 nm.

The silicon wafer having the obtained resist film was subjected to patternwise irradiation using an EUV exposure device (manufactured by Exitech Ltd., Micro Exposure Tool, NA 0.3, Quadrupol, outer sigma 0.68, inner sigma 0.36). Furthermore, as a reticle, a mask having a line size = 20 nm and a line:space = 1:1 was used.

The resist film after the exposure was baked at 90°C for 60 seconds, developed with an aqueous tetramethylammonium hydroxide solution (2.38%-by-mass) for 30 seconds, and then rinsed with pure water for 30 seconds. Thereafter, the resist film was spin-dried to obtain a positive tone pattern.

### (Evaluation)

The obtained pattern was evaluated in the same manner as in the evaluation of the LWR of a pattern in <EUV Exposure and Organic Solvent Development>.

The results are shown in the following table. The meaning of each column in the table is as described above.

**[Table 7]**

| Table 7 | Characteristics | | | | | | | | | Evaluation results |
|---|---|---|---|---|---|---|---|---|---|---|
| | Resist composition | Photoacid generator 1 | | | | Resist composition | | | | Photoacid generator 1 |
| | | Type | Formula | Type | Condition X | Type | Formula | F-AL | Condition X | |
| Example 2-1 | Re-1 | A-1 | (2) | A | A | - | - | - | - | 3.4 |
| Example 2-2 | Re-2 | A-2 | (2) | A | A | - | - | - | - | 3.1 |
| Example 2-3 | Re-3 | A-3 | (2) | A | A | - | - | - | - | 3.2 |
| Example 2-4 | Re-4 | A-4 | (2) | B | B | - | - | - | - | 4.2 |
| Example 2-5 | Re-5 | A-5 | (2) | A | B | - | - | - | - | 3.6 |
| Example 2-6 | Re-6 | A-6 | (2) | A | A | - | - | - | - | 2.9 |
| Example 2-7 | Re-7 | A-7 | (1) | A | A | B-3 | (3) | A | A | 3.1 |
| Example 2-8 | Re-8 | A-8 | (1) | B | B | B-1 | - | - | - | 4.5 |
| Example 2-9 | Re-9 | A-9 | (1) | A | A | - | - | - | - | 3.4 |
| Example 2-10 | Re-10 | A-10 | (3) | A | A | - | - | - | - | 3.2 |
| Example 2-11 | Re-11 | A-ll | (3) | B | B | - | - | - | - | 4.3 |
| Example 2-12 | Re-12 | A-12 | (3) | A | B | - | - | - | - | 3.8 |
| Example 2-13 | Re-13 | A-13 | (3) | A | A | - | - | - | - | 3.5 |
| Example 2-14 | Re-14 | A-6 | (2) | A | A | B-2 | (2) | A | A | 3.1 |
| Example 2-15 | Re-15 | A-13 | (3) | A | A | B-4 | (2) | A | A | 3.2 |
| Example 2-16 | Re-16 | A-2 /A-10 | (2)/(3) | A/A | A/A | - | - | - | - | 3.1 |
| Example 2-17 | Re-17 | A-1 /A-6 | (2)/(2) | A/A | A/A | B-4 | (2) | A | A | 3.3 |
| Example 2-18 | Re-18 | A-4 | (2) | B | B | B-1 | - | - | - | 4.2 |
| Example 2-19 | Re-19 | A-5 | (2) | A | B | - | - | - | - | 3.7 |
| Example 2-20 | Re-20 | A-8 | (1) | B | B | B-5 | (1) | B | B | 4.1 |
| Comparative Example 2-1 | Re-21 | Z-1 | - | - | - | - | - | - | - | 5.7 |
| Comparative Example 2-2 | Re-22 | Z-2 | - | - | - | - | - | - | - | 5.5 |

As shown in the table, it was confirmed that in a case where a pattern is obtained by performing EUV exposure and alkali development, the resist composition of the embodiment of the present invention is capable of forming a pattern having excellent LWR performance even with a use of the resist composition that has been stored for a long period of time.

In addition, it was confirmed that in a case where a specific compound in which the fluorine-containing group is a fluoroalkyl group is used, the effect of the present invention is more excellent (see the comparison between Examples in which the specific compounds satisfying the requirements in the "F-AL" column were used and Examples in which the specific compounds not satisfying the requirements were used).

It was confirmed that in a case where a specific compound satisfying the condition X is used, the effect of the present invention is more excellent (see the comparison between Examples in which the specific compounds satisfying the requirements in the "Condition X" column were used and Examples in which the specific compounds not satisfying the requirements were used).

### <ArF Liquid Immersion Exposure and Organic Solvent Development>

### (Pattern Formation)

A composition for forming an organic antireflection film, ARC29SR (manufactured by Brewer Science, Inc.), was applied onto a silicon wafer and baked at 205°C for 60 seconds to form an antireflection film having a film thickness of 98 nm. The resist composition shown in Table 8 was applied thereon and baked at 100°C for 60 seconds to form a resist film (actinic ray-sensitive or radiation-sensitive film) having a film thickness of 90 nm.

Furthermore, in Example 3-8, Example 3-9, and Example 3-10, a topcoat film was formed on the upper layer of the resist film (the types of topcoat compositions used are shown in Table 8). The film thickness of the topcoat film was 100 nm in any case.

The resist film was exposed through a 6% halftone mask having a 1:1 line-and-space pattern with a line width of 45 nm, using an ArF excimer laser liquid immersion scanner (XT1700i, manufactured by ASML, NA 1.20, Dipole, outer sigma: 0.950, inner sigma: 0.850, Y deflection). Ultrapure water was used as the immersion liquid.

The resist film after the exposure was baked at 90°C for 60 seconds, developed with n-butyl acetate for 30 seconds, and then rinsed with 4-methyl-2-pentanol for 30 seconds. Then, the film was spin-dried to obtain a negative tone pattern.

### (Evaluation)

In a case where a 45 nm (1:1) line-and-space pattern resolved with an optimum exposure amount upon resolving a line pattern having an average line width of 45 nm was observed from the upper part of the pattern using a critical dimension scanning electron microscope (SEM (S-9380II manufactured by Hitachi, Ltd.)). The line width of the pattern was observed at any points (100 points), and a measurement deviation thereof was evaluated with 3σ and taken as an LWR. A smaller value of LWR indicates better LWR performance. LWR (nm) is preferably 4.0 nm or less, more preferably 3.5 nm or less, and still more preferably 3.0 nm or less.

The results are shown in the following table. The meaning of each column in the table is as described above.

**[Table 8]**

| Table 8 | Characteristics | | | | | | | | | | Evaluation results |
|---|---|---|---|---|---|---|---|---|---|---|---|
| | Resist composition | Photoacid generator 1 | | | | Photoacid generator 2 | | | | Topcoat composition | LWR (nm) after time passage (organic solvent development) |
| | | Type | Formula | F-AL | Condition X | Type | Formula | F-AL | Condition X | | |
| Example 3-1 | Re-23 | A-2 | (2) | A | A | - | - | - | - | - | 2.5 |
| Example 3-2 | Re-24 | A-3 | (2) | A | A | - | - | - | - | - | 2.7 |
| Example 3-3 | Re-25 | A-4 /A-8 | (2)/(1) | B/B | B/B | - | - | - | - | - | 3.8 |
| Example 3-4 | Re-26 | A-5 | (2) | A | B | - | - | - | - | - | 3.2 |
| Example 3-5 | Re-27 | A-7 | (1) | A | A | - | - | - | - | - | 2.9 |
| Example 3-6 | Re-28 | A-8 | (1) | B | B | B-1 | - | - | - | - | 3.7 |
| Example 3-7 | Re-29 | A-10 | (3) | A | A | B-5 | (1) | B | B | - | 2.9 |
| Example 3-8 | Re-30 | A-11 | (3) | B | B | B-1 | - | - | - | TC-1 | 3.8 |
| Example 3-9 | Re-31 | A-10 | (3) | A | A | - | - | - | - | TC-2 | 2.5 |
| Example 3-10 | Re-32 | A-4 | (2) | B | B | B-1 | - | - | - | TC-3 | 3.6 |
| Comparative Example 3-1 | Re-33 | Z-1 | - | - | - | - | - | - | - | - | 4.9 |
| Comparative Example 3-2 | Re-34 | Z-2 | - | - | - | - | - | - | - | - | 4.8 |

As shown in the table, it was confirmed that in a case where a pattern is obtained by performing ArF exposure and organic solvent development, the resist composition of the embodiment of the present invention is capable of forming a pattern having excellent LWR performance even with a use of the resist composition that has been stored for a long period of time.

In addition, it was confirmed that in a case where a specific compound in which the fluorine-containing group is a fluoroalkyl group is used, the effect of the present invention is more excellent (see the comparison between Examples in which the specific compounds satisfying the requirements in the "F-AL" column were used and Examples in which the specific compounds not satisfying the requirements were used).

It was confirmed that in a case where a specific compound satisfying the condition X is used, the effect of the present invention is more excellent (see the comparison between Examples in which the specific compounds satisfying the requirements in the "Condition X" column were used and Examples in which the specific compounds not satisfying the requirements were used).

### <ArF Liquid Immersion Exposure and Alkali Development>

### (Pattern Formation)

A composition for forming an organic antireflection film, ARC29SR (manufactured by Brewer Science, Inc.), was applied onto a silicon wafer and baked at 205°C for 60 seconds to form an antireflection film having a film thickness of 98 nm. A resist composition shown in Table 9 was applied thereon and baked at 100°C for 60 seconds to form a resist film having a film thickness of 90 nm. Furthermore, in Example 4-8, Example 4-9, and Example 4-10, a topcoat film was formed on the upper layer of the resist film (the types of topcoat compositions used are shown in Table 9). The film thickness of the topcoat film was 100 nm in any case.

The resist film was exposed through a 6% halftone mask having a 1:1 line-and-space pattern with a line width of 45 nm, using an ArF excimer laser liquid immersion scanner (XT1700i, manufactured by ASML, NA 1.20, Dipole, outer sigma: 0.950, inner sigma: 0.890, Y deflection). Ultrapure water was used as the immersion liquid.

The resist film after the exposure was baked at 90°C for 60 seconds, developed with an aqueous tetramethylammonium hydroxide solution (2.38%-by-mass) for 30 seconds, and then rinsed with pure water for 30 seconds. Thereafter, the resist film was spin-dried to obtain a positive tone pattern.

### (Evaluation)

The obtained pattern was evaluated in the same manner as in the evaluation of the LWR of a pattern in <ArF Liquid Immersion Exposure and Organic Solvent Development>.

The results are shown in the following table. The meaning of each column in the table is as described above.

**[Table 9]**

| Table 9 | Characteristics | | | | | | | | | | Evaluation results |
|---|---|---|---|---|---|---|---|---|---|---|---|
| | Resist composition | Photoacid generator 1 | | | | Photoacid generator 2 | | | | Topcoat composition | LWR (nm) after time passage (alkali development) |
| | | Type | Formula | F-AL | Condition X | Type | Formula | F-AL | Condition X | | |
| Example 4-1 | Re-23 | A-2 | (2) | A | A | - | - | - | - | - | 2.8 |
| Example 4-2 | Re-24 | A-3 | (2) | A | A | - | - | - | - | - | 2.5 |
| Example 4-3 | Re-25 | A-4 /A-8 | (2)/(1) | B/B | B/B | - | - | - | - | - | 3.8 |
| Example 4-4 | Re-26 | A-5 | (2) | A | B | - | - | - | - | - | 3.3 |
| Example 4-5 | Re-27 | A-7 | (1) | A | A | - | - | - | - | - | 2.9 |
| Example 4-6 | Re-28 | A-8 | (1) | B | B | B-1 | - | - | - | - | 3.7 |
| Example 4-7 | Re-29 | A-10 | (3) | A | A | B-5 | (1) | B | B | - | 2.7 |
| Example 4-8 | Re-30 | A-ll | (3) | B | B | B-1 | - | - | - | TC-1 | 3.7 |
| Example 4-9 | Re-31 | A-10 | (3) | A | A | - | - | - | - | TC-2 | 2.6 |
| Example 4-10 | Re-32 | A-4 | (2) | B | B | B-1 | - | - | - | TC-3 | 3.9 |
| Comparative Example 4-1 | Re-33 | Z-1 | - | - | - | - | - | - | - | - | 4.7 |
| Comparative Example 4-2 | Re-34 | Z-2 | - | - | - | - | - | - | - | - | 4.8 |

As shown in the table, it was confirmed that in a case where a pattern is obtained by performing ArF exposure and alkali development, the resist composition of the embodiment of the present invention is capable of forming a pattern having excellent LWR performance even with a use of the resist composition that has been stored for a long period of time.

In addition, it was confirmed that in a case where a specific compound in which the fluorine-containing group is a fluoroalkyl group is used, the effect of the present invention is more excellent (see the comparison between Examples in which the specific compounds satisfying the requirements in the "F-AL" column were used and Examples in which the specific compounds not satisfying the requirements were used).

It was confirmed that in a case where a specific compound satisfying the condition X is used, the effect of the present invention is more excellent (see the comparison between Examples in which the specific compounds satisfying the requirements in the "Condition X" column were used and Examples in which the specific compounds not satisfying the requirements were used).

## Claims

1. An actinic ray-sensitive or radiation-sensitive resin composition comprising:
one or more specific compounds selected from the group consisting of a compound represented by General Formula (1), a compound represented by General Formula (2), and a compound represented by General Formula (3); and
an acid-decomposable resin, 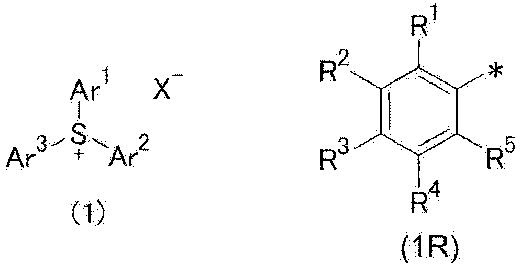
in General Formula (1), X⁻ represents an organic anion,
Ar¹ and Ar² each independently represent an aromatic hydrocarbon ring group having at least one substituent, and
Ar³ represents an aromatic hydrocarbon ring group represented by General Formula (1R),
in General Formula (1R), ^{∗} represents a bonding position,
R¹ to R⁵ each independently represent a hydrogen atom, a fluorine atom, or an alkyl group which may have a substituent,
provided that at least one of R¹ or R⁵ represents a fluorine-containing group selected from the group consisting of a fluorine atom and a fluoroalkyl group, and
in General Formula (1), at least two of Ar¹, Ar², or Ar³ represent groups having different structures, 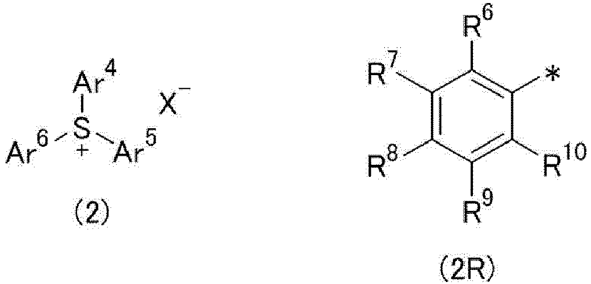
in General Formula (2), X⁻ represents an organic anion,
Ar⁴ and Ar⁵ each independently represent an aromatic hydrocarbon ring group having at least one substituent,
Ar⁶ represents an aromatic hydrocarbon ring group represented by General Formula (2R),
in General Formula (2R), ^{∗} represents a bonding position, R⁶, R⁷, R⁹, and R¹⁰ each independently represent a hydrogen atom, a fluorine atom, or an alkyl group which may have a substituent,
R⁸ represents a fluorine-containing group selected from the group consisting of a fluorine atom and a fluoroalkyl group,
provided that in General Formula (2), at least two of Ar⁴, Ar⁵, or Ar⁶ represent groups having different structures, and
in General Formula (2), the aromatic hydrocarbon ring group represented by each of Ar⁴, Ar⁵, and Ar⁶ has a total of two or more fluorine-containing groups, 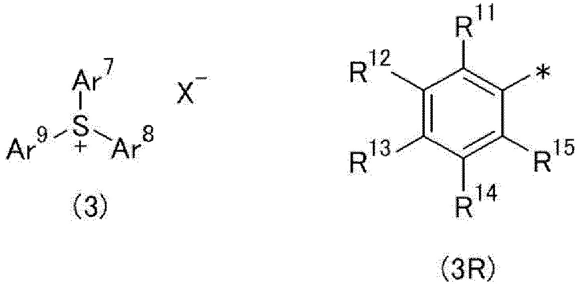
in General Formula (3), X⁻ represents an organic anion,
Ar⁷ and Ar⁸ each independently represent an aromatic hydrocarbon ring group which may have an organic group other than an electron-withdrawing group,
provided that the aromatic hydrocarbon ring group represented by each of Ar⁷ and Ar⁸ has a total of one or more organic groups other than an electron-withdrawing group,
Ar⁹ represents an aromatic hydrocarbon ring group represented by General Formula (3R),
in General Formula (3R), ^{∗} represents a bonding position, R¹¹, R¹³, and R¹⁵ each independently represent a hydrogen atom, a fluorine atom, or an alkyl group which may have a substituent, and
R¹² and R¹⁴ each independently represent a fluorine-containing group selected from the group consisting of a fluorine atom and a fluoroalkyl group.

2. The actinic ray-sensitive or radiation-sensitive resin composition according to claim 1,
wherein the fluorine-containing group in each of General Formulae (1) to (3) is a fluoroalkyl group.

3. The actinic ray-sensitive or radiation-sensitive resin composition according to claim 1 or 2,
wherein in General Formula (1), the aromatic hydrocarbon ring group represented by each of Ar¹, Ar², and Ar³ has a total of three or more fluoroalkyl groups or has a total of one or more organic groups other than an electron-withdrawing group, and a total number of carbon atoms included in the total of one or more organic groups other than an electron-withdrawing group is 3 or more,
in General Formula (2), the aromatic hydrocarbon ring group represented by each of Ar⁴, Ar⁵, and Ar⁶ has a total of three or more fluorine-containing groups, or
the aromatic hydrocarbon ring group represented by each of Ar⁴, Ar⁵, and Ar⁶ has a total of one or more linear or branched organic groups other than an electron-withdrawing group, and a total number of carbon atoms included in the total of one or more linear or branched organic groups other than an electron-withdrawing group is 3 or more, and
in General Formula (3), a total number of carbon atoms included in the organic groups other than an electron-withdrawing group, contained in the aromatic hydrocarbon ring group represented by each of Ar⁷, Ar⁸, and Ar⁹, is 3 or more.

4. The actinic ray-sensitive or radiation-sensitive resin composition according to any one of claims 1 to 3, wherein Ar⁶ represents a group represented by General Formula (2S), 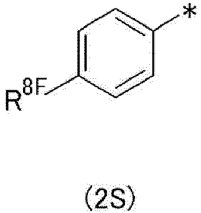
in General Formula (2S), ^{∗} represents a bonding position, and
R^{8F} represents a fluoroalkyl group.

5. The actinic ray-sensitive or radiation-sensitive resin composition according to claim 4, wherein Ar⁵ and Ar⁶ each represent a group represented by General Formula (2S).

6. A resist film formed of the actinic ray-sensitive or radiation-sensitive resin composition according to any one of claims 1 to 5.

7. A pattern forming method comprising:
a step of forming a resist film on a substrate, using the actinic ray-sensitive or radiation-sensitive resin composition according to any one of claims 1 to 5;
a step of exposing the resist film; and
a step of developing the exposed resist film using a developer to form a pattern.

8. A method for manufacturing an electronic device, comprising the pattern forming method according to claim 7.
